# EUROPEAN PATENT SPECIFICATION

(11) **EP 1 814 846 B1**
(45) Date of publication and mention of the grant of the patent: **08.04.2009**
(21) Application number: 05806462.7
(22) Date of filing: 08.11.2005
(51) Int. Cl.: C07C 317/00, A61K 31/10, A61K 31/145, A61P 3/04, A61P 3/06, A61P 3/10

(54) **11BETA-HSD1 INHIBITORS**
11BETA-HSD1-INHIBITOREN
INHIBITEURS DE LA 11BETA-HSD1

(30) Priority: 08.11.2004 EP 04026441
(43) Date of publication of application: 08.08.2007
(73) Proprietor: Evotec AG, 22525 Hamburg (DE)
(72) Inventor: COULTER, Thomas Stephen, Wantage OX 12 7PA (GB); TAYLOR ,Steven, Didcot OX11 7 AU (GB); FRYATT, Tara, Didcot OX11 7XU (GB); AICHER, Babette, 60318 Frankfurt (DE); SCHNEIDER, Martin, 37085 Goettingen (DE)
(74) Representative: Dick, Alexander
(86) International application number: PCT/EP2005/011933
(87) International publication number: WO 2006/048330

(56) References cited:
- WO-A-03/078398
- WO-A-20/04041264
- ZIESSEL, RAYMOND ET AL: "Convenient and multistep preparation of oligopyridines bearing multiple dansyl and nitroxide radicals" SYNTHESIS , (14), 2145-2154 CODEN: SYNTBF; ISSN: 0039-7881, 2003, XP001205895
- YU. A. AIZINA ET AL.: "N-(2,2,2-Trichloroethylidene)- and N-(1-Hydroxy-2,2,2-tri-chloroethyl)amides in C-Amidoalkylation Reaction of Functionally-substituted Aromatic Compounds" RUSSIAN JOURNAL OF ORGANIC CHEMISTRY, vol. 38, no. 2, 2002, pages 235-238, XP009047371
- G.B. SINGH ET AL.: "Mannich Bases of Salicylic Acid Hydrazide" J. INDIAN CHEM. SOC., vol. 56, 1979, pages 168-170, XP009047370

## Description

The present invention relates to a novel class of 11β-HSD1 inhibitors, including pharmaceutically acceptable salts thereof, which are useful as therapeutic compounds, particularly in the treatment of type 2 diabetes mellitus, often referred to as non-insulin dependent diabetes mellitus (NIDDM), and of conditions that are often associated with this disease, such as metabolic syndrome, obesity and lipid disorders. The present invention also relates to processes for preparing said compounds.

Diabetes is a disease derived from multiple causative factors and characterized by elevated levels of plasma glucose (hyperglycemia) in the fasting state or after administration of glucose during an oral glucose tolerance test. There are two generally recognized forms of diabetes. In type 1 diabetes, or insulin-dependent diabetes mellitus (IDDM), patients produce little or no insulin, the hormone which regulates glucose utilization. In type 2 diabetes, or noninsulin-dependent diabetes mellitus (NIDDM), insulin is still produced in the body. Patients often have hyperinsulinemia (plasma insulin levels that are the same or even elevated in comparison with non-diabetic subjects); however, these patients have developed insulin resistance, which is a resistance to the effect of insulin in stimulating glucose and lipid metabolism in the main insulin-sensitive tissues, which are muscle, liver and adipose tissues. Patients who are insulin resistant but not diabetic have elevated insulin levels that compensate for the insulin resistance so that serum glucose levels are not elevated. In patients with NIDDM, the plasma insulin levels, even when they are elevated, are insufficient to overcome the pronounced insulin resistance.

Insulin resistance is not primarily due to a diminished number of insulin receptors but to a post-insulin receptor binding defect that is not yet completely understood. This resistance to insulin responsiveness results in insufficient insulin activation of glucose uptake, oxidation and storage in muscle and inadequate insulin repression of lipolysis in adipose tissue and of glucose production and secretion in the liver (gluconeogenesis and glycogenolysis).

Persistent or uncontrolled hyperglycemia that occurs with diabetes is associated with increased and premature morbidity and mortality. Often abnormal glucose homeostasis is associated both directly and indirectly with obesity, hypertension, and alterations of the lipid, lipoprotein and apolipoprotein metabolism and other metabolic and hemodynamic disease. Therefore patients with type 2 diabetes mellitus are at an especially increased risk of macrovascular and microvascular complications, including atherosclerosis, coronary heart disease, stroke, peripheral vascular disease, hypertension, nephropathy, neuropathy, and retinopathy.

Therefore, therapeutic control of glucose homeostasis, lipid metabolism, obesity, and hypertension are critically important in the clinical management and treatment of diabetes mellitus and metabolic syndrome.

Many patients who have insulin resistance but have not developed type 2 diabetes are at a risk of developing at least several symptoms selected from a group of symptoms that are often referred to as syndrome X, or the metabolic syndrome.

This syndrome is characterized by insulin resistance, abdominal/visceral obesity, hyperinsulinemia, high blood pressure, low HDL, and high VLDL (dyslipidemia). These patients, whether or not they develop overt diabetes mellitus, are at increased risk of the macrovascular and microvascular complications of type 2 diabetes listed above (e. g. atherosclerosis and coronary heart disease/cardiovascular disease).
The metabolic syndrome often starts with continuous weight increase leading to significant visceral obesity. Increased visceral adipose tissue mass, which is not only an energy storage organ but also an important endocrine organ secreting hormones and signalling molecules, is believed to be a major source for the development of all the diseases that are summarized as metabolic syndrome especially dyslipidemia, hypertension, insulin resistance and β-cell dysfunction. Progression of visceral obesity can lead toinsulin resistance causing impaired glucose tolerance and finally type 2 diabetes mellitus. Diabetes, dyslipidemia and hypertension are major risk factors for cardiovascular disease, a main cause for death in western world.
Increased 11β-HSD 1 activity in adipocytes is believed to be one of the essential factors that are responsible for the increase of visceral adipose tissue mass because overexpression in adipose tissue in mice causes visceral obesity and finally a disease state representing human metabolic syndrome (Masuzaki, H. et al. 2001).Furthermore, increased 11β-HSD 1 activity in visceral adipose tissue is found in patients with visceral obesity and/or type 2 diabetes mellitus and in patients with metabolic syndrome.
On the other hand deletion of 11β-HSD 1 activity in mice protects against diet-induced obesity and related diseases like, glucose intolerance, insulin resistance, type 2 diabetes mellitus and metabolic syndrome (Kotelevtsev, Y. et al. 1997; Morton, NM. et al. 2001 and 2004) and ameliorates age-related learning impairments in addition (Yau, JLW. et al. 2001).

The available treatments for type 2 diabetes have not changed substantially in many years, and these treatments have recognized limitations.

Physical exercise and reductions in dietary intake of calories often dramatically improve the diabetic condition, but compliance with this treatment is very poor because of well-entrenched sedentary lifestyles and excess food consumption, especially of foods containing high amounts of sugar and saturated fat. Increasing the plasma level of insulin by administration of sulfonylureas (e. g. tolbutamide and glipizide) or meglitinide, which stimulate the pancreatic β-cells to secrete more insulin, and/or by injection of insulin when sulfonylureas or meglitinide become ineffective, can result in insulin concentrations high enough to stimulate the very insulin-resistant tissues.

However, dangerously low levels of plasma glucose can result from administration of insulin or insulin secretagogues (sulfonylureas or meglitinide), and an increased level of insulin resistance due to the even higher plasma insulin levels can occur. The biguanides increase insulin sensitivity resulting in some correction of hyperglycemia.

However, the two biguanides, phenformin and metformin, can induce lactic acidosis and nausea/diarrhea. Metformin has fewer side effects than phenformin and is often prescribed for the treatment of type 2 diabetes.

The glitazones (i. e. 5-benzylthiazolidine-2,4-diones) are a newer class of compounds with the potential for ameliorating hyperglycemia and other symptoms of type 2 diabetes. These agents substantially increase insulin sensitivity in muscle, liver and adipose tissue in several animal models of type 2 diabetes, resulting in partial or complete correction of the elevated plasma levels of glucose without occurrence of hypoglycemia. The glitazones that are currently marketed are agonists of the peroxisome proliferator activated receptor (PPAR) gamma subtype. PPARgamma agonism is generally believed to be responsible for the improved insulin sensititization that is observed with the glitazones. Besides these positive effects glitazones promote adipocyte differentiation leading to undesirable weight gain. Newer PPAR agonists that are being developed for treatment of type 2 diabetes and/or dyslipidemia are agonists of one or more of the PPAR alpha, gamma and delta subtypes.

There is a continuing need for new methods of treating the disease.

New biochemical approaches that have been recently introduced or are under active development include treatment with alpha-glucosidase inhibitors (e. g. acarbose), protein tyrosine phosphatase-1B (PTP-1B) inhibitors, and inhibitors of the dipeptidyl peptidase-IV (DPP-IV) enzyme. Inhibition of the expression of PTP-1B by the use of antisense oligonucleotides is also under investigation.

Another method of treating type 2 diabetes that has been suggested in the literature is the use of inhibitors of the 11-β-hydroxysteroid dehydrogenase type 1 enzyme (11β-HSD 1) to reduce the amount of active glucocorticoids in tissues where glucose is metabolized.

Mono- and bicyclic thiazole sulfonamides are disclosed in WO-A 01/90090, WO-A 01/90091, WO-A 01/90092, WO-A 01/90093, WO-A 01/90094, WO-A 03/043999 and WO-A 2004/037251. Thiophene and thiadiazole sufonamides are known from WO-A 03/044009 and WO-A 03/044000. Additionally compounds containing a triazole moiety are known from WO-A 03/065983 and WO-A 03/104208.

Aryl and heteroaryl ketones are known from WO-A 04/011410. 1,4-Disubstituted piperidines are known from WO-A 04/033427 and 2-oxo-ethanesulfonamides are known from WO-A 04/041264. Adamantyl acetamides are known from WO-A 04/056744 and WO-A 04/056745. Amide derivatives are known from WO-A 04/065351.

Compounds with similar structure but related to a different technical field are known from WO 03/078398, R. Ziessel et al., Synthesis 14 (2003), 2145-2154) and Y.A. Aizina et al., Russian Journal of Organic Chemisty 38 (2002), 235-238.

Thus, the object of the present invention is to provide a new class of compounds as 11β-HSD 1 inhibitors which may be effective in the treatment of type 2 diabetes, metabolic syndrome and other 11β-HSD 1 modulated diseases.

Accordingly, the present invention provides compounds of formula (I) 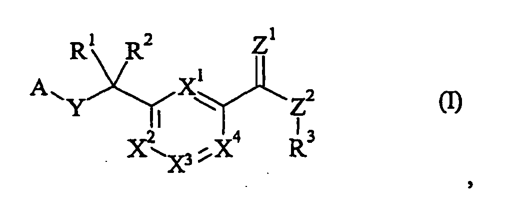 or a pharmaceutically acceptable salt, prodrug or metabolite thereof, wherein
X¹, X², X³, X⁴ are independently selected from the group consisting ofN; and CR⁴, wherein R⁴ is independently H; halogen; CN; C(O)OR^{4a}; C(O)N(R^{4a}R^{4b}); OR^{4a}; N(R^{4a}R^{4b}); phenyl; C₁₋₆ alkyl; or C₃₋₇ cycloalkyl, wherein C₁₋₆ alkyl and C₃₋₇ cycloalkyl are optionally substituted with one or more halogen, which are the same or different;
R^{4a}, R^{4b} are independently selected from the group consisting of H; C₁₋₆ alkyl; and C₃₋₇ cycloalkyl;
R¹, R² are independently selected from the group consisting of H; C₁₋₆ alkyl; C₃₋₇ cycloalkyl; and halogen, wherein C₁₋₆ alkyl and C₃₋₇ cycloalkyl are optionally substituted with halogen;
Optionally R¹, R² are combined to form oxo (=O);
Y is -N(R^{1a})S(O)₂-; -S(O)₂N(R^{1b})-; or -N(R^{1a})S(O)₂N(R^{1b})-;
A is T; or C₁₋₆ alkyl, wherein C₁₋₆ alkyl is optionally substituted with one or more R⁵, wherein R⁵ is independently halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); or T;
R^{1a}, R^{1b} are independently selected from the group consisting of H; allyl; benzyl; CH₂-C(O)NH₂; CH₂-COOH; CH₂-C(O)O-C₁₋₆ alkyl; C₁₋₆ alkyl; and C₃₋₇ cycloalkyl, wherein C₁₋₆ alkyl and C₃₋₇ cycloalkyl are optionally substituted with one or more R^{1c}, wherein R^{1c} is independently halogen; C₁₋₆ alkyl; -CH=CH₂; phenyl; C(O)NH₂; COOH; C(O)O-C₁₋₆ alkyl or C₃₋₇ cycloalkyl;
Optionally A and R^{1a} form together with the nitrogen to which they are attached to a heterocycle or heterobicycle, optionally substituted with one or more R⁷ which are the same or different;
R⁶, R^{6a} R^{6b} are independently selected from the group consisting ofH; C₁₋₆ alkyl; and T, herein C₁₋₆ alkyl is optionally substituted with one or more halogen, which are the same of different;
T is C₃₋₇ cycloalkyl; heterocyclyl; heterobicyclyl; phenyl; naphthyl; indenyl; indanyl; tetralinyl; decalinyl; or adamantyl, wherein T is optionally substituted with one or more R⁷, which are the same or different;
R⁷ is independently halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R⁸⁸; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), where the ring is at least partially saturated; C(O)R⁸; T¹; or C₁₋₆ alkyl, wherein C₁₋₆ alkyl is optionally substituted with one or more R⁹;
R⁸, R^{8a}, R^{8b} are independently selected from the group consisting ofH; T¹; and C₁₋₆ alkyl, wherein C₁₋₆ alkyl is optionally substituted with one or more R¹⁰;
R⁹, R¹⁰ are independently selected from the group consisting of halogen; CN; COOR¹¹; OR¹¹, C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); and T¹;
R¹¹, R^{11a}, R^{11b} are independently selected from the group consisting of H; C₁₋₆ alkyl; and T¹;
T¹ is C₃₋₇ cycloalkyl; heterocyclyl; heterobicyclyl; phenyl; naphthyl; indenyl; indanyl; tetralinyl; decalinyl; or adamantyl, wherein T¹ is optionally substituted with one or more R¹², wherein R¹² is independently halogen; CN; COOR¹³; OR¹³; C(O)N(R¹³R^{13a}); S(O)₂N¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}; OC(O)N(R¹³R^{13a}); oxo (=O), where the ring is at least partially saturated; C(O)R¹³; C₁₋₆ alkyl; phenyl; C₃₋₇ cycloalkyl; or heterocyclyl, wherein C₁₋₆ alkyl; phenyl; C₃₋₇ cycloalkyl; and heterocyclyl are optionally substituted with one or more halogen, which are the same or different;
R¹³, R^{13a}, R^{13b} are independently selected from the group consisting of H; C₁₋₆ alkyl; phenyl; C₃₋₇ cycloalkyl; and heterocyclyl, wherein C₁₋₆ alkyl; phenyl; C₃₋₇ cycloalkyl; and heterocyclyl are optionally substituted with one or more halogen, which are the same or different;
Z¹ is =O or =S;
Optionally Z¹ is =N-R¹⁵ and R¹⁵, R³ jointly form together with the atoms to which they are attached a heterocycle or heterobicycle, wherein the heterocycle or heterobicycle is optionally substituted with one or more R¹⁶;
Z² is O; S; or N-R¹⁴;
R³, R¹⁴ are independently selected from the group consisting of H; T²; C₁₋₆ alkyl, wherein C₁₋₆ alkyl is optionally substituted with one or more R¹⁷, provided that when one of R³ and R¹⁴ is H and the other is ethyl, R¹⁷ is other than -COOH;
Optionally R³, R¹⁴ jointly form together with the nitrogen to which they are attached a heterocycle or heterobicycle, wherein the heterocycle or heterobicycle is optionally substituted with one or more R¹⁸;
T² is C³⁻⁷ cycloalkyl, heterocyclyl; heterobicyclyl; phenyl; naphthyl; indenyl; indanyl; tetralinyl; decalinyl; or adamantyl, wherein T² is optionally substituted with one or more R¹⁹; provided that when A is phenyl; Y is -S(O)₂NH-, wherein the sulfur is attached to A; R¹, R² are H; X¹, X³, X⁴ are CH; X² is C-R⁴, wherein R⁴ is OCH₃; Z¹ is =O; and Z² is NH, T² is other than unsubstituted phenyl;
R¹⁶, R¹⁸, R¹⁹ are independently selected from the group consisting of halogen; CN; COOR²⁰; OR²⁰; C(O)N(R²⁰R^{20a}); S(O)₂N(R²⁰R^{20a}); S(O)N(R²⁰R^{20a}); S(O)₂R²⁰; N(R²⁰)S(O)₂N(R^{20a}R^{20b}); SR²⁰; N(R²⁰R^{20a}); OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); oxo (=O), where the ring is at least partially saturated; C(O)R²⁰; C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²¹;
R²⁰, R^{20a}, R^{20b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more halogen, which are the same or different;
R¹⁷, R²¹ are independently selected from the group consisting of halogen; CN; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²³;
R²², R^{22a}, R^{22b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more R^{23a};
R²³, R^{23a} are independently selected from the group consisting of halogen; CN; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24a}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); and C₁₋₆ alkyl, wherein C₁₋₆ alkyl is optionally substituted with one or more halogen, which are the same or different;
R²⁴, R^{24a}, R^{24b} are independently selected from the group consisting of H; C₁₋₆ alkyl, wherein C₁₋₆ alkyl is optionally substituted with one or more halogen, which are the same or different

With regard to compounds of formula (I) according to the present invention as such it is preferred that the following compounds are excluded:
- Compounds where Z¹ is =N-R¹⁵ and R¹⁵, R³ jointly form together with the atoms to which they are attached a heterocycle or heterobicycle, wherein the heterocycle or heterobicycle is optionally substituted with one or more R¹⁶, when the heterocycle is 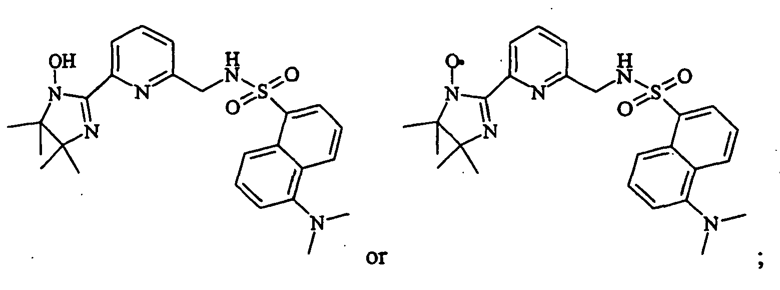
- Compounds, where R³, R¹⁴ jointly form together with the nitrogen to which they are attached a heterocycle or heterobicycle, wherein the heterocycle or heterobicycle is optionally substituted with one or more R¹⁸, when the heterobicycle is unsubstituted or in 3,6 and/or 7 position substituted 1,2,3,4-tetrahydroquinoline and its 4-oxa, thia, aza and/or 8-aza derivatives; and
- N-[1-(3-aminocarbonyl-4-hydroxyphenyl)-2,2,2-trichloroethyl]-4-chlorobenzenesulfonamide.

With regard to compounds of formula (I) according to the present invention for use as a medicament and as only active ingredient of a pharmaceutical composition it is preferred that the following compounds are excluded:
- Compounds, where R³, R¹⁴ jointly form together with the nitrogen to which they are attached a heterocycle or heterobicycle, wherein the heterocycle or heterobicycle is optionally substituted with one or more R¹⁸, when the heterobicycle is unsubstituted or in 3,6 and/or 7 position substituted 1,2,3,4-tetrahydroquinoline and its 4-oxa, thia, aza and/or 8-aza derivatives.

In case a variable or substituent can be selected from a group of different variants and such variable or substituent occurs more than once the respective variants can be the same or different.

Within the meaning of the present invention the terms are used as follows:
"Alkyl" means a straight-chain or branched carbon chain that may contain double or triple bonds. It is generally preferred that alkyl doesn't contain double or triple bonds. Each hydrogen of an alkyl carbon may be replaced by a substituent
"C₁₋₄ alkyl" means an alkyl chain having 1 - 4 carbon atoms, e.g. if present at the end of a molecule: methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, or e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-, when two moieties of a molecule are linked by the alkyl group. Each hydrogen of a C₁₋₄ alkyl carbon may be replaced by a substituent
"C₁₋₆ alkyl" means an alkyl chain having 1 - 6 carbon atoms, e.g. if present at the end of a molecule: C₁₋₄ alkyl, methyl, ethyl, -CH=CH₂, -C≡CH, n-propyl, isopropyl, -CH=H-CH₃, -CH₂-CH=CH₂, n-butyl, isobutyl, -CH=H-CH₂-CH₃, -CH=CH-CH=CH₂, sec-butyl; tert-butyl, n-pentane, n-hexane, or e.g. -CH₂-, -CH₂-CH₂-, -CH=CH-, -CH(CH₃)-, -C(CH₂)-, - CH₂-CH₂-CH₂-, -CH(C₂H₅)-, -CH(CH₃)₂-, when two moieties of a molecule are linked by the alkyl group. Each hydrogen of a C₁₋₆ alkyl carbon may be replaced by a substituent
"C₃₋₇ cycloalkyl" or "C₃₋₇ cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent
"Halogen" means fluoro, chloro, bromo or iodo. It is generally preferred that halogen is fluoro or chloro.
"Heterocyclyl" or "heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.
"Heterobicyclyl" or "heterobicycle" means a heterocycle which is condensed with phenyl, C₃₋₇ cycloalkyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine.

Preferred compounds of formula (I) are those compounds in which one or more of the residues contained therein have the meanings given below, with all combinations of preferred substituent definitions being a subject of the present invention. With respect to all preferred compounds of the formulas (I) the present invention also includes all tautomeric and stereoisomeric forms and mixtures thereof in all ratios, and their pharmaceutically acceptable salts.

In preferred embodiments of the present invention, the substituents A, Y, Z¹, Z², R¹ to R³ and X¹ to X⁴ of the formula (I) independently have the following meaning. Hence, one or more of the substituents A, Y, Z¹, Z², R¹ to R³ and X¹ to X⁴ can have the preferred or more preferred meanings given below.

Preferably, at most three of X¹, X², X³ and X⁴ are N; more preferred at most one of X¹, X², X³ and X⁴ is N.

Preferably, R¹ and R² are H or combined to form =O.

Preferably, R⁴ is F; Cl; Br; CH₃; OCH₃, NH₂; or phenyl.

Preferably, Y is -*S(O)₂-NH-, or -*S(O₂)-N(CH₃)- wherein the atom marked with an asterisk is attached to A.

Preferably, A is phenyl, adamantyl; C₃₋₇ cycloalkyl; heterocyclyl; or C₁₋₆ alkyl, wherein C₁₋₆ alkyl is otionally substituted with T. More preferred, A is phenyl; cyclohexyl; thiophenyl; benzothiophenyl; naphthyl; diazolyl; or pyridyl.

It is also preferred, that A is T and T is optionally substituted with up to 3 R⁷, independently being F; Cl; CH₃; OCH₃; N(CH₃)₂; T¹; OT¹; or NHT¹.

Preferably, T¹ is phenyl, which is optionally substituted with up to three halogens, independently being F; or Cl.

It is also preferred that A is T and T is heterobicyclyl, more preferred T is decahydroquinoline or tetrahydroisoquinoline, wherein the ring nitrogen is directly linked to the sulphur of the sulphonamide group representing Y.

Preferably, Z¹ is =O and Z² is -NR¹⁴.

Preferably, R¹⁴ is H.

Preferably, R³ is phenyl; benzyl; heterocyclyl; C₃₋₇ cycloalkyl; or C₁₋₆ alkyl, wherein C₁₋₆ alkyl is optionally substituted with O-C₁₋₆ alkyl or C₃₋₇ cycloalkyl.

It is also preferred, that R³ is T² and T² is substituted with up to three R¹⁹, independently being F; Cl; CH₃; OCH₃; N(CH₃)₂; COOH; COOCH₃; CF₃; or C(O)NH₂.

Preferably, T² is phenyl; cyclohexyl; or pyridyl.

Preferably, R³, R¹⁴ are independently H or C₁₋₆ alkyl independently selected from the group consisting of ethyl; isopropyl; n-propyl; n-butyl, wherein C₁₋₆ alkyl is optionally substituted with N(CH₃)₂; or N(C₂H₅)₂.

It is also preferred, that Z¹ is =N-R¹⁵ and R¹⁵, R³ jointly form a heterocycle. Preferably, the heterocycle is oxazole; oxadiazole; diazole; or triazole, wherein the heterocycle is optionally substituted with one R¹⁶ being C₁₋₆ alkyl, optionally substituted with N(CH₃)₂ or N(CH₂CH₃)₂; C₃₋₇ cycloalkyl; phenyl; or heterocyclyl.

More preferred, R¹⁶ is methyl; ethyl; iso-propyl; cyclohexyl; phenyl; or piperidinyl.

It is also preferred, that R³, R¹⁴ jointly form a heterocycle. Preferably the heterocycle is piperazine; morpholine; piperidine; or pyrrolidine, wherein the heterocycle is optionally substituted with methyl.

Compounds of the formula (I) in which some or all of the above-mentioned groups have the preferred or more preferred meanings are also an object of the present invention.

Where tautomerism, like e.g. keto-enol tautomerism, of compounds of general formula (I) may occur, the individual forms, like e.g. the keto and enol form, are comprised separately and together as mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) may be obtained from stereoselective synthesis using optically pure starting materials.

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) as 11β-HSD1 inhibitors. The biochemical mechanism, where the 11β-HSD1 enzyme may play a role is described in more detail in WO-A 03/065983 under the paragraph "Biochemical Mechanism" starting on page 18, which paragraph is herewith incorporated by reference.

Accordingly, the tissue specific 11β-HSD1 enzyme and its counterpart 11β-HSD2 may play a role for the modulation of glucocorticoid concentrations. Glucocorticoids (also called corticosteroids) are steroid hormones that play an important role in mammals, including humans. Control or modulation of glucocorticoid activity is important in regulating physiological processes in a wide range of tissues and organs. 11β-HSD1 and 11β-HSD2 have different cofactor requirements and substrate affinities. The 11β-hydroxysteroid dehydrogenase type 2 enzyme (11β-HSD2) is a high affinity enzyme that generally uses NAD⁺ as the preferred cofactor and rapidly dehydrogenates 11β-hydroxyglucocorticoids, such as cortisol, to 11-keto glucocorticoids, such as cortisone. The 11β-hydroxysteroid dehydrogenase type 1 enzyme (11β-HSD1) is a low affinity enzyme that generally uses NADP⁺/NADPH as a cofactor rather than NAD⁺ (Agarwal et al., 1994, J. Biol. Chem., 269: 25959-25962). In vitro studies have shown that 11β-HSD1 is capable of acting as both a reductase and a dehydrogenase. Therefore, selectivity may play a role with regard to inhibitors of 11β-HSD1. However, 11β-HSD1 in vivo mainly acts as a reductase, converting 11-ketoglucocorticoids, such as cortisone, to 11β-hydroxyglucocorticoids, such as cortisol. Therefore 11β-HSD1 regulates the intracellular pool of the insulin antagonists 11β-hydroxyglucocorticoids, such as cortisol in humans, especially in metabolically active tissues such as liver and adipose tissue. Hence, increased 11β-HSD1 activity might be a cause for the development of type 2 diabetes mellitus, metabolic syndrome and related diseases, e. g. obesity, cardiovascular disease and hypertension.

11β-HSD1 enzyme related or modulated diseases are also described in more detail in WO-A 03/065983 under the paragraph "Utilities" starting on page 20, which paragraph is herewith incorporated by reference.

Another disease related to the 11β-HSD1 enzyme is Alzheimer's disease and its inherent mild cognitive impairment (MCI) that is a common feature of aging but is, at present, untreatable (Ritchie, K. & Touchon, J. 2000). MCI and Alzheimer's disease are associated with a poor quality of life and loss of independence in older people and results in huge socioeconomic costs (Fillit, H. et al. 2002). In aged animals and humans, interindividual differences in cognitive function have been ascribed to variations in long-term glucocorticoid exposure (Lupien, S. J. et al. 1998; Seckl, J. R. & Olsson, T. 1995). The hippocampus plays a central role in the formation of long-lasting memories, highly expresses receptors for glucocorticoids in rodents (Reul, J. M. H. M. & de Kloet, E. R. 1985) and humans (Seckl, J. R. et al. 1991), and is particularly sensitive to the deleterious actions of chronic glucocorticoid excess (Landfield, P. W. et al. 1978; Meaney, M. J. et al. 1995). Dysregulation of the hypothalamic-pituitary-adrenal (HPA) axis with resultant chronically increased exposure of the hippocampus to elevated glucocorticoid levels has been hypothesized to contribute to the decline in cognitive function with aging, through the detrimental effects of glucocorticoids on hippocampal neurons (McEwen, B. S. & Sapolsky, R. M. 1995; McEwen, B. S. 1999). In human populations, including those with Alzheimer's disease and normal aging, higher cortisol levels have been associated with poorer memory and hippocampal shrinkage/neuronal loss (Lupien, S. J. et al. 1998; Newcomer, J. W. et al. 1994; de Leon, M. J. et al. 1988). 11β-HSDs are also expressed in the adult rat brain (Moisan, M.-P. et al. 1990; Lakshmi, V. et al. 1991; Sakai, R. R. et al. 1992) where 11β-HSD1 is abundant, but 11β-HSD2 is largely absent (Roland, B. L. et al. 1995). In intact rat hippocampal cells in primary culture, which express solely the 11β-HSD1 isozyme, the reaction catalyzed is reduction. Such reactivation of glucocorticoids by 11β-HSD1 allows intrinsically inert 11-keto steroids to mimic active glucocorticoids in potentiating kainate-induced neurotoxicity (Rajan, V. et al. 1996). Carbenoxolone, which inhibits unspecifically both isozymes of 11β-HSD in vitro and in vivo (Stewart, P. M. et al. 1990), prevents regeneration of glucocorticoids by 11β-HSD1 and thereby protects primary cultures of hippocampal cells from glucocorticoid-mediated exacerbation of excitatory amino acid neurotoxicity (Rajan, V. et al. 1996). Regeneration of glucocorticoids by 11β-HSD1 within neurons seems important in vivo because 11β-HSD1 knockout mice are protected from glucocorticoid-associated hippocampal dysfunction with aging (Yau, J. L. W. et al. 2001). Therefore specific inhibition of 11β-HSD1 might additionally protect against age-dependent cognitive impairment. Furthermore, administration of the 11β-HSD inhibitor carbenoxolone improved verbal fluency in healthy elderly men and improved verbal memory in patients with type 2 diabetes (Sandeep T. C. et al. 2004).

Highly active antiretroviral therapy (HAART) in patients infected with human immunodeficiency virus (HIV) is associated with a poorly understood lipodystrophic and hypertriglyceridaemic syndrome, which resembles Cushing's syndrome, but in which plasma cortisol is not elevated. In adipose tissue of these patients with HAART-associated lipodystrophy, 11β-HSD1 mRNA is increased and its concentration is correlated with features of insulin resistance. Therefore highly active antiretroviral therapy (HAART)-associated lipodystrophy might be explained by increased local regeneration of cortisol from inactive cortisone within adipose tissue, catalyzed by the enzyme 11β-HSD1 (Sutinen J. et al., Diabetologia Epub 29.09.2004). 11β-HSD1 is expressed in both osteoclasts and osteoclasts (J.W. Tomlinson, Minerva Endocrinol Vol 30(2005), 37-46). Sine its activity increases with age in primary cultures of osteoblasts, a role in the pathogenesis of age related bone loss (osteoporosis) is likely (M.S. Cooper et al, J Bone Miner Res Vol17(2002), 979-86).

Glucocorticoids are known to control the proliferation of undifferentiated cells and the differentiation of precursor cells to fully differentiated cells (T.M. Stulnig et al, Diabetologica 47 (2004), 1-11. The activity of 11β-HSD1 may therefore contribute to the development of cancer cells and the growth of tumours.

Abnormal corticosteroid levels enhance the vulnerability of specific hippocampal neurons. Glucocorticoid-receptor immunoactivity in the hippocampus was found higher in seizure sensitive compared to seizure-resistant gerbils (I.K. Hwang et al, Neurosci Res. 53 (2005), 14-24). Controlling glucocorticoid levels through 11β-HSD1 activity may therefore be of therapeutic use for epilepsy.

It is also believed that 11β-HSD1 activity may be of therapeutic use for depression (WO-A 2005/060963; WO-A 2005/047250; WO-A 2005/075471).

Accordingly, the present invention provides compounds of formula (I) or pharmaceutically acceptable salts thereof for use as a medicament

Furthermore, the present invention provides the use of a compound of formula (I) or a pharmaceutically acceptable salt thereof for the manufacture of a medicament for the treatment or prophylaxis of non-insulin dependent diabetes mellitus, hyperglycemia, low glucose tolerance, insulin resistance, β-cell dysfunction, obesity, lipid disorders, dyslipidemia, hyperlipidemia, hypertriglyceridemia, hypercholesterolemia, low HDL levels, high LDL levels, atherosclerosis and its sequelae, vascular restenosis, pancreatitis, abdominal obesity, neurodegenerative disease, retinopathy, nephropathy, neuropathy, Syndrome X, Alzheimer's disease, osteoporosis, cancer, epilepsy, depression, HAART-associated lipodystrophy or other conditions and disorders where insulin resistance is a component or that may be treated by inhibition of the 11β-HSD1 enzyme.

The present invention provides pharmaceutical compositions comprising a compound of formula (I) or a pharmaceutically acceptable salt thereof as active ingredient together with a pharmaceutically acceptable carrier, optionally in combination with one or more other pharmaceutical compositions.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may comprise one or more additional compounds as active ingredients like one or more compounds of formula (I) not being the first compound in the composition or other 11β-HSD1 inhibitors.

Other active ingredients are disclosed, e.g., in WO-A 03/065983 under the paragraph "Combination Therapy" starting on page 27, which paragraph is herewith incorporated by reference.

Accordingly, other active ingredients may be DPP-IV inhibitors; insulin sensitizers, e.g. PPAR agonists and biguanides; insulin and insulin mimetics; sulfonylureas and other insulin secretagogues; α-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents, e.g. HMG-CoA reductase inhibitors, sequestrants, nicotinyl alcohol, nicotinic acid or a salt thereof, PPARγ agonists, PPARα/γ dual agonists, inhibitors of cholesterol absorption, acyl CoA: cholesterol acyltransferase inhibitors, and anti-oxidants; PPAR agonists; antiobesity compounds ; ileal bile acid transporter inhibitors; anti-inflammatory agents; or protein tyrosine phosphatase-1B (PTP-1B) inhibitors.

Other active ingredients may be anti-hypertensive compounds or compounds for the treatment of cardiovascular diseases, particularly atherosclerosis.

The active ingredients may be comprised in one or more different pharmaceutical compositions (combination of pharmaceutical compositions).

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally, for example, as liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) may also be administered parenterally. Solutions or suspensions of these active compounds can be prepared in water suitably mixed with a surfactant such as hydroxypropyl-cellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of formula (I) are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

Some abbreviations that may appear in this application are as follows.

### Abbreviations

### Designation

- Boc: *^{t}*Butoxycarbonyl
- Boc₂O: Di-*tert*-butyl-dicarbonate
- *n*BuLi: *n*-Butyllithium
- DCM: Dichloromethane
- DIPEA: Diisopropylethylamine
- DMAP: Dimethylaminopyridine
- DMF: *N*,*N*-Dimethylformamide
- DPPA: Diphenyl phosphoryl azide
- EDC: 1-(3-Dimethylaminopropyl)-3-ethylcarbodiimide hydrochloride
- EtOAc: Ethyl acetate
- EtOH: Ethanol
- FMPE: 2-(4-Formyl-3-methoxphenoxy)ethyl polystyrene
- HOBt: 1-Hydroxybenzotriazole
- MeOH: Methanol
- STAB: Sodium triacetoxyborohydride
- TBTU: 2-(1H-Benzotriazole-1-yl)-1,1,3,3-tetramethyluronium tetrafluoroborate
- TEA: Triethylamine
- TFA: Trifluoroacetic acid
- THF: Tetrahydrofuran
- TMOF: Trimethylorthoformate
- TMSCHN₂: (Trimethylsilyl)diazomethane

Available starting materials for the synthesis of preferred embodiments of the invention may be amines of formula (II) and sulfonyl chlorides of the formula (III). They may be purchased from commercially available sources such as Array, Sigma Aldrich, Fluka, ABCR or be synthesized by one skilled in the art.

In general preferred compounds of the formula (I) 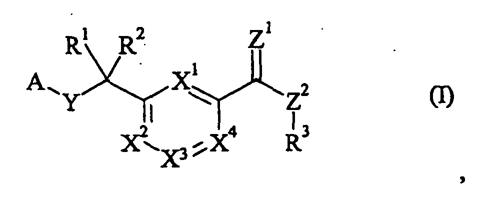 wherein the variables have the above described meanings may be prepared by reducing a compound of formula (II) 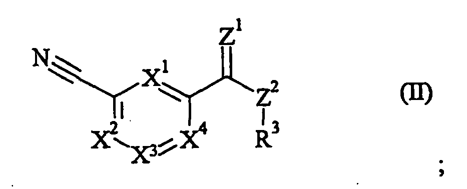 and coupling the resulting amine (III) 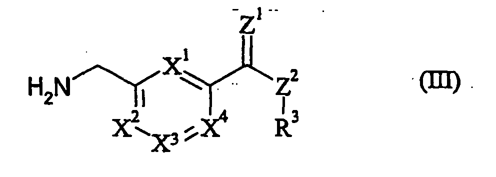 with A-Y-Cl, wherein A is T or C₁-₆ alkyl; or
with A-H and SO₂Cl₂, wherein A is T-N(R^{1c})-.

More specific routes to prepare preferred embodiments of compounds of the present invention are outlined in schemes A to I.

Exemplified conditions for a reaction according to scheme A are given in the experimental section - Route 1

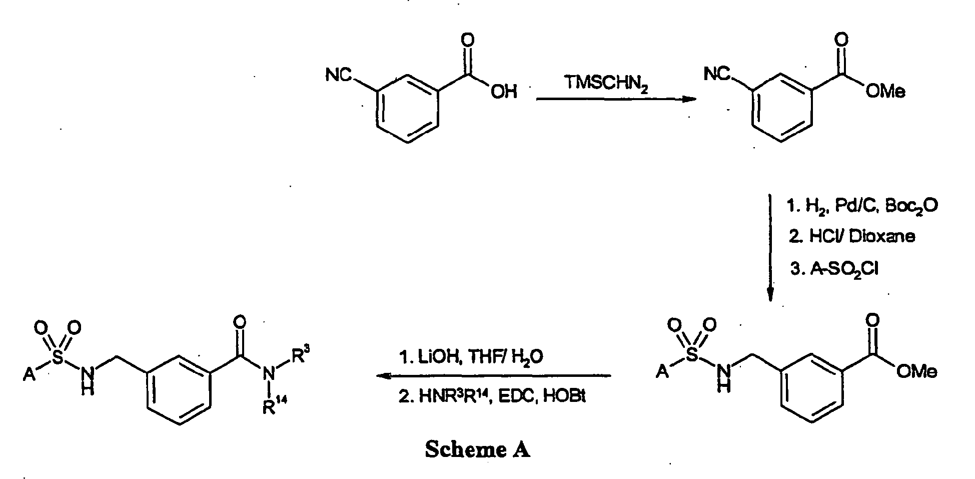

An alternative route to these compounds is being investigated using solid phase reagents (J. Comb. Chem.2000, 2, 66-74, Tetrahedron Lett. 2002, 43, 4741-4745). Key intermediate 1C (prepared as described in route 1) is loaded onto a benzaldehyde resin before the remainder of the chemistry is carried out (scheme B).

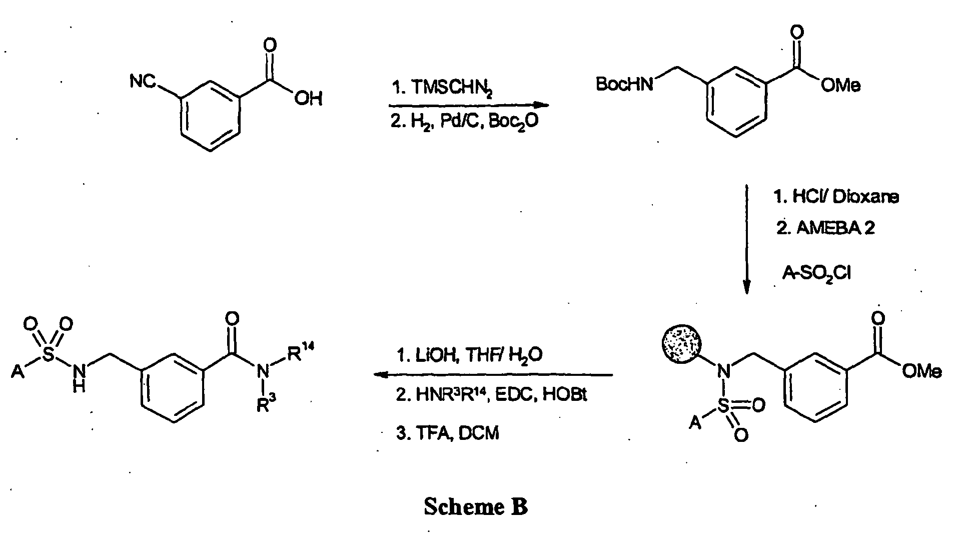

Exemplified conditions for a reaction according to scheme C are given in the experimental section (where R⁴= F) - Route 2

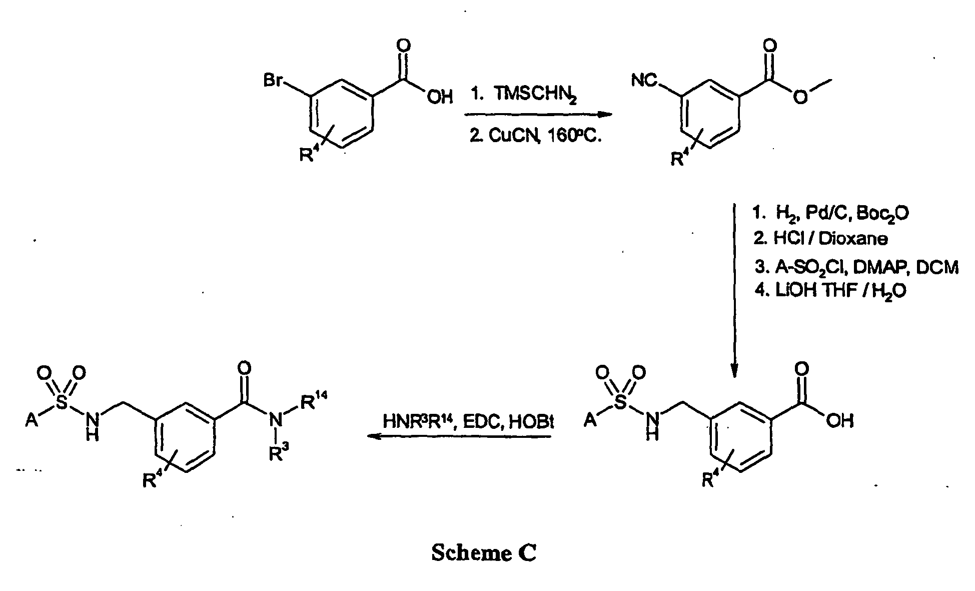

The sulfamide moiety could be introduced using a literature procedure (J. Med. Chem, 1972, 15, 5, 538) starting from key intermediate 1C.

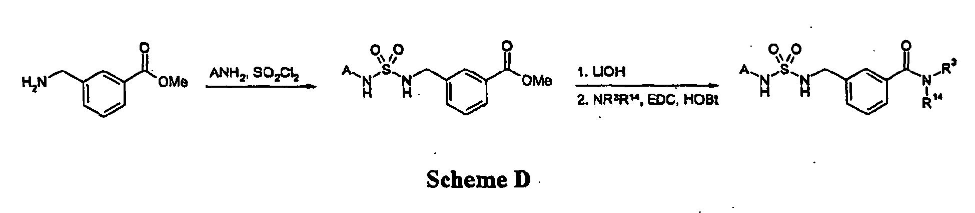

The synthesis of oxadiazoles from the carboxylic acid and amidoximes is described in Tetrahedron Lett. 2001, 42, 1495-1498 and J. Org. Chem. 2003, 68, 7316-7321. The carboxylic acid precursor would be prepared by route 1

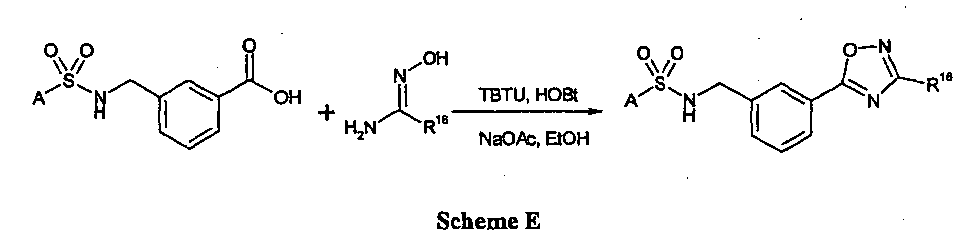

### Pyridyl Replacements for the Benzyl (Central) Ring

Three series of compounds are described below in which the nitrogen occupies a different position in the central ring.

### Series 1

The key nitrile intermediate could be synthesised by reaction of ethyl pyruvate and 4-cyanopyridine, according to literature procedure (Tet. Lett., 1973, 645). It is anticipated that the remainder of the synthesis would utilise methodology already developed for route 1.

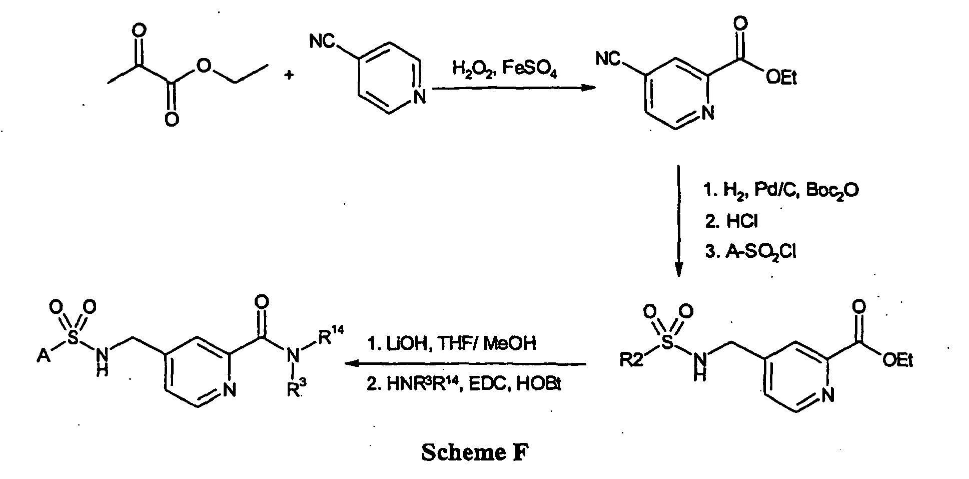

### Series 2

Ethyl 5-bromonicotinate could be reacted with copper cyanide in order to displace the bromine atom and introduce a nitrile. With the nitrile in hand the methodology for the remainder of the route should follow that of route 1.

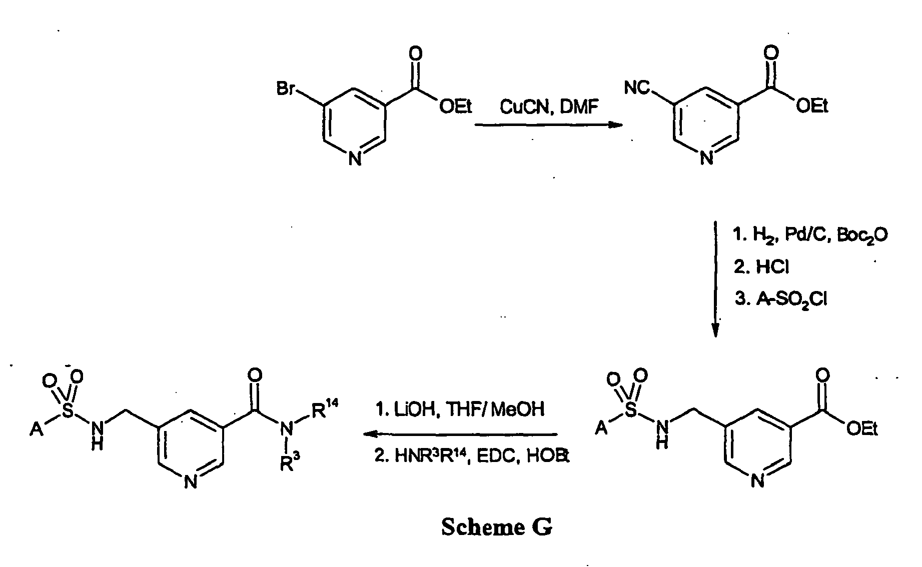

### Series 3

The route for the synthesis of these compounds could be synthesised using the same conditions as for series 2 except that the starting material would be ethyl 2-bromoisonicotinate. Subsequent steps should follow route 1.

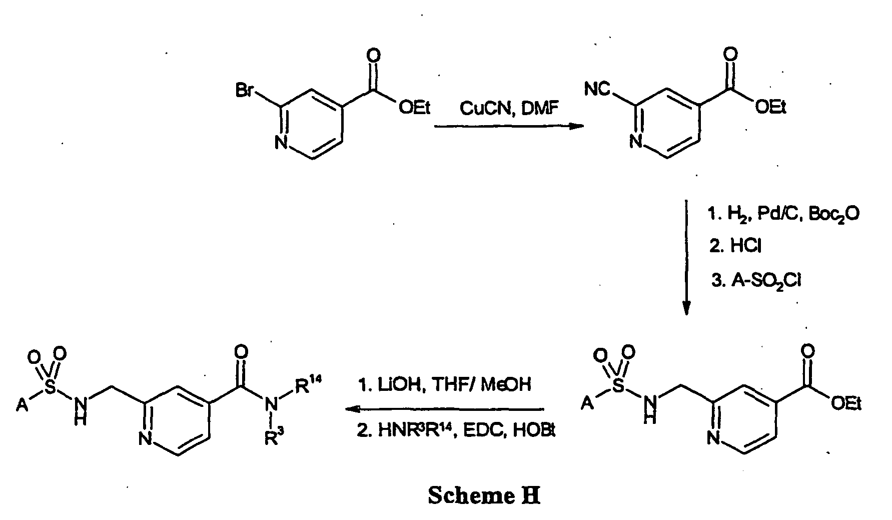

### Pyrimidinyl Replacement for the Benzyl (Central) Ring

The route to the key intermediate in this synthesis will involve a cyanide displacement of the 4-chloro substituent (Bioorg. Med. Chem. Lett. 1999, 9, 1973-1978), subsequent hydrolysis and methyl ester formation will give the desired functionality. In the course of the hydrolysis the 2-chloro substituent may be also hydrolysed which would be reintroduced using POCl₃ followed by a second displacement with sodium cyanide. The remainder of the route will then use the same methodology as that described for route 1.

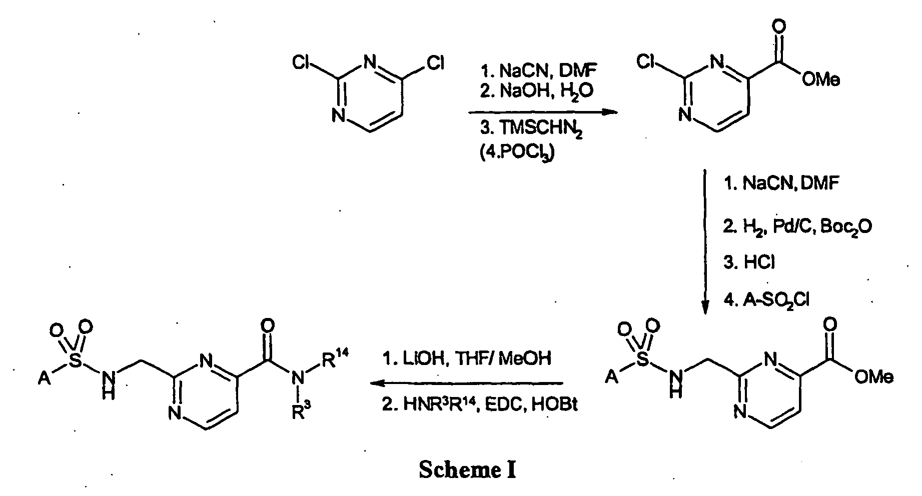

### Examples

### Example 1: Preparation of 3-[(3-Chloro-2-methylbenzene sulfonylamino)-methyl]-N, N-diethylbenzamide (Route 1)

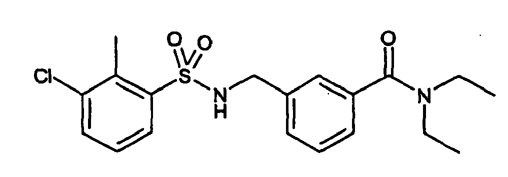

### 1A. 3-Cyano-benzoic acid methyl ester

To a solution of 3-cyanobenzoic acid (7.35 g, 50 mmol) in DCM/ MeOH (80 ml/ 20 ml) is added trimethylsilyldiazomethane (2M solution in ether; 1 eq, 50 mmol) and the mixture is stirred for 1 h. Acetic acid is added dropwise until the yellow colour disappeared. The solvents are then removed *in vacuo*. The residue is dissolved in DCM and washed with water. The organic phase is dried (MgSO₄) and concentrated *in vacuo* to give the title compound (5 g, 62%) LC/MS: 93% MH⁺, m/z not ionised, Rt = 1.14 mins.

### 1B. 3-(tert-Butoxycarbonylamino-methyl) benzoin acid methyl ester

To a solution of title compound 1A, 3-cyano-benzoic acid methyl ester, (5.80 g, 36.0 mmol) in ethyl acetate (25 ml) in a 500ml pressure bomb is added di-*tert*-butyl-dicarbonate (1.2 eq, 43.2 mmol) and 10% palladium on carbon (580 mg) the vessel is charged with hydrogen at 5 atm and left to stir overnight. The vessel is recharged with hydrogen and left to stir for a further 5 h. The mixture is filtered through celite, the filtrate is concentrated in *vacuo* to give the title compound (10.01 g, >100% starting material remaining). LC/MS: >75% MH⁺, m/z not ionised, Rt = 1.36 mins.

### 1C. 3-Aminomethyl-benzoic acid methyl ester

To a solution of title compound 1B, 3-(*tert*-butoxycarbonylamino-methyl) benzoic acid methyl ester, (4 g, 15.1 mmol) in DCM (40 ml) is added 4M HCl in dioxane (4 eq, 60.4 mmol) and the mixture is stirred at room temperature for 4.5 h. The solvents are removed *in vacuo*. The residue is redissolved in DCM and carbonate resin is added and the mixture is stirred at room temperature overnight. The mixture is filtered and the solvent is removed *in vacuo* to give the title compound which is used without purification. LC/MS: 86% MH⁺, m/z 166, Rt = 0.69 mins.

### 1D. 3-[(3-Chloro-2-methylbenzene sulfonylamino)-methyl]-benzoic acid methyl ester

To a solution of title compound 1C, 3-aminomethyl-benzoic acid methyl ester, (1 g, 6.06 mmol) and 3-chloro-2-methylphenylsulfonyl chloride (1.1 eq, 6.66 mmol) in DCM (30 ml) is added pyridine (3 eq, 18.18 mmol). The mixture is allowed to stir at room temperature for 16 h. The solvent is removed *in vacuo*. The residue is redissolved in DCM and is washed with water. The organic layer is acidified with 1M HCl and washed with DCM. The aqueous phase is basified with 1M NaOH solution and extracted with DCM. The organic phases are dried (Na₂SO₄) and concentrated *in vacuo* to give the title compound (1.33 g, 62%). LC/MS: 55% MH⁺, m/z 354, Rt = 1.49 mins.

### 1E. 3-[(3-Chloro-2-methylbenzene sulfonylamino)-methyl]-benzoic acid

To a solution of title compound 1D, 3-[(3-chloro-2-methylbenzene sulfonylamino)-methyl]-benzoic acid methyl ester, (1.13 g, 3.2 mmol) in THF/ MeOH (3:2 50 ml) is added lithium hydroxide (5 eq, 16.06 mmol) and the mixture is heated to 50°C for 32 h. After cooling, the solvents are removed *in vacuo*. The residue is dissolved in ethyl acetate and water. The aqueous phase is washed with ethyl acetate. The aqueous phase is acidified with 2M HCl and re-extracted with ethyl acetate. The combined organic phases are dried (Na₂SO₄) and concentrated *in vacuo* to give the title compound (0.91 g, 83%).

### 1F. 3-[(3-Chloro-2-methyl-benzene sulfonylamino)-methyl]- N, N-diethylbenzamide

To a solution of title compound 1E, 3-[(3-chloro-2-methylbenzene sulfonylamino)-methyl]-benzoic acid, (0.6 g, 1.77 mmol) in THF (9 ml) is added thionyl chloride (4 eq, 7.07 mmol) and a few drops of DMF, the mixture is left to stir at room temperature. After completion the reaction mixture is concentrated *in vacuo*. Diethylamine (1 eq, 0.42 mmol) is dissolved in DCM (2 ml) and a solution of the acid chloride (150 mg, 0.42 mmol) in DCM (3 ml) is added and the mixture is stirred at room temperature overnight. The reaction mixture is concentrated *in vacuo*. The crude product is purified by column chromatography with DCM/ MeOH (gradient 0-2%) as the eluent to give the title compound (77.6 mg, 47%). LC/MS: 94% MH⁺, m/z 395, Rt = 1.41 mins.

### Example 2: Preparation of N, N-Diethyl-3-fluoro-5-[(4'-fluoro-biphenyl-4-sulfonylamino)-methyl]-benzamide (Route 2)

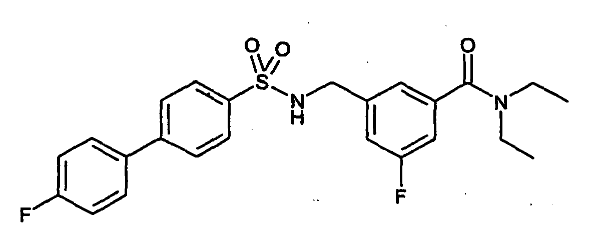

### 2A. 3-Bromo-5-fluorobenzoic acid methyl ester

To a solution of 3-bromo-5-fluorobenzoic acid (2 g, 9.13 mmol) in DCM/ MeOH (20 ml/ 5 ml) is added trimethylsilyldiazomethane (2M solution in ether; 1 eq, 9.13 mmol) and the mixture is stirred for 1 h. Acetic acid was added dropwise until the yellow colour disappeared. The solvents are then removed *in vacuo*. The residue is dissolved in DCM and washed with K₂CO₃. The organic phase is dried (MgSO₄) and concentrated *in vacuo* to give the title compound (1.5 g, 71%) LC/MS: 91% MH⁺, m/z not ionised, Rt = 1.55 mins.

### 2B. 3-Cyano-5-fluorobenzoic acid methyl ester

To a solution of title compound 2A, 3-bromo-5-fluorobenzoic acid methyl ester, (1 g, 3 mmol) in degassed DMF (5 ml) in a schlenk tube is added copper cyanide (1.1 eq, 3.3 mmol). The mixture is heated to 160°C for 18 h. After cooling the reaction mixture is poured into 1M K₂CO₃ and ethyl acetate. To improve separation NH₄OH solution (5 ml) is added and the aqueous phase is re-extracted with ethyl acetate. The combined organic phases are dried (MgSO₄) and concentrated *in vacuo*. The crude product is purified by column chromatography with DCM/ MeOH (99:1) as the eluent to give the title compound (0.47 g, 60%) LC/MS: 100% MH⁺, m/z not ionised, Rt =1.24 mins.

### 2C. 3-(tert-Butoxycarbonylamino-methyl)-5-fluoro-benzoic acid methyl ester

To a solution of title compound 2B, 3-cyano-5-fluorobenzoic acid methyl ester, (0.8 g, 4.47 mmol) in ethyl acetate (20 ml) is added di-*tert*-butyl-dicarbonate (1.2 eq, 5.36 mmol) and 10% palladium on carbon (0.1 eq) the vessel is charged with hydrogen and left to stir overnight. The vessel is recharged with hydrogen and left to stir for a further 5 h. The mixture is filtered through celite, the filtrate is concentrated *in vacuo*. The crude product is purified by column chromatography with heptane/ ethyl acetate (85:15) to give the title compound (0.41 g, 32%). LC/MS: 94% MH⁺, m/z not ionised, Rt = 1.43 mins.

### 2D. 3-Aminomethyl-5-fluoro-benzoic acid methyl ester

To a solution of title compound 2C, 3-(*tert*-butoxycarbonylamino-methyl)-5-fluorobenzoic acid methyl ester, (0.41 g, 1.45 mmol) in DCM (10 ml) is added 4M HCl in dioxane (4 eq, 5.65 mmol) and the mixture is stirred at room temperature for 4.5 h. The solvents are removed *in vacuo*. The residue is redissolved in DCM and carbonate resin is added and the mixture is stirred at room temperature overnight. The mixture is filtered and the solvent is removed *in vacuo* to give the title compound (0.25 g, 94%). LC/MS: 48% MH⁺, m/z 184, Rt = 0.76 mins.

### 2E. 3-Fluoro-5-[(4'-fluoro-biphenyl-4-sulfonylamino)-methyl] benzoic acid methyl ester

To a solution of title compound 2D, 3-aminomethyl-5-fluoro-benzoic acid methyl ester, (0.25 g, 1.37 mmol) and 4-fluorobiphenylsulfonyl chloride (1.0 eq, 1.37 mmol) in DCM (10 ml) is added DMAP (1.1 eq, 1.50 mmol). The mixture is allowed to stir at room temperature for 16 h. The solvent is removed *in vacuo* to give the title compound (0.57 g, 100%). LC/MS: 48% MH⁺, m/z 418, Rt = 1.57 mins.

### 2F. 3-Fluoro-5-[(4'-fluoro-biphenyl-4-sulfonylamino)-methyl] benzoic acid

To a solution of title compound 2E, 3-fluoro-5-[(4'-fluoro-biphenyl-4-sulfonylamino)-methyl] benzoic acid methyl ester, (0.57 g, 1.37 mmol) in THF/ H₂O (2:1 5ml):2.5 ml) is added lithium hydroxide (2 eq, 2.73 mmol) and the mixture is stirred at room temperature for 5 h. The solvents are removed *in vacuo*. The residue is dissolved in DCM and water. The aqueous phase is washed with DCM. The aqueous phase is acidified with 1M HCl to pH= 4 and re-extracted with DCM. A solid precipitates from the DCM phase which is collected by filtration to give the title compound (0.16 g, 29%). LC/MS: 100% MH⁺, m/z 404, Rt = 1.40 mins.

### 2G. N, N-Diethyl-3-fluoro-5-[(4'-fluoro-biphenyl-4-sulfonylamino)-methyl]-benzamide

To a solution of title compound 2F, 3-fluoro-5-[(4'-fluom-biphenyl-4-sulfonylamino)-methyl] benzoic acid, (0.08 g, 0.19 mmol) in DCM (2 ml) is added EDC (1 eq, 0.19 mmol) and HOBt (1 eq, 0.19 mmol). Diethylamine (1 eq, 0.19 mmol) is added and the mixture is stirred at room temperature for 18 h. The solvent is removed *in vacuo*. The crude product is purified by column chromatography with DCM/ MeOH (gradient 0.5-1 %) as the eluent to give the title compound (22 mg, 25%). LC/MS: 100% MH⁺, m/z 459, Rt = 1.52 mins.

### Example 3

Synthesis of 3-Aminomethyl-benzoic acid methyl ester - Intermediate 1C.

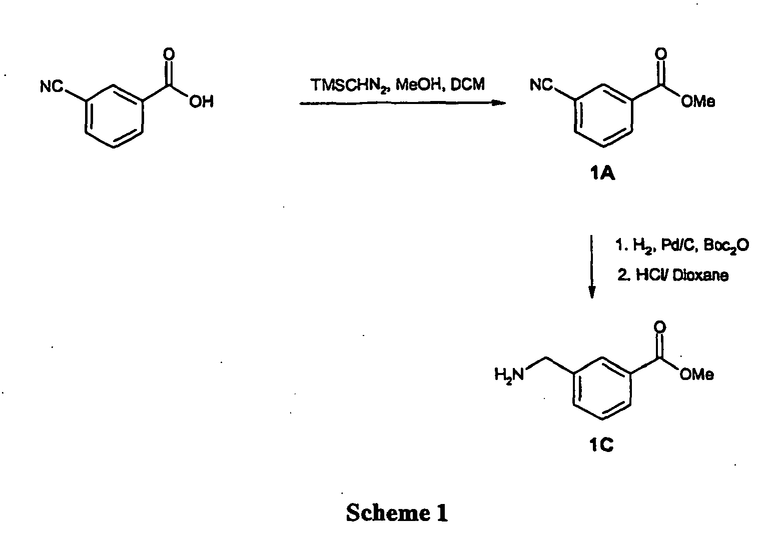

### 1A. 3-Cyano-benzoic acid methyl ester

To a solution of 3-cyanobenzoic acid (7.35 g, 50 mmol) in DCM/ MeOH (80 ml/ 20 ml) was added trimethylsilyldiazomethane (2M solution in ether; 1 eq) and the mixture was stirred for 1 h. Acetic acid was added dropwise until the yellow colour disappeared. The solvents were then removed *in vacuo*. The residue was dissolved in DCM and washed with water. The organic phase was dried (MgSO₄) and concentrated *in vacuo* to give the title compound (5 g, 62%) LC/MS: 93% MH⁺, m/z not ionised, Rt = 1.14 mins.

### 1B. 3-(tert-Butoxycarbonylaminomethyl) benzoic acid methyl ester

To a solution of title compound 1A, 3-cyano-benzoic acid methyl ester, (5.80 g, 36.0 mmol) in ethyl acetate (25 ml) in a 500ml pressure bomb was added di-tert-butyl-dicarbonate (1.2 eq) and 10% palladium on carbon (580 mg) and the vessel was charged with hydrogen at 5 atm and stirred overnight. The vessel was recharged with hydrogen and stirred for a further 5 h. The mixture was filtered through celite, the filtrate was concentrated *in vacuo* to give the title compound (10.01 g, >100% starting material remaining). LC/MS: >75% MH⁺, m/z not ionised, Rt = 1.36 mins.

### 1C. 3-Aminomethyl-benzoic acid methyl ester

To a solution of title compound 1B, 3-(*tert*-butoxycarbonylaminomethyl) benzoic acid methyl ester, (4 g, 15.1 mmol) in DCM (40 ml) was added 4M HCl in dioxane (4 eq) and the mixture was stirred at room temperature for 4.5 h. The solvents were removed *in vacuo*. The residue was redissolved in DCM and carbonate resin was added and the mixture was stirred at room temperature overnight. The mixture was filtered and the solvent was removed *in vacuo* to give the title compound which was used without purification. LC/MS: 86% MH⁺, m/z 166, Rt = 0.69 mins.

### Synthetic Route 1 - Solution Phase Synthesis

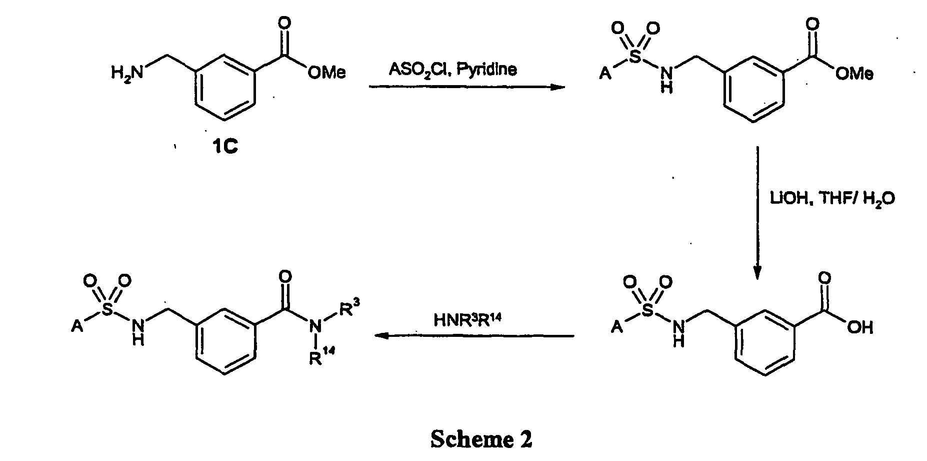

### Method D - Sulfonamide formation.

To a solution of 3-aminomethyl-benzoic acid methyl ester 1C (6.06 mmol) and sulfonyl chloride (1.2 eq) in DCM (30 ml) was added pyridine (20 eq) and the mixture was stirred at room temperature for 16 h. The solvent was removed *in vacuo* and the residue was redissolved in DCM and was washed with water. The organic phase was dried (Na₂SO₄) and concentrated *in vacuo*. The crude mixture was purified by column chromatography with DCM/ EtOAc as the eluent to give the title compound.

### Method E - Saponification.

To a solution of title intermediate D (3.2 mmol) in THF/ H₂O (3:2 50 ml) was added lithium hydroxide (2 eq) and the mixture was heated to 50°C for 5 h. After cooling, the solvents were removed *in vacuo* and the residue was dissolved in ethyl acetate and water. The aqueous phase was acidified to pH=2 with 1M HCl and extracted with ethyl acetate. The combined organic phases were dried (MgSO₄) and concentrated *in vacuo* to give the title compound.

### Method F - Amide Formation.

To a solution of title intermediate E (1.77 mmol) in THF (9 ml) was added thionyl chloride (4 eq) and a few drops of DMF, the mixture was stirred at room temperature. After completion the reaction mixture was concentrated *in vacuo*. The amine (1 eq) was dissolved in DCM (2 ml) and a solution of the acid chloride (0.42 mmol) in DCM (3 ml) was added and the mixture was stirred at room temperature overnight. The reaction mixture was concentrated *in vacuo*. The crude product was purified by column chromatography with DCM/ MeOH as the eluent to give the title compound.

### Method G - Amide Formation.

To a solution of title intermediate E (0.45 mmol) in DMF/ DCM (1:2, 4.5 ml) was added HBTU (1.2 eq) and HOBt (1.2 eq) and the mixture was stirred at room temperature for 10 mins. The amine (1.2 eq) was added and the mixture was stirred at room temperature overnight. The mixture was diluted with DCM and washed with brine. The organic phase was dried (Na₂SO₄) and concentrated *in vacuo*. The crude product was purified by column chromatography with DCM/ MeOH as the eluent to give the title compound.

### Method H - Amide Formation.

To a solution of title intermediate E (0.45 mmol) in DMF/ DCM (1:2, 4.5 ml) was added HBTU (1.2 eq) and HOBt (1.2 eq) and the mixture was stirred at room temperature for 10 mins. The HCl salt of the amine (1.2 eq) and DIPEA (1.2 eq) were added and the mixture was stirred at room temperature overnight. The mixture was diluted with DCM and washed with brine. The organic phase was dried (Na₂SO₄) and concentrated *in vacuo*. The crude product was purified by column chromatography with DCM/ MeOH as the eluent to give the title compound.

Compounds synthesised by route 1 are detailed below:

### 1D. 3-[(Biphenyl-4-sulfonylamino)-methyl]-benzoic acid methyl ester

The compound was synthesised using method D to give title compound 1D (3.21 g, 32%) LC/MS: 98% MH⁺, m/z 382, Rt = 1.56 mins.

### 1E. 3-[(Biphenyl-4-sulfonylamino)-methyl]-benzoic acid

The compound was synthesised using method E to give title compound 1E (3.67 g, 100%) LC/MS: 100% MH⁺, m/z 368, Rt = 1.40 mins.

### 1F1. 3-[(Biphenyl-4-sulfonylamino)-methyl]-N,N-diethyl-benzamide

The compound was synthesised using method F to give title compound 1F1 (10.5 mg, 6%) LC/MS: 100% MH⁺, m/z 423, Rt = 1.51 mins.

### 1F2. Biphenyl-4-sulfonic acid 3-(morpholine-4-carbonyl)-benzylamide

The compound was synthesised using method F to give title compound 1F2 (15.8 mg, 9%) LC/MS: 96% MH⁺, m/z 437, Rt = 1.36 mins.

### 1F3. Biphenyl-4-sulfonic acid 3-(4-methyl-piperazine-1-carbonyl)-benzylamide

The compound was synthesised using method F to give title compound 1F3 (15.7 mg, 8%) LC/MS: 100% MH⁺, m/z 450, Rt = 1.17 mins.

### 2D. 3-[(3-Chloro-2-methyl-benzenesulfonylamino)-methyl]-benzoic acid methyl ester

The compound was synthesised using method D to give title compound 2D (0.34 g, 38%) LC/MS: 96% MH⁺, m/z 354, Rt = 1.55 mins.

### 2E. 3-[(3-Chloro-2-methyl-benzenesulfonylamino)-methyl]-benzoic acid

The compound was synthesised using method E to give title compound 2E (0.14 g, 100%) LC/MS: 84% MH⁺, m/z 340, Rt = 1.35 mins.

### 2F1. 3-[(3-Chloro-2-methyl-benzenesulfonylamino)-methyl]-N,N-diethyl-benzamide

The compound was synthesised using method F to give title compound 2F1 (10.7 mg, 6%) LC/MS: 94% MH⁺, m/z 395, Rt = 1.41 mins.

### 2G2. 3-[(3-Chloro-2-methyl-benzenesulfonylamino)-methyl]-N-(4-triuoromethylbenzyl)-benzamide

The compound was synthesised using method F to give title compound 2G2 (34.4 mg, 34%) LC/MS: 96% MH⁺, m/z 497, Rt = 2.41 mins.

### 2G3. 4-({3-[(3-Chloro-2-methyl-benzenesulfonylamino)-methyl]-benzoylamino}-methyl)-benzoic acid methyl ester

The compound was synthesised using method G to give title compound 2G3 (105.5 mg, 100%) LC/MS: 65% MH⁺, m/z 486, Rt = 2.13 mins.

### 3D. 3-[(Naphthalene-2-sulfonylamino)-methyl]-benzoic acid methyl ester

The compound was synthesised using method D to give title compound 3D (1.93 g, 42%) LC/MS: 90% MH⁺, m/z 356, Rt = 1.47 mins.

### 4D. 3-[(Benzo[b]thiophene-2-sulfonylamino)-methyl]-benzoic acid methyl ester

The compound was synthesised using method D to give title compound 4D (1.5 g, 41%) LC/MS: 95% MH⁺, m/z 362, Rt = 1.54 mins.

### 5D. 3-[(5-Chloro-3-methyl-benzo[b]thiophene-2-sulfonylamino)-methyl]-benzoic acid methyl ester

The compound was synthesised using method D to give title compound 5D (2.7 g, 44%) LC/MS: 86% MH⁺, m/z not ionised, Rt = 1.68 mins.

### 5E. 3-[(5-Chloro-3-methyl-benzo[b]thiophene-2-sulfonylamino)-methyl]-benzoic acid

The compound was synthesised using method E to give title compound 5E (1.4 g, >100%) LC/MS: 76% MH⁺, m/z 396, Rt = 1.50 mins.

### 5G1. 3-[(5-Chloro-3-methyl-benzo[b]thiophene-2-sulfonylamino)-methyl]-N,N-diethyl-benzamide

The compound was synthesised using method G to give title compound 5G1 (23 mg, 40%) LC/MS: 100% MH⁺, m/z 451, Rt = 2.35 mins.

### 5G2. 3-[(5-Chloro-3-methyl-benzo[b]thiophene-2-sulfonylamino)-methyl]-N-cyclohexyl-benzamide

The compound was synthesised using method G to give title compound 5G2 (22 mg, 37%) LC/MS: 82% MH⁺, m/z 477, Rt = 2.47 mins

### 5G3. 4-({3-[(5-Chloro-3-methyl-benzo[b]thiophene-2-sulfonylamino)-methyl]-benzoylamino}-methyl)-benzoic acid methyl ester

The compound was synthesised using method G to give title compound 5G3 (22 mg, 32%) LC/MS: 98% MH⁺, m/z 543, Rt = 2.38 mins

### Synthetic Route 2

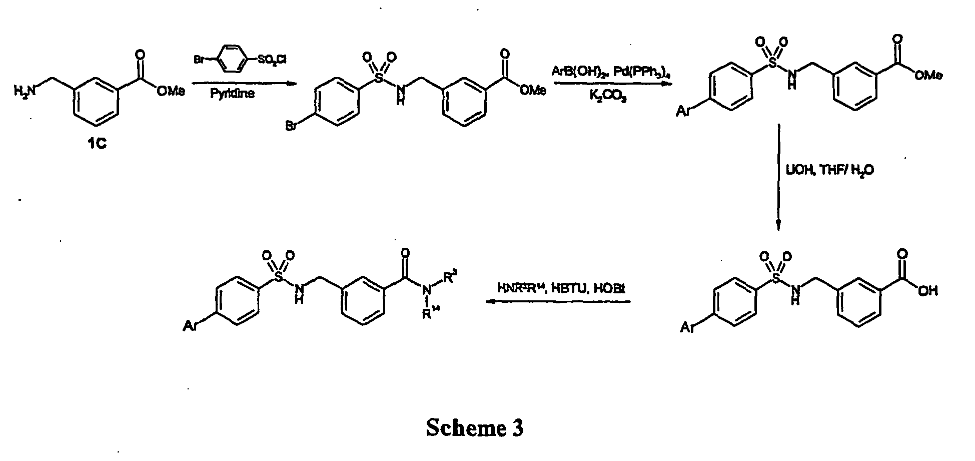

### Method I - 3-[(4-Bromo-benzenesulfonylamino)-methyl]-benzoic acid methyl ester

To a solution of title intermediate 1C (3 g, 14.88 mmol) and 4-bromophenylsulfonyl chloride (1.2 eq) in DCM (120 ml) was added pyridine (20 eq) and the mixture was allowed to stir at room temperature for 16 h. The solvent was removed in *vacuo* and the residue was redissolved in DCM and washed with water. The organic phase was dried (Na₂SO₄) and concentrated *in vacuo*. The crude mixture was purified by column chromatography with DCM as the eluent to give the title compound (2.51 g, 44%).
LC/MS: 85% MH⁺, m/z 386, Rt = 1.52 mins.

### Method J - Suzuki reaction.

A solution of the sulfonamide intermediate I (110 mg, 0.29 mmol) and the boronic acid (1.5 eq) in toluene/ EtOH (2:1; 2 ml):1 ml) was degassed under argon. Pd(PPh₃)₄ (0.1 eq) and potassium carbonate (1.5 eq) were added and the mixture was degassed under argon and the mixture was heated to 95°C for 16 h. After cooling the mixture was filtered through celite and concentrated *in vacuo*. The crude residue was purified by column chromatography with DCM as the eluent to give the title compound.

### Method E - Saponification.

As detailed in synthetic route 1 to give intermediate E.

### Method G - Amide Formation.

As detailed in synthetic route 1 to give compound G.

Compounds synthesised by route 2 are detailed below:

### 6J. 3-[(4'-Cyano-biphenyl-4-sulfonylamino)-methyl]-benzoic acid methyl ester

The compound was synthesised using method J to give title compound 6J (0.76 g, 31%) LC/MS: 60% MH⁺, m/z 407, Rt = 2.16 mins.

### 6E. 3-[(4'-Cyano-biphenyl-4-sulfonylamino)-methyl]-benzoic acid

The compound was synthesised using method E to give title compound 6E (0.24 g, 68%) MS: m/z 392

### 6G1. 3-[(4'-Cyano-biphenyl-4-sulfonylamino)-methyl]-N,N-diethyl-benzamide

The compound was synthesised using method G to give title compound 6G1 (24 mg, 42%) LC/MS: 100% MH⁺, m/z 448, Rt = 2.20 mins.

### 6G2. 3-[(4'-Cyano-biphenyl-4-sulfonylamino)-methyl]-N-cyclohexyl-benzamide

The compound was synthesised using method G to give title compound 6G2 (24 mg, 40%) LC/MS: 100% MH⁺, m/z 474, Rt = 2.30 mins.

### 6G3. 3-[(4'-Cyano-biphenyl-4-sulfonylamino)-methyl]-N-cyclohexylmethyl-benzamide

The compound was synthesised using method G to give title compound 6G3 (31 mg, 50%) LC/MS: 100% MH⁺, m/z 488, Rt = 2.39 mins.

### 6G4. 4-({3-[(4'-Cyano-biphenyl-4-sulfonylamino)-methyl]-benzoylamino}-methyl)-benzoic acid

The compound was synthesised using method G to give title compound 6G4 (24 mg, 36%) LC/MS: 88% MH⁺, m/z 526, Rt = 2.06 mins.

### Synthetic Route 3 - Solid Phase Synthesis

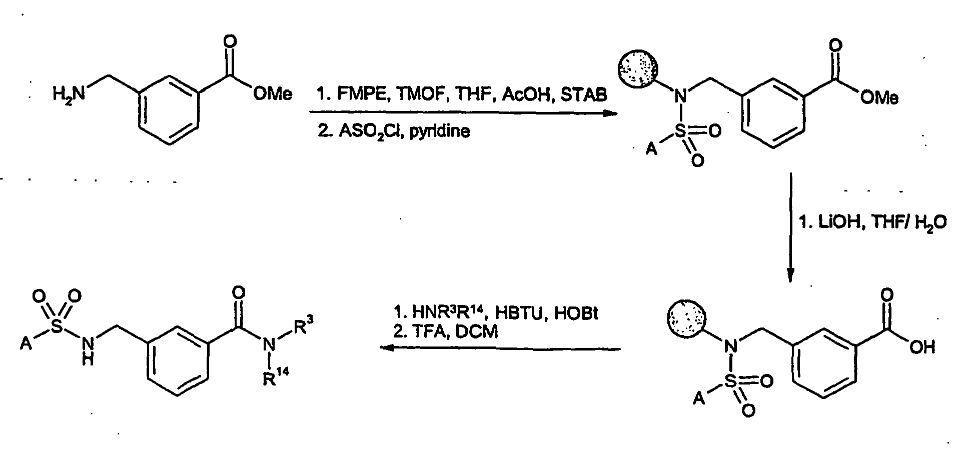

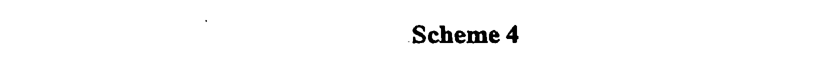

### Method K- Resin Loading

The HCl salt of 3-aminomethyl-benzoic acid methyl ester 1C was dissolved in saturated sodium hydrogen carbonate and DCM. The phases were separated and the aqueous phase was extracted with DCM. The organic phases were dried (MgSO₄) and concentrated *in vacuo* to liberate the free amine.

A solution of 3-aminomethyl-benzoic acid methyl ester 1C (9.1 mmol, 1.5 eq), FMPE resin (1 eq) and acetic acid (1 eq) in THF (50 ml) and TMOF (5 ml) was shaken at room temperature for 45 min. STAB (1.5 eq) was added and the mixture was shaken at room temperature overnight. The resin was filtered and washed with MeOH, water, DMF, [MeOH, DCM] x2 and the resin was dried *in vacuo*.

### Method L- Sulfonamide Formation

To a suspension of resin loaded 3-aminomethyl-benzoic acid methyl ester K (0.13 mmol) in DCM (4 ml) and pyridine (10 eq) was added the sulfonyl chloride (1.5 eq) and the mixture was shaken at room temperature overnight. A small sample of resin was washed with DMF, MeOH, DCM and stained with chloranil to determine the presence of free NH. On completion the resin was filtered and washed with DCM, MeOH, [DMF, DCM, MeOH] x3, DCM, MeOH and the resin was dried *in vacuo*.

### Method M- Saponification

To a suspension of resin L (0.65 mmol) in 1,4-dioxane (20 ml) was added 1M lithium hydroxide (20 eq) and the mixture was shaken at 50°C overnight. A small sample of resin was cleaved with 60% TFA/ DCM and analysed by LCMS. On completion the resin was filtered and washed with [water, DMF] x2, [DCM, MeOH] x3 and the resin was dried *in vacuo*.

### Method N- Amide Formation

To a suspension of resin M (0.24 mmol) in DMF (5 ml) was added HBTU (5 eq) and HOBt (5 eq) and the mixture was left to shake at room temperature for 10 mins. The amine (5 eq) was added and the mixture was shaken at room temperature overnight. A small sample of the resin was cleaved using 40% TFA in DCM and analysed by LCMS. If reactions were incomplete a further 5 eqs of HBTU, HOBt and amine were added and shaking continued for a further 24 hr. Once the reactions were complete the resin was filtered and washed with DMF, 5% Na₂CO₃ (aq), H₂O, H₂O/ acetone, MeOH, DCM, MeOH, DCM, MeOH. The final compounds were cleaved from resin with 60% TFA in DCM (2ml, x3) and the resulting solutions were concentrated. Typically the crude mixtures were purified by column chromatography with DCM/ EtOAC (up to 20% EtOAc) as the eluent to give the final compound.

Compounds synthesised using route 3 are detailed below:

### 7M. PS- 3-[(Biphenyl-4-sulfonylamino)-methyl]-benzoic acid

The compound was synthesised using methods L and M to give title compound 7M.

### 7N1. Biphenyl-4-sulfonic acid 3-(piperidine-1-carbonyl)-benzylamide

The compound was synthesised using method N to give title compound 7N1 (2.3 mg, 4%) LC/MS: 100% MH⁺, m/z 435, Rt = 1.55 mins.

### 7N2. N-Benzyl-3-[(biphenyl-4-sulfonylamino)-methyl]-benzamide

The compound was synthesised using method N to give title compound 7N2 (1.9 mg, 3%) LC/MS: 100% MH⁺, m/z 457, Rt = 1.73 mins.

### 7N3. 3-[(Biphenyl-4-sulfonylamino)-methyl]-N-methyl-benzamide

The compound was synthesised using method N to give title compound 7N3 (0.9 mg, 1%) LC/MS: 100% MH⁺, m/z 381, Rt = 1.45 mins.

### 7N4. 3-[(Biphenyl-4-sulfonylamino)-methyl]-N-(3-methoxy-propyl)-benzamide

The compound was synthesised using method N to give title compound 7N4 (5.8 mg, 7%) LC/MS: 100% MH⁺, m/z 439, Rt = 1.38 mins.

### 7N5. 3-[(Biphenyl-4-sulfonylamino)-methyl]-N-cyclohexyl-benzamide

The compound was synthesised using method N to give title compound 7N5 (7 mg, 9%) LC/MS: 100% MH⁺, m/z 449, Rt = 1.57 mins.

### 7N6. 3-[(Biphenyl-4-sulfonylamino)-methyl]-N-cyclohexylmethyl-benzamide

The compound was synthesised using method N to give title compound 7N6 (2.6 mg, 3%) LC/MS: 100% MH⁺, m/z 463, Rt = 1.69 mins.

### 7N7. 3-[(Biphenyl-4-sulfonylamino)-methyl]-N-p-tolyl-benzamide

The compound was synthesised using method N to give title compound 7N7 (5.8 mg, 7%) LC/MS: 95% MH⁺, m/z 457, Rt = 2.21 mins.

### 7N8. 4-({3-[(Biphenyl-4-sulfonylamino)-methyl]-benzoylamino}-methyl)-benzoic acid

The compound was synthesised using method N to give title compound 7N8 (3.9 mg, 4%) LC/MS: 95% MH⁺, m/z 501, Rt = 1.45 mins.

### 7N9. 3-[(Biphenyl-4-sulfonylamino)-methyl]-N,N-dimethyl-benzamide

The compound was synthesised using method N to give title compound 7N9 (7.7 mg, 10%) LC/MS: 100% MH⁺, m/z 395, Rt = 1.47 mins.

### 7N10. Biphenyl-4-sulfonic acid 3-(pyrrolidine-1-carbonyl)-benzylamide

The compound was synthesised using method N to give title compound 7N10 (10.6 mg, 13%) LC/MS: 100% MH⁺, m/z 421, Rt = 2.04 mins.

### 8M. PS- 3-[(3-Chloro-2-methyl-benzenesulfonylamino)-methyl]-benzoic acid

The compound was synthesised using methods L and M to give title compound 8M. LC/MS: 91% MH⁺, m/z 340, Rt = 1.31 mins.

### 8N1. 3-[(3-Chloro-2-methyl-benzenesulfonylamino)-methyl]-N-cyclohexyl-benzamide

The compound was synthesised using method N to give title compound 8N1 (10.6 mg, 13%) LC/MS: 95% MH⁺, m/z 421, Rt = 2.11 mins.

### 8N2. 3-[(3-Chloro-2-methyl-benzenesulfonylamino)-methyl]-N-cyclohexylmethyl-benzamide

The compound was synthesised using method N to give title compound 8N2 (6.7 mg, 8%) LC/MS: 100% MH⁺, m/z 435, Rt = 2.43 mins.

### 9M. PS- 3-[(Benzo[b]thiophene-2-sulfonylamino)-methyl]-benzoic acid

The compound was synthesised using methods L and M to give title compound 9M. LC/MS: 35% MH⁺, m/z 348, Rt = 1.32 mins.

### 9N1. 3-[(Benzo[b]thiophene-2-sulfonylamino)-methyl]-N,N-diethyl-benzamide

The compound was synthesised using method N to give title compound 9N1 (2.2 mg, 3%) LC/MS: 100% MH⁺, m/z 403, Rt = 2.03 mins.

### 9N2. Benzo[b]thiophene-2-sulfonic acid 3-(4-methyl-piperazine-1-carbonyl)-benzylamide

The compound was synthesised using method N to give title compound 9N2 (6.4 mg, 8%) LC/MS: 100% MH⁺, m/z 430, Rt = 1.74 mins.

### 9N3. 3-[(Benzo[b]thiophene-2-sulfonylamino)-methyl]-N-cyclohexyl-benzamide

The compound was synthesised using method N to give title compound 9N3 (10.8 mg, 13%) LC/MS: 100% MH⁺, m/z 429, Rt = 2.12 mins.

### 9N4. 3-[(Benzo[b]thiophene-2-sulfonylamino)-methyl]-N-cyclohexylmethyl-benzamide

The compound was synthesised using method N to give title compound 9N4 (9.7 mg, 11%) LC/MS: 100% MH⁺, m/z 443, Rt = 2.18 mins.

### 10M. PS- 3-[(Toluene-4-sulfonylamino)-methyl]-benzoic acid

The compound was synthesised using methods L and M to give title compound 10M. LC/MS: 79% MH⁺, m/z 306, Rt = 1.21 mins.

### 10N1. N,N-Diethyl-3-[(toluene-4-sulfonylamino)-methyl]-benzamide

The compound was synthesised using method N to give title compound 10N1 (9.8 mg, 14%) LC/MS: 97% MH⁺, m/z 361, Rt = 1.32 mins.

### 11M. PS- 3-[(4-Methoxy-benzenesulfonylamino)-methyl]-benzoic acid

The compound was synthesised using methods L and M to give title compound 11M. LC/MS: 82% MH⁺, m/z 322, Rt = 1.16 mins.

### 11N1. N,N-Diethyl-3-[(4-methoxy-benzenesulfonylamino)-methyl]-benzamide

The compound was synthesised using method N to give title compound 11N1 (4.6 mg, 6%) LC/MS: 100% MH⁺, m/z 377, Rt = 1.27 mins.

### 11N2. 4-Methoxy-N-[3-(morpholine-4-carbonyl)-benzyl]-benzenesulfonamide

The compound was synthesised using method N to give title compound 11N2 (6.4 mg, 12%) LC/MS: 100% MH⁺, m/z 391, Rt = 1.12 mins.

### 12M. PS- 3-[(4-Chloro-benzenesulfonylamino)-methyl]-benzoic acid

The compound was synthesised using methods L and M to give title compound 12M. LC/MS: 98% MH⁺, m/z 326, Rt = 1.25 mins.

### 12N1. 3-[(4-Chloro-benzenesulfonylamino)-methyl]-N,N-diethyl-benzamide

The compound was synthesised using method N to give title compound 12N1 (15.4 mg, 20%) LC/MS: 100% MH⁺, m/z 381, Rt = 1.35 mins.

### 13M. PS- 3-[(Thiophene-2-sulfonylamino)-methyl]-benzoic acid

The compound was synthesised using methods L and M to give title compound 13M. LC/MS: 86% MH⁺, m/z 298, Rt =1.12 mins.

### 13N1. N,N-Diethyl-3-[(thiophene-2-sulfonylamino)-methyl]-benzamide

The compound was synthesised using method N to give title compound 13N1 (14.9 mg, 21%) LC/MS: 100% MH⁺, m/z 353, Rt = 1.24 mins.

### 14M. PS- 3-[(3-Chloro-benzenesulfonylamino)-methyl]-benzoic acid

The compound was synthesised using methods L and M to give title compound 14M. LC/MS: 96% MH⁺, m/z 326, Rt = 1.27 mins.

### 14N1. 3-[(3-Chloro-benzenesulfonylamino)-methyl]-N,N-diethyl-benzamide

The compound was synthesised using method N to give title compound 14N1 (11.4 mg, 23%) LC/MS: 100% MH⁺, m/z 381, Rt = 1.98 mins.

### 15M. PS- 3-[(Toluene-3-sulfonylamino)-methyl]-benzoic acid

The compound was synthesised using methods L and M to give title compound 15M. LC/MS: 97% MH⁺, m/z 306, Rt = 1.23 mins.

### 15N1. N,N-Diethyl-3-[(toluene-3-sulfonylamino)-methyl]-benzamide

The compound was synthesised using method N to give title compound 15N1 (11.7 mg, 24%) LC/MS: 100% MH⁺, m/z 361, Rt = 1.94 mins.

### 16M. PS- 3-[(3-Methoxy-benzenesulfonylamino)-methyl]-benzoic acid

The compound was synthesised using methods L and M to give title compound 16M. LC/MS: 93% MH⁺, m/z 322, Rt = 1.20 mins.

### 16N1. N,N-Diethyl-3-[(3-methoxy-benzenesulfonylamino)-methyl]-benzamide

The compound was synthesised using method N to give title compound 16N1 (12.8 mg, 26%) LC/MS: 100% MH⁺, m/z 377, Rt = 1.90 mins.

### 17M. PS- 3-(Benzenesulfonylamino-methyl)-benzoic acid

The compound was synthesised using methods L and M to give title compound 17M. LC/MS: 98% MH⁺, m/z 292, Rt = 1.16 mins.

### 17N1. 3-(Benzenesulfonylamino-methyl)-N,N-diethyl-benzamide

The compound was synthesised using method N to give title compound 17N1 (9.9 mg, 14%) LC/MS: 97% MH⁺, m/z 347, Rt = 1.88 mins.

### 18M. PS- 3-[(Naphthalene-2-sulfonylamino)-methyl]-benzoic acid

The compound was synthesised using methods L and M to give title compound 18M. LC/MS: 100% MH⁺, m/z 342, Rt = 1.31 mins.

### 18N1. N,N-Diethyl-3-[(naphthalene-2-sulfonylamino)-methyl]-benzamide

The compound was synthesised using method N to give title compound 18N1 (14.6 mg, 22%) LC/MS: 100% MH⁺, m/z 397, Rt = 2.03 mins.

### 18N2. Naphthalene-2-sulfonic acid 3-(morpholine-4-carbonyl)-benzylamide

The compound was synthesised using method N to give title compound 18N2 (4.4 mg, 6%) LC/MS: 100% MH⁺, m/z 411, Rt = 1.89 mins.

### 18N3. N-Cyclohexyl-3-[(naphthalene-2-sulfonylamino)-methyl]-benzamide

The compound was synthesised using method N to give title compound 18N3 (30 mg, 28%) LC/MS: 88% MH⁺, m/z 423, Rt = 2.28 mins.

### 18N4. N-Cyclohexylmethyl-3-[(naphthalene-2-sulfonylamino)-methyl]-benzamide

The compound was synthesised using method N to give title compound 18N4 (48 mg, 44%) LC/MS: 94% MH⁺, m/z 437, Rt = 2.36 mins.

### 18N5. 4-({3-[(Naphthalene-2-sulfonylamino)-methyl]-benzoylamino}-methyl)-benzoic acid

The compound was synthesised using method N to give title compound 18N5 (26 mg, 22%) LC/MS: 90% MH⁺, m/z 475, Rt = 2.02 mins.

### 18N6. 3-[(Naphthalene-2-sulfonylamino)-methyl]-N-p-tolyl-benzamide

The compound was synthesised using method N to give title compound 18N6 (20 mg, 19%) LC/MS: 95% MH⁺, m/z 431, Rt = 2.33 mins.

### 19M. PS- 3-[(Naphthalene-1-sulfonylamino)-methyl]-benzoic acid

The compound was synthesised using methods L and M to give title compound 19M. LC/MS: 100% MH⁺, m/z 342, Rt = 1.28 mins.

### 19N1. N,N-Diethyl-3-[(naphthalene-1-sulfonylamino)-methyl]-benzamide

The compound was synthesised using method N to give title compound 19N1 (17.5 mg, 26%) LC/MS: 100% MH⁺, m/z 397, Rt = 2.00 mins.

### 19N2. Naphthalene-1-sulfonic acid 3-(morpholine-4-carbonyl)-benzylamide

The compound was synthesised using method N to give title compound 19N2 (15.7 mg, 23%) LC/MS: 100% MH⁺, m/z 411, Rt = 1.86 mins.

### 20M. PS- 3-[(4-Phenozy-benzenesulfonylamino)-methyl]-benzoic acid

The compound was synthesised using methods L and M to give title compound 20M.

### 20N1. N,N-Diethyl-3-[(4-phenozy-benzenesulfonylamino)-methyl]-benzamide

The compound was synthesised using method N to give title compound 20N1 (11.9 mg, 20%) LC/MS: 100% MH⁺, m/z 439, Rt = 1.49 mins.

### 20N2. N-[3-(Morpholine-4-carbonyl)-benzyl]-4-phenoxy-benzenesulfonamide

The compound was synthesised using method N to give title compound 20N2 (9.1 mg, 15%) LC/MS: 100% MH⁺, m/z 453, Rt = 1.98 mins.

### 21M. PS- 3-[(6-Phenoxy-pyridine-3-sulfonylamino)-methyl]-benzoic acid

The compound was synthesised using methods L and M to give title compound 21M

### 21N1. N,N-Diethyl-3-[(6-phenoxy-pyridine-3-sulfonylamino)-methyl]-benzamide

The compound was synthesised using method N to give title compound 21N1 (9.2 mg, 16%) LC/MS: 100% MH⁺, m/z 440, Rt = 1.40 mins.

### 21N2. 6-Phenoxy-pyridine-3-sulfonic acid 3-(morpholine-4-carbonyl)-benzylamide

The compound was synthesised using method N to give title compound 21N2 (10.1 mg, 17%) LC/MS: 100% MH⁺, m/z 454, Rt = 1.28 mins.

### 22M. PS- 3-[(4-Phenylamino-benzenesulfonylamino)-methyl]-benzoic acid

The compound was synthesised using methods L and M to give title compound 22M

### 22N1. N,N-Diethyl-3-[(4-phenylamino-benzenesulfonylamino)-methyl]-benzamide

The compound was synthesised using method N to give title compound 22N1 (12.4 mg, 21 %) LC/MS: 100% MH⁺, m/z 43 8, Rt = 1.47 mins.

### 22N2. N-[3-(Morpholine-4-carbonyl)-benzyl]-4-phenylamino-benzenesulfonamide

The compound was synthesised using method N to give title compound 22N2 (6.8 mg, 11 %) LC/MS: 100% MH⁺, m/z 452, Rt = 1.29 mins.

### Synthetic Route 4

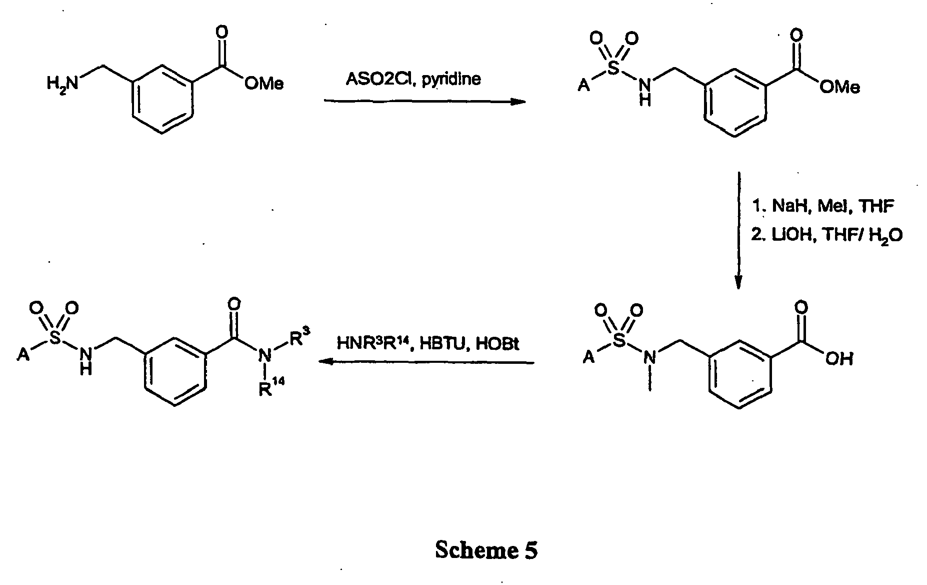

### Method O - N-methylation

A solution of title intermediate D (4.2 mmol) in THF (50 ml) was added to a suspension of NaH (1.5 eq) in THF (30 ml) at 0°C and the mixture was stirred for 1.5 hr. Methyl iodide (2 eq) was added and the mixture was stirred at room temperature overnight. The reaction was quenched with water (40 ml) and the THF was removed *in vacuo*. The residue was partitioned between EtOAc and water and the aqueous phase was extracted with EtOAc. The combined organic phases were dried (MgSO₄) and concentrated *in vacuo* to give the title compounds. The *N*-methylation reaction gave mixtures of *N*-methylated methyl ester and carboxylic acid which were saponified without further purification.

### Method E - Saponification.

As detailed in synthetic route 1 to give intermediate E.

### Method G - Amide Formation.

As detailed in synthetic route 1 to give compound G.

Compounds synthesised by route 4 are detailed below:

### 23O. 3-{[(Biphenyl-4-sulfonyl)-methyl-amino]-methyl}-benzoic acid methyl ester

The compound was synthesised from intermediate 1D using method O to give title compound 230 (0.52 g, 100%, mixture of ester and acid) LC/MS: 6% ester:82% acid MH⁺, m/z 395, Rt = 1.71 mins.

### 23E. 3-{[(Biphenyl-4-sulfonyl)-methyl-amino]-methyl}-benzoic acid

The compound was synthesised using method E to give title compound 23E (0.49 g, 97%) LC/MS: 95% MH⁺, m/z 382, Rt = 1.65 mins.

### 23G1. Biphenyl-4-sulfonic acid methyl-[3-(morpholine-4-carbonyl)-benzyl]-amide

The compound was synthesised using method G to give title compound 23G1 (16.9 mg, 14%) LG/MS: 100% MH⁺, m/z 451, Rt = 2.07 mins.

### 240. 3-{[(3-Chloro-2-methyl-benzenesulfonyl)-methyl-amino]-methyl}-benzoic acid methyl ester

The compound was synthesised from intermediate 2D using method O to give title compound 240 (2.1 g, >100%, mixture of ester and acid ) LC/MS: 15% ester:74% acid MH⁺, m/z 368, Rt = 1.65 mins.

### 24E. 3-{[(3-Chloro-2-methyl-benzenesulfonyl)-methyl-amino]-methyl}-benzoic acid

The compound was synthesised using method E to give title compound 24E (1.39 g, 93%) LC/MS: 89% MH⁺, m/z 354, Rt = 1.52 mins.

### 24G1. 3-{[(3-Chloro-2-methyl-benzenesulfonyl)-methyl-amino]-methyl}-N,N-diethylbenzamide

The compound was synthesised using method G to give title compound 24G1 (12 mg, 10%) LC/MS: 100% MH⁺, m/z 409, Rt = 1.63 mins.

### 24G2. 3-Chloro-2,N-dimethyl-N-[3-(4-methyl-piperazine-1-carbonyl)-benzyl]-benzenesulfonamide

The compound was synthesised using method G to give title compound 24G2 (41 mg, 33%) LC/MS: 96% MH⁺, m/z 436, Rt = 1.31 mins.

### 24G3. 3-{[(3-Chloro-2-methyl-benzenesulfonyl)-methyl-amino]-methyl}-N-cyclohexylmethyl-benzamide

The compound was synthesised using method G to give title compound 24G3 (26 mg, 41 %) LC/MS: 96% MH⁺, m/z 449, Rt = 4.40 mins.

### 24G4. 4-[(3-{[(3-Chloro-2-methyl-benzenesulfonyl)-methyl-amino]-methyl}-benzoylamino)-methyl]-benzoic acid

The compound was synthesised using method G to give title compound 24G4 (40 mg, 58%) LC/MS: 91% MH⁺, m/z 487, Rt = 2.19 mins.

### 24G5. 3-{[(3-Chloro-2-methyl-benzenesulfonyl)-methyl-amino]-methyl}-N-cyclohexyl-benzamide

The compound was synthesised using method G to give title compound 24G5 (25 mg, 41 %) LC/MS: 90% MH⁺, m/z 435, Rt = 4.25 mins.

### 24G6. 4-[(3-{[(3-Chloro-2-methyl-benzenesulfonyl)-methyl-amino]-methyl}-benzoylamino)-methyl]-benzoic acid methyl ester

The compound was synthesised using method G to give title compound 24G6 (30 mg, 42%) LC/MS: 96% MH⁺, m/z 501, Rt = 3.45 mins.

### 250. 3-{[(Benzo[b]thiophene-2-sulfonyl)-methyl-amino]-methyl}-benzoic acid methyl ester

The compound was synthesised from intermediate 4D using method O to give title compound 250 (0.21 g, 100%) LC/MS: 88% MH⁺, m/z 376, Rt = 1.65 mins.

### 25E. 3-{[(Benzo[b]thiophene-2-sulfonyl)-methyl-amino]-methyl}-benzoic acid

The compound was synthesised using method E to give title compound 25E (0.2 g, 100%) LC/MS: 97% MH⁺, m/z 362, Rt = 1.47 mins.

### 25G1. 3-{[(Benzo[b]thiophene-2-sulfonyl)-methyl-amino]-methyl}-N,N-diethylbenzamide

The compound was synthesised using method G to give title compound 25G1 (93.6 mg, 41%) LC/MS: 95% MH⁺, m/z 417, Rt = 2.15 mins.

### 25G2. 3-{[(Benzo[b]thiophene-2-sulfonyl)-methyl-amino]-methyl}-N-cyclohexyl-benzamide

The compound was synthesised using method G to give title compound 25G2 (24 mg, 39%) LC/MS: 95% MH⁺, m/z 443, Rt = 3.52 mins.

### 25G3. 3-{[(Benzo[b]thiophene-2-sulfonyl)-methyl-amino]-methyl}-N-cyclohexylmethyl-benzamide

The compound was synthesised using method G to give title compound 25G3 (25 mg, 40%) LC/MS: 89% MH⁺, m/z 457, Rt = 3.88 mins.

### 25G4. 3-{[(Benzo[b]thiophene-2-sulfonyl)-methyl-amino]-methyl}-N-(4-trifluoromethyl-benzyl)-benzamide

The compound was synthesised using method G to give title compound 25G4 (24 mg, 33%) LC/MS: 97% MH⁺, m/z 519, Rt = 3.99 mins.

### 25G5. 4-[(3-{[(Benzo[b]thiophene-2-sulfonyl)-methyl-amino]-methyl}-benzoylamino)-methyl]-benzoicacid methyl ester

The compound was synthesised using method G to give title compound 25G5 (26mg, 37%) LC/MS: 96% MH⁺, m/z 509, Rt = 2.95 mins.

### 25G6. 4-[(3-{[(Benzo[b]thiophene-2-sulfonyl)-methyl-amino]-methyl}-benzoylamino)-methyl]-benzoic acid

The compound was synthesised using method G to give title compound 25G6 (43 mg, 63%) LC/MS: 93% MH⁺, m/z 494, Rt = 1.90 mins.

### 25G7. 3-{[(Benzo[b]thiophene-2-sulfonyl)-methyl-amino]-methyl}-N-p-tolyl-benzamide

The compound was synthesised using method G to give title compound 25G7 (28 mg, 45%) LC/MS: 97% MH⁺, m/z 451, Rt = 3.82 mins.

### 26O. 3-{[Methyl-(naphthalene-2-sulfonyl)-amino]-methyl}-benzoic acid methyl ester

The compound was synthesised from intermediate 3D using method O to give title compound 260 (1.99 g, 100%) LC/MS: 46% ester: 46% acid MH⁺, m/z 370, Rt = 1.68 mins.

### 26E. 3-{[Methyl-(naphthalene-2-sulfonyl)-amino]-methyl}-benzoic acid

The compound was synthesised using method E to give title compound 26E (1.75 g, 92%) LC/MS: 90% MH⁺, m/z 356, Rt = 1.48 mins.

### 26G1. N,N-Diethyl-3-{[methyl-(naphthalene-2-sulfonyl)-amino]-methyl}-benzamide

The compound was synthesised using method G to give title compound 26G1 (28 mg, 49%) LC/MS: 94% MH⁺, m/z 411, Rt = 2.55 mins.

### 26G2. N-Cyclohexyl-3-{[methyl-(naphthalene-2-sulfonyl)-amino]-methyl}-benzamide

The compound was synthesised using method G to give title compound 26G2 (27 mg, 44%) LC/MS: 94% MH⁺, m/z 437, Rt = 3.41 mins.

### 26G3. N-Cyclohexylmethyl-3-{[methyl-(naphthalene-2-sulfonyl)-amino]-methyl}-benzamide

The compound was synthesised using method G to give title compound 26G3 (26 mg, 41%) LC/MS: 95% MH⁺, m/z 451, Rt = 4.14 mins.

### 26G4. 3-{[Methyl-(naphthalene-2-sulfonyl)-amino]-methyl}-N-(4-trifluoromethylbenzyl)-benzamide

The compound was synthesised using method G to give title compound 26G4 (28 mg, 39%) LC/MS: 98% MH⁺, m/z 513, Rt = 3.85 mins.

### 26G5. 4-[(3-{[Methyl-(naphthalene-2-sulfonyl)-amino]-methyl}-benzoylamino)-methyl]-benzoic acid methyl ester

The compound was synthesised using method G to give title compound 26G5 (26 mg, 37%) LC/MS: 95% MH⁺, m/z 503, Rt = 2.87 mins.

### 26G6. 3-{[Methyl-(naphthalene-2-sulfonyl)-amino]-methyl}-N-p-tolyl-benzamide

The compound was synthesised using method G to give title compound 26G6 (21 mg, 34%) LC/MS: 97% MH⁺, m/z 445, Rt = 2.49 mins.

### 27O. 3-{[(5-Chloro-3-methyl-benzo[b]thiophene-2-sulfonyl)-methyl-amino]-methyl}-benzoic acid methyl ester

The compound was synthesised from intermediate 5D using method O to give title compound 270 (0.28 g, 73%) MS: m/z 423.

### 27E. 3-{[(5-Chloro-3-methyl-benzo[b]thiophene-2-sulfonyl)-methyl-amino]-methyl}-benzoic acid

The compound was synthesised using method E to give title compound 27E (0.2 g, 87%) MS: m/z 409.

### 27G1. 3-{[(5-Chloro-3-methyl-benzo[b]thiophene-2-sulfonyl)-methyl-amino]-methyl}-N,N-diethyl-benzamide

The compound was synthesised using method G to give title compound 27G1 (17mg, 30%) LC/MS: 100% MH⁺, m/z 465, Rt = 2.52 mins.

### 27G2. 3-{[(5-Chloro-3-methyl-benzo[b]thiophene-2-sulfonyl)-methyl-amino]-methyl}-N-cyclohexyl-benzamide

The compound was synthesised using method G to give title compound 27G2 (19mg, 32%) LC/MS: 96% MH⁺, m/z 491, Rt = 2.63 mins.

### 27G3. 3-{[(5-Chloro-3-methyl-benzo[b]thiophene-2-sulfonyl)-methyl-amino]-methyl}-N-cyclohexylmethyl-benzamide

The compound was synthesised using method G to give title compound 27G3 (40 mg, 65%) LC/MS: 99% MH⁺, m/z 505, Rt = 2.71 mins.

### 27G4. 3-{[(5-Chloro-3-methyl-benzo[b]thiophene-2-sulfonyl)-methyl-amino]-methyl}-N-(4-trifluoromethyl-benzyl)-benzamide

The compound was synthesised using method G to give title compound 27G4 (30 mg, 43%) LC/MS: 100% MH⁺, m/z 567, Rt = 2.67 mins.

### 27G5. 4-[(3-{[(5-Chloro-3-methyl-benzo[b]thiophene-2-sulfonyl)-methyl-amino]-methyl}-benzoylamino)-methyl]-benzoic acid methyl ester

The compound was synthesised using method G to give title compound 27G5 (24 mg, 35%) LC/MS: 94% MH⁺, m/z 557, Rt = 2.55 mins.

### 27G6. 4-[(3-{[(5-Chloro-3-methyl-benzo[b]thiophene-2-sulfonyl)-methyl-amino]-methyl}-benzoylamino)-methyl]-benzoic acid

The compound was synthesised using method G to give title compound 27G6 (25 mg, 38%) LC/MS: 91% MH⁺, m/z 543, Rt = 2.36 mins.

### Synthetic Route 5

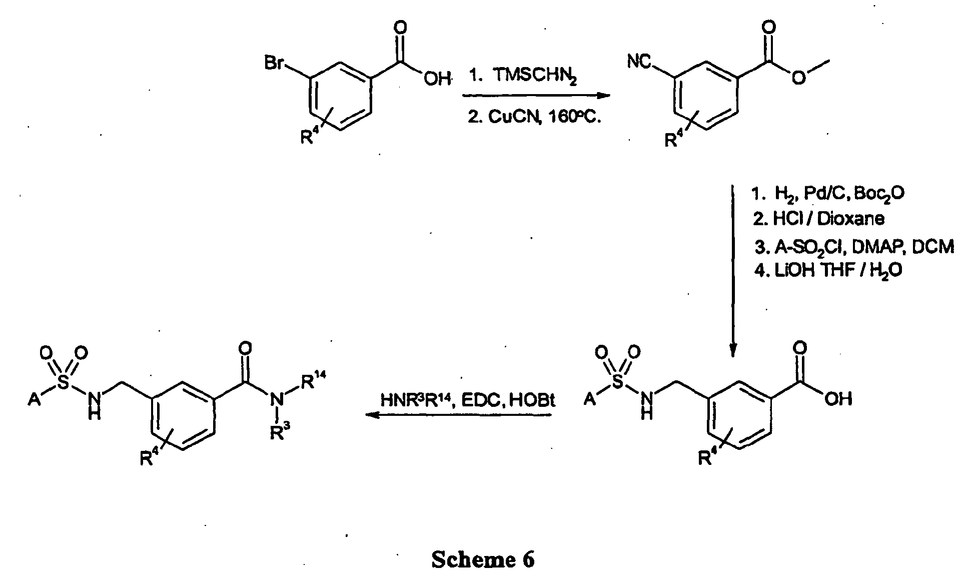

### Method P - 3-Bromo-5-fluorobenzoic acid methyl ester

To a solution of 3-bromo-5-fluorobenzoic acid (2 g, 9.13 mmol) in DCM/ MeOH (20 ml/ 5 ml) was added trimethylsilyldiazomethane (2M solution in ether; 1 eq) and the mixture was stirred for 1 h. Acetic acid was added dropwise until the yellow colour disappeared. The solvents were removed *in vacuo* and the residue was dissolved in DCM and washed with K₂CO₃. The organic phase was dried (MgSO₄) and concentrated *in vacuo* to give the title compound (1.5 g, 71%) LC/MS: 91% MH⁺, m/z not ionised, Rt = 1.55 mins.

### Method Q - 3-Cyano-5-fluorobenzoic acid methyl ester

To a solution of title compound P, 3-bromo-5-fluorobenzoic acid methyl ester, (1 g, 3 mmol) in degassed DMF (5 ml) in a schlenk tube was added copper cyanide (1.1 eq) and the mixture was heated to 160°C for 18 h. After cooling the reaction mixture was poured into 1M K₂CO₃ and ethyl acetate. To improve separation NH₄OH solution (5 ml) was added and the aqueous phase re-extracted with ethyl acetate. The combined organic phases were dried (MgSO₄) and concentrated *in vacuo*. The crude product was purified by column chromatography with DCM/ MeOH (99:1) as the eluent to give the title compound (0.47 g, 60%) LC/MS: 100% MH⁺, m/z not ionised, Rt = 1.24 mins.

### Method R - 3-(tert-Butoxycarbonylamino-methyl)-5-fluoro-benzoic acid methyl ester

To a solution of title intermediate Q, 3-cyano-5-fluorobenzoic acid methyl ester, (0.8 g, 4.47 mmol) in ethyl acetate (20 ml) was added di-*tert*-butyl-dicarbonate (1.2 eq) and 10% palladium on carbon (0.1 eq) the vessel was charged with hydrogen and the mixture was stirred overnight. The vessel was recharged with hydrogen and stirred for a further 5 h. The mixture was filtered through celite and the filtrate was concentrated *in vacuo*. The crude product was purified by column chromatography with heptane/ ethyl acetate (85:15) to give the title compound (0.41 g, 32%). LC/MS: 94% MH⁺, m/z not ionised, Rt = 1.43 mins.

### Method S - 3-Aminomethyl-5-fluoro-benzoic acid methyl ester

To a solution of title intermediate R, 3-(*tert*-butoxycarbonylamino-methyl)-5-fluorobenzoic acid methyl ester, (0.41 g, 1.45 mmol) in DCM (10 ml) was added 4M HCl in dioxane (4 eq) and the mixture was stirred at room temperature for 4.5 h. The solvents were removed *in vacuo*. The residue was redissolved in DCM and carbonate resin added and the mixture stirred at room temperature overnight The mixture was filtered and the solvent removed *in vacuo* to give the title compound (0.25 g, 94%). LC/MS: 48% MH⁺, m/z 184, Rt = 0.76 mins.

### Method T - Sulfonamide formation

To a solution of title intermediate S, 3-aminomethyl-5-fluoro-benzoic acid methyl ester, (1.37 mmol) and the sulfonyl chloride (1.0 eq) in DCM (10 ml) was added DMAP (1.1 eq, 1.50 mmol). The mixture was stirred at room temperature for 16 h. The solvent was removed *in vacuo* to give the title compound.

### Method U - Saponification

To a solution of title intermediate T (1.37 mmol) in THF/ H₂O (2:1 5ml:2.5 ml) was added lithium hydroxide (2 eq) and the mixture is stirred at room temperature for 5 h. The solvents were removed *in vacuo* and the residue was dissolved in DCM and water. The aqueous phase was washed with DCM and then acidified with 1M HCl to pH= 4 and re-extracted with DCM. The solid which precipitated from the DCM phase was collected by filtration to give the title compound.

### Method V - Amide formation

To a solution of title intermediate U (0.19 mmol) in DCM (2 ml) was added EDC (1 eq) and HOBt (1 eq). The amine (1 eq) was added and the mixture was stirred at room temperature for 18 h. The solvent was removed *in vacuo* and the crude product was purified by column chromatography with DCM/ MeOH as the eluent to give the title compound.

Compounds synthesised by route 5 are detailed below:

### 28T. 3-Fluoro-5-[(4'-fluoro-biphenyl-4-sulfonylamino)-methyl]-benzoic acid methyl ester

The compound was synthesised using method T to give title compound 28T(0.57 g, 100%) LC/MS: 83% MH⁺, m/z 418, Rt = 1.57 mins.

### 28U. 3-Fluoro-5-[(4'-fluoro-biphenyl-4-sulonylamino)-methyl]-benzoic acid

The compound was synthesised using method U to give title compound 28U (50 mg, 9%) LC/MS: 100% MH⁺, m/z 404, Rt = 1.41 mins.

### 28V1. N,N-Diethyl-3-fluoro-5-[(4'-fluoro-biphenyl-4-sulfonylamfno)-methyl]-benzamide

The compound was synthesised using method V to give title compound 28V1 (14.7 mg, 15%) LC/MS: 100% MH⁺, m/z 459, Rt = 1.52 mins.

### 28V2. 4'-Fluoro-biphenyl-4-sulfonic acid 3-fluoro-5-(morpholine-4-carbonyl)-benzylamide

The compound was synthesised using method V to give title compound 28V2 (10.7 mg, 12%) LC/MS: 100% MH⁺, m/z 473, Rt = 1.4 mins.

### 29T. 3-[(Biphenyl-4-sulfonylamino)-methyl]-5-fluoro-benzoic acid methyl ester

The compound was synthesised using method T to give title compound 29T (0.37 g, 35%) LC/MS: 100% MH⁺, m/z 400, Rt = 1.6 mins.

### 29U. 3-[(Biphenyl-4-sulfonylamino)-methyl]-5-fluoro-benzoic acid

The compound was synthesised using method U to give title compound 29U (0.33 g, 99%) LC/MS: 100% MH⁺, m/z 386, Rt = 1.43 mins.

### 29V1. 3-[(Biphenyl-4-sulfonylamino)-methyl]-N,N-diethyl-5-fluoro-benzamide

The compound was synthesised using method V to give title compound 29V1 (47.8 mg, 39%) LC/MS: 100% MH⁺, m/z 441, Rt = 1.52 mins.

### 29V2. Biphenyl-4-sulfonic acid 3-fluoro-5-(morpholine-4-carbonyl)-benzylamide

The compound was synthesised using method V to give title compound 29V2 (7.8 mg, 6%) LC/MS: 100% MH⁺, m/z 455, Rt = 1.39 mins.

### 29V3. Biphenyl-4-sulfonic acid 3-fluoro-5-(4-methyl-piperaine-1-carbonyl)-benzylamide

The compound was synthesised using method V to give title compound 29V3 (31 mg, 23%) LC/MS: 100% MH⁺, m/z 468, Rt = 1.22 mins.

### Synthetic Route 6.

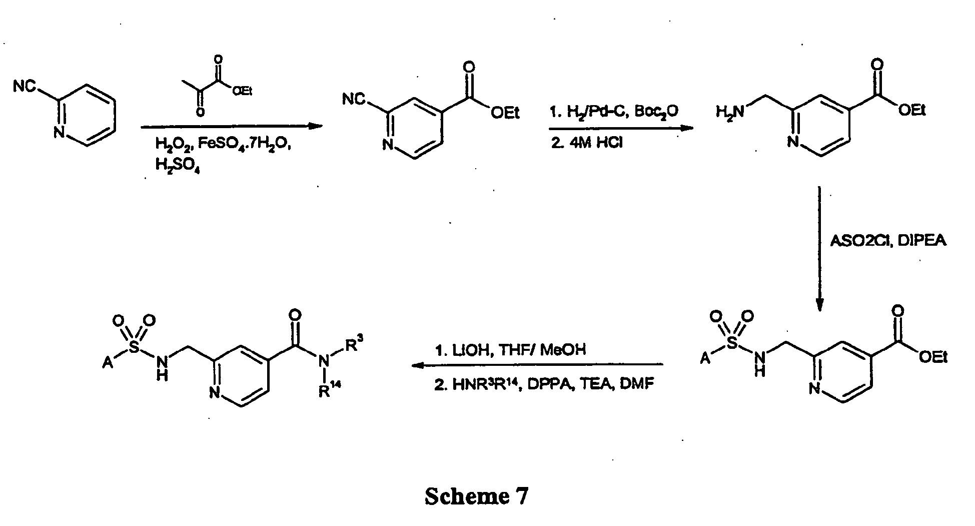

### Method W - 2-Cyano-isonicotinic acid ethyl ester.

35% wt aq hydrogen peroxide (3 eq) was added dropwise to ethyl pyruvate (4.5 eq) at - 10°C.The resulting solution was added to a solution of 2-cyanopyridine (20 g, 192.1 mmol), conc. sulphuric acid (3 eq) and iron (II) sulphate heptahydrate (3 eq) in DCM/ H2O (1: 1.6; 100 ml: 160 ml) at -5°C and the mixture was stirred for 1 h. The reaction mixture was poured into ice water (300 ml) and the two phases were separated. The aqueous phase was extracted with DCM and the combined organic phases were washed with brine, dried (Na₂SO₄) and concentrated *in vacuo*. The crude residue was purified by dry flash chromatography with cyclohexane/ EtOAc (9:1) as the eluent to give the title compound (3.46 g, 10%). LC/MS: 100% MH⁺, m/z 177, Rt = 1.17 mins.

### Method X - 2-(tert-Butoxycarbonylamino-methyl)-isonicotinic acid ethyl ester

To a solution of title intermediate W, 2-cyano-isonicotinic acid ethyl ester, (25 mmol) in ethanol (500 ml) was added di-*tert*-butyl-dicarbonate (1.2 eq) and 10% palladium on carbon (900 mg), the vessel was charged with hydrogen and stirred at room temperature for 3 h. The mixture was filtered through celite and the filtrate was concentrated *in vacuo*. The crude residue was purified by column chromatography with cyclohexane/ EtOAc (4:1) as the eluent to give the title compound (3.85 g, 55%). LC/MS: 95% MH⁺, m/z 281, Rt = 1.18 mins.

### Method Y - 2-Aminomethyl-isonicotinic acid ethyl ester

To a solution of title intermediate X, 2-(tert-butoxycarbonylamino-methyl)-isonicotinic acid ethyl ester, (13.7 mmol) in DCM (35 ml) was added 4M HCl in dioxane (5.6 eq) and the mixture was stirred at room temperature for 4 h. The solvents were removed *in vacuo* to give the HCl salt of the title compound which was used without purification (3.28 g, >100%). LC/MS: 100% MH⁺, m/z 181, Rt = 0.77 mins.

### Method Z - Sulfonamide formation

To a solution of intermediate Y, 2-aminomethyl-isonicotinic acid ethyl ester (3.16 mmol) and the sulfonyl chloride (1.05 eq) in DCM (15 ml) was added DIPEA (3.5 eq) and the mixture was stirred at room temperature for 1 h. The reaction mixture was washed with 1M NaOH solution and the organic phase was dried (Na₂SO₄) and concentrated *in vacuo*. The crude residue was purified by column chromatography with DCM/ MeOH as the eluent to give the title compound.

### Method AA - Saponification.

To a solution of intermediate Z (3.28 mmol) in THF/ H₂O (3:2 50 ml) was added lithium hydroxide (8 eq) and the mixture was stirred at room temperature for 2 h. The solvents were removed *in vacuo* and the residue was suspended in ethyl acetate and washed with 1M HCl. The aqueous phase was extracted with ethyl acetate and the combined organic phases were dried (MgSO₄) and concentrated *in vacuo* to give the title compound.

### Method BB - Amide Formation.

To a solution of intermediate AA (0.23 mmol) and TEA (3 eq) in DMF (2 ml) was added the amine (2 eq) and DPPA (4 eq) and the mixture was stirred at room temperature for 1 h. The reaction mixture was basified with 15% NaOH (1 ml) and extracted with DCM. The combined organic phases were washed with brine, dried (Na₂SO₄) and concentrated *in vacuo*. The crude mixture was triturated with DCM, the solid impurity removed by filtration and the filtrate was concentrated *in vacuo* to give the title compound.

Compounds synthesised by route 6 are detailed below:

### 30Z. 2-[(Biphenyl-4-sulfonylamino)-methyl]-isonicotinic acid ethyl ester

The compound was synthesised using method Z to give title compound 30Z (1.3 g, 99%) LC/MS: 95% MH⁺, m/z 397, Rt = 1.47 mins.

### 30AA. 2-[(Biphenyl-4-sulfonylamino)-methyl]-isonicotinic acid

The compound was synthesised using method AA to give title compound 30AA (1.27 g, 88%) LC/MS: m/z 369, Rt = 1.2 mins, purity could not be determined due to poor solubility; NMR confirmed purity >90%.

### 30BB1. 2-[(Biphenyl-4-sulfonylamino)-methyl]-N,N-diethyl-isonicotinamide

The compound was synthesised using method BB to give title compound 30BB1 (24.1 mg, 25%) LC/MS: 100% MH⁺, m/z 424, Rt = 1.9 mins.

### 30BB2. Biphenyl-4-sulfonic acid [4-(morpholine-4-carbonyl)-pyridin-2-ylmethyl]-amide

The compound was synthesised using method BB to give title compound 30BB2 (50.9 mg, 51%) LC/MS: 100% MH⁺, m/z 438, Rt = 1.63 mins.

### 30BB3. Biphenyl-4-sulfonic acid [4-(4-methyl-piperazine-1-carbonyl)-pyridin-2-ylmethyl]-amide

The compound was synthesised using method BB to give title compound 30BB3 (45.2 mg, 44%) LC/MS: 100% MH⁺, m/z 451, Rt = 1.68 mins.

### Synthetic Route 7

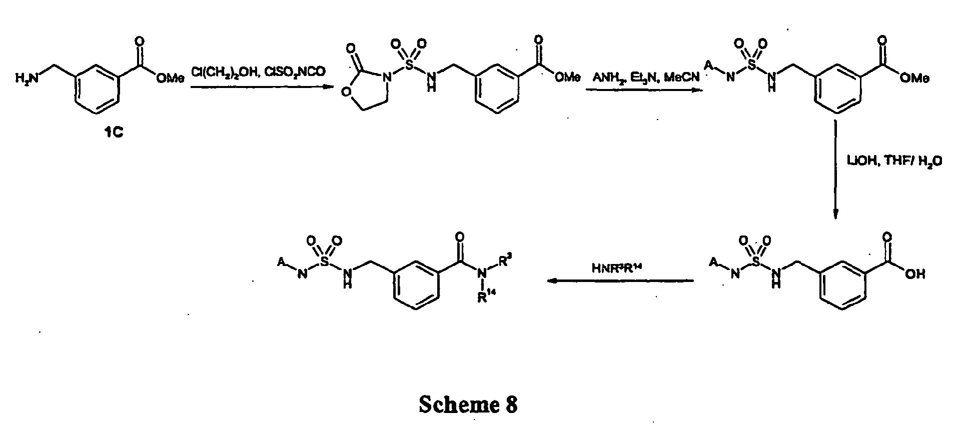

### Method CC- Oxazolidinone Formation

Chloroethanol (1 eq) was added dropwise to chlorosulfonyl isocyanate (1 eq) in DCM (30 ml) at 0°C and the mixture was stirred for 1 h. A solution of 3-aminomethyl-benzoic acid methyl ester. HCl 1C (14.88 mmol) and triethylamine (3 eq) in DCM (30 ml) was added dropwise maintaining the temperature between 0-5°C. The reaction was stirred at room temperature for 3 days. The reaction was quenched with 1 M HCl and the aqueous phase was extracted with DCM. The combined organic phases were washed with brine, dried (Na₂SO₄) and concentrated The crude mixture was purified by column chromatography with DCM/ MeOH (98:2) as the eluent to give the title compound (2.59 g, 50%). LC/MS: 90% MH⁺, m/z 315, Rt = 1.14 mins.

### Method DD- Sulfamide formation

To a solution of the oxazolidinone intermediate CC (1.59 mmol) and triethylamine (1 eq) in acetonitrile (2 ml) was added a solution of the amine (1 eq) in acetonitrile (1ml) and the mixture was heated to 80°C for 4 h. After cooling, the reaction mixture was concentrated *in vacuo*. The crude reaction mixtures were purified by column chromatography with DCM/MeOH as the eluent to give the title compound.

### Method EE - Saponification.

To a solution of intermediate DD (1.48 mmol) in THF/ MeOH (3:2 15 ml) was added lithium hydroxide (8 eq) and the mixture was stirred at room temperature for 3.5 days. The solvents were removed *in vacuo* and the residue was dissolved in DCM. The organic phase was washed with 1M HCl and the aqueous phase was extracted with DCM. The combined organic phases were washed with brine, dried (Na₂SO₄) and concentrated *in vacuo* to give the title compound.

### Method FF- Amide Formation

To a solution of intermediate EE (0.22 mmol) and triethylamine (3 eq) in DMF (2 ml) was added the amine (2 eq) and DPPA (2.5 eq). The reaction mixture was stirred at room temperature for 3 h. The reaction mixture was basifed with 15% NaOH (1 ml) and the mixture was extracted with DCM. The combined organic phases were washed with brine, dried (Na₂SO₄) and concentrated *in vacuo*. The residue was dissolved in DCM and filtered (white solid removed) and the filtrate was concentrated *in vacuo*. The crude mixture was purified either by semi-prep HPLC or column chromatography with DCM/ EtOAc as the eluent to give the title compound.

Amide formations also carried out as detailed in synthetic route 1 methods G, H and V.

Compounds synthesised using route 7 are detailed below:

### 31DD. 3-[(Biphenyl-4-aminosulfonyl)aminomethyl]-benzoic acid methyl ester

The compound was synthesised using method DD to give title compound 31DD (0.18 g, 33%) LC/MS: 100% MH⁺, m/z 397, Rt = 1.53 mins.

### 31EE. 3-[(Biphenyl-4-aminosulfonyl)aminomethyl]-benzoic acid

The compound was synthesised using method EE to give title compound 31EE (0.17 g, 98%) LC/MS: 100% MH⁺, m/z 383, Rt = 1.21 mins.

### 31V1. 3-[(Biphenyl-4-aminosulfonyl)aminomethyl]-N,N-diethyl-benzamide

The compound was synthesised using method V to give title compound 31V1 (4.7 mg, 8%) LC/MS: 100% MH⁺, m/z 438, Rt = 2.07 mins.

### 32DD. 3-[(Biphenyl-3-aminosulfonyl)aminomethyl]-benzoic acid methyl ester

The compound was synthesised using method DD to give title compound 32DD (0.59 g, 93%) LC/MS: 100% MH⁺, m/z 397, Rt = 1.51 mins.

### 32EE. 3-[(Biphenyl-3-aminosulfonyl)aminomethyl]-benzoic acid

The compound was synthesised using method EE to give title compound 32EE (0.39 g, 69%) LC/MS: 100% MH⁺, m/z 383, Rt = 1.37 mins.

### 32FF1. 3-[(Biphenyl-3-aminosulfnyl)aminomethyl]-N,N-diethyl-benzamide

The compound was synthesised using method FF to give title compound 32FF1 (50.7 mg, 53%) LC/MS: 100% MH⁺, m/z 438, Rt = 2.07 mins.

### 33DD. 3-[(Adamantane-2-aminosulfonyl)aminomethyl]-benzoic acid methyl ester

The compound was synthesised using method DD to give title compound 33DD (0.43 g, 71%) LC/MS: 100% MH⁺, m/z 379, Rt = 1.57 mins.

### 33EE. 3-[(Adamantane-2-aminosulfonyl)aminomethyl]-benzoic acid

The compound was synthesised using method EE to give title compound 33EE (0.8 g, 100%) LC/MS: 100% MH⁺, m/z 365, Rt = 1.39 mins.

### 33FF1. 3-[(Adamantane-2-aminosulfonyl)aminomethyl]-N,N-diethyl-benzamide

The compound was synthesised using method FF to give title compound 33FF1 (28.1 mg, 24%) LC/MS: 100% MH⁺, m/z 420, Rt = 1.55 mins.

### 34DD. 3-[(Decahydroisoquinoline-2-sulfonyl)aminomethyl]-benzoic acid methyl ester

The compound was synthesised using method DD to give title compound 34DD (0.47 g, 81 %) LC/MS: 100% MH⁺, m/z 367, Rt = 1.64 mins.

### 34EE. 3-[(Decahydroisoquinoline-2-sulfonyl)aminomethyl]-benzoic acid

The compound was synthesised using method EE to give title compound 34EE (0.24 g, 53%) LC/MS: 100% MH⁺, m/z 353, Rt = 1.44 mins.

### 34FF1. N,N-Diethyl-3-[(decahydroisoquinoline-2-sulfonyl)aminomethyl]-benzamide

The compound was synthesised using method FF to give title compound 34FF1 (28 mg, 29%) LC/MS: 100% MH⁺, m/z 408, Rt = 2.16 mins.

### 34G2. N-Cyclohexyl-3-[(decahydroisoquinoline-2-sulfonyl)aminomethyl]-benzamide

The compound was synthesised using method G to give title compound 34G2 (65.6 mg, 84%) LC/MS: 98% MH⁺, m/z 434, Rt = 2.49 mins.

### 34H3. 4-{[3-((Decahydroisoqukoline-2-sulfonyl)aminomethyl)-benzoylamino]-methyl}-benzoic acid methyl ester

The compound was synthesised using method H to give title compound 34H3 (42.4 mg, 6%) LC/MS: 100% MH⁺, m/z 500, Rt = 2.38 mins.

### 35DD. 3-[(1,2,3,4-Tetrahydroisoquinoline-2-sulfonyl)aminomethyl]-benzoic acid methyl ester

The compound was synthesised using method DD to give title compound 35DD (1.28 g, 50%) LC/MS: 90% MH⁺, m/z 361, Rt = 2.13 mins.

### 35EE. 3-[(1,2,3,4-Tetrahydroisoquinoline-2-sulfonyl)aminomethyl}-benzoic acid

The compound was synthesised using method EE to give title compound 35EE (1.45 g, 100%) LC/MS: 82% MH⁺, m/z 347, Rt = 1.89 mins.

### 35G1. N,N-Diethyl-3-[(1,2,3,4-tetrahydroisoquinoline-2-sulfonyl)aminomethyl]-benzamide

The compound was synthesised using method G to give title compound 35G1 (25 mg, 22%) LC/MS: 100% MH⁺, m/z 402, Rt = 2.18 mins.

### 35G2. N-Cyclohexyl-3-[(1,2,3,4-tetrahydroisoquinoline-2-sulfonyl)aminomethyl]-benzamide

The compound was synthesised using method G to give title compound 3652 (21 mg, 17%) LC/MS: 93% MH⁺, m/z 428, Rt = 2.28 mins.

### 35G3. N-Cyclohexylmethyl-3-[(1,2,3,4-tetrahydroisoquinoline-2-sulfonyl)aminomethyl]-benzamide

The compound was synthesised using method G to give title compound 35G3 (26 mg, 20%) LC/MS: 97% MH⁺, m/z 442, Rt = 2.37 mins.

### 35H4. 4-{[3-((1,2,3,4-Tetrahydroisoquinoline-2-snlfonyl)aminomethyl)-benzoylamino]-methyl}-benzoic acid methyl ester

The compound was synthesised using method H to give title compound 35H4 (0.45 g, 79%) LC/MS: 92% MH⁺, m/z 494, Rt = 2.23 mins.

### Synthetic Route 8

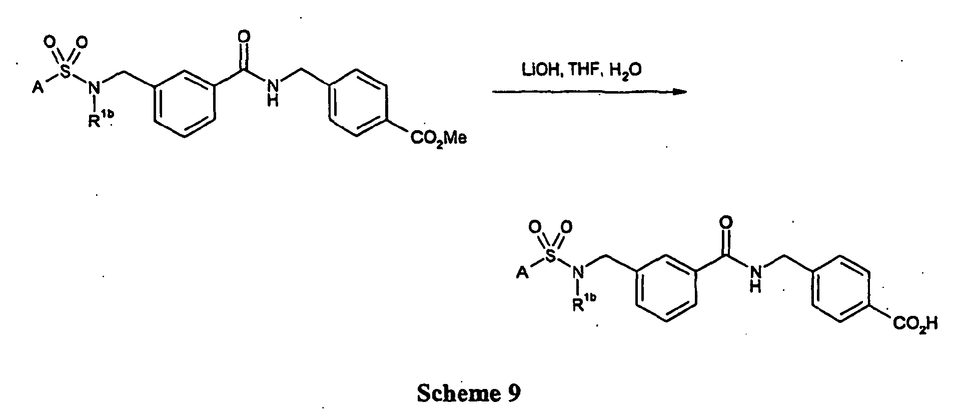

### Method GG - Saponification.

To a solution of the intermediate ester (0.05 mmol) in THF/ H₂O (2:1 1.5 ml) was added lithium hydroxide (5 eq) and the mixture was stirred at room temperature for 24hr. The solvents were removed *in vacuo* and the residue was dissolved in ethyl acetate and water. The aqueous phase was acidified to pH=2 with 1M HCl and extracted with ethyl acetate. The combined organic phases were dried (Na₂SO₄) and concentrated *in vacuo* to give the title compound.

Compounds synthesised using route 8 are detailed below:

### 34GG. 4-{[3-((Decahydroisoquinoline-2-sulfouyl)aminomethyl)-benzoylamino]-methyl}-benzoic acid

The compound was synthesised from intermediate 34H3 using method GG to give title compound 34GG (125 mg, 43%) LC/MS: 100% MH⁺, m/z 486, Rt = 2.16 mins.

### 35GG. 4-{[3-((1,2,3,4-tetrahydroisoquinoline-2-sulfonyl)aminomethyl)-benzoylamino]-methyl}-benzoic acid

The compound was synthesised from intermediate 35H4using method GG to give title compound 35GG (56 mg, 30%) LC/MS: 100% MH⁺, m/z 480, Rt = 2.02 mins.

### Synthetic Route 9

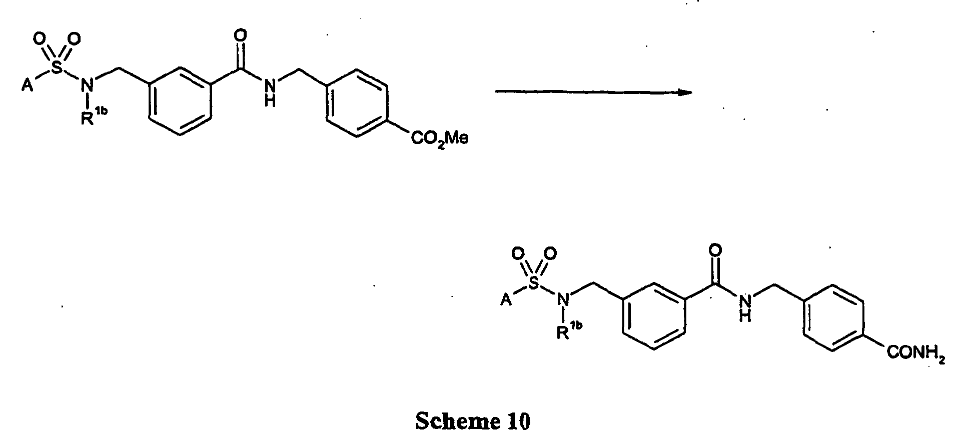

### Method HH - Carboxamide Formation.

A solution of the intermediate ester (0.01 mmol) in ammonium hydroxide (28% v/w, 2 ml) was heated to 80°C in a sealed tube overnight After cooling the mixture was concentrated *in vacuo*. The crude material was purified by column chromatography with DCM/ MeOH (up to 5% MeOH) as the eluent to give the title compound.

### Method II - Carboxamide Formation.

To a solution of the intermediate ester (0.01 mmol) in THF/ H2O (1:1, 2 ml) was added lithium hydroxide (2.5 eq) and the mixture heated to 80°C for 3 h. After cooling, the mixture was acidified to pH=4 with 1M HCl and extracted with ethyl acetate. The combined organic phases were dried (MgSO₄) and concentrated *in vacuo* to give the carboxylic acid.

To a solution of the carboxylic acid (0.01 mmol) in THF (2 ml) at -15°C was added methyl chloroformate (1.2 eq) and TEA (1.5 eq) and the mixture was allowed to warm to room temperature for 15 min. The reaction was cooled to 0°C and gaseous ammonia was bubbled through the solution for 10 min. The mixture was allowed to warm to room temperature and was concentrated *in vacuo*. The crude product was purified by column chromatography with DCM/ MeOH as the eluent to give the title compound.

Compounds synthesised using route 9 are detailed below:

### 2HH. 4-({3-[(3-Chloro-2-methyl-benzenesulfonylamino)-methyl]-benzoylamino}-methyl)-benzamide

The compound was synthesised from intermediate 2G3 using method HH to give title compound 2HH (4 mg, 4%) LC/MS: 96% MH⁺, m/z 472, Rt = 1.90 mins.

### 24II. 4-[(3-{[(3-Chloro-2-methyl-benzenesulfonyl)-methyl-amino]-methyl}-benzoylamino)-methyl]-benzamide

The compound was synthesised from intermediate 24G6 using method HH to give title compound 24II (25 mg, 52%) LC/MS: 100% MH⁺, m/z 486, Rt = 2.07 mins.

### 25II. 4-[(3-{[(Benzo[b]thiophene-2-sulfonyl)-methyl-amino]-methyl}-benzoylamino)-methyl]-benzamide

The compound was synthesised from intermediate 25G5 using method HH to give title compound 25II (28 mg, 57%) LC/MS: 98% MH⁺, m/z 494, Rt = 2.10 mins.

### 26II. 4-[(3-{[Methyl-(naphthalene-2-sulfonyl)-amino]-methyl}-benzoylamino)-methyl]-benzoic acid

The compound was synthesised from intermediate 26G5 using method HH to give title compound 26II (33 mg, 68%) LC/MS: 94% MH⁺, m/z 488, Rt = 2.07 mins.

### 27II. 4-[(3-{[(5-Chloro-3-methyl-benzo[b]thiophene-2-sulfonyl)-methyl-amino]-methyl}-benzoylamino)-methyl]-benzamide

The compound was synthesised from intermediate 27G5 using method HH to give title compound 27II (20 mg, 41 %) LC/MS: 97% MH⁺, m/z 542, Rt = 2.27 mins.

### 34HH. 4-{[3-((Decahydroisoquinoline-2-sulfonyl)aminomethyl)-benzoylamino]-methyl}-benzamide

The compound was synthesised from intermediate 34H3 using method HH to give title compound 34HH (57 mg, 20%) LC/MS: 100% MH⁺, m/z 485, Rt = 2.04 mins.

### 35HH. 4-{[3-((1,2,3,4-tetrahydroisoquinoline-2-sulfonyl)aminomethyl)-benzoylamino]-methyl}-benzamide

The compound was synthesised from intermediate 35H4 using method HH to give title compound 35HH (13 mg, 7%) LC/MS: 100% MH⁺, m/z 479, Rt =1.90 mins.

### Synthetic Route 10

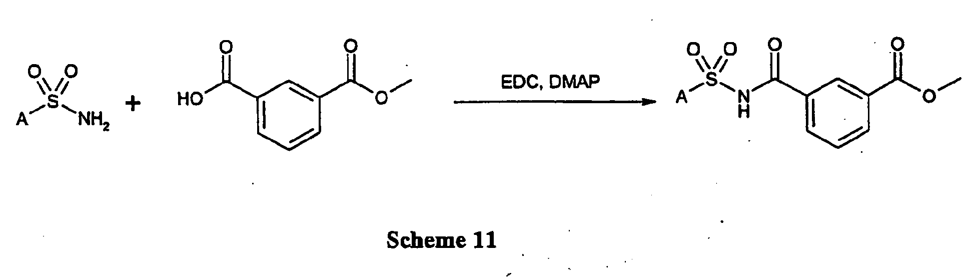

### Method JJ - Acylsulfonamide Formation.

To a solution of *mono*-methyl isophthalate (0.8 g, 4.44 mmol, 1.2 eq) in DCM/ DMF (3:1, 16 ml) was added EDC (1.2 eq), DMAP (1.2 eq) and the sulfonamide (1 eq) and the mixture was stirred at room temperature for 16 h. The reaction mixture was concentrated *in vacuo* and the residue was redissolved in DCM. The organic phase was washed with water and 1M NaOH, dried (Na₂SO₄) and concentrated *in vacuo.* The crude material was purified by column chromatography with DCM/ MeOH (+1% AcOH) as the eluent to give the title compound.

### 36JJ. 3-(3-Chloro-2-methyl-benzenesulfonylaminocarbonyl)-benzoic acid methyl ester

The compound was synthesised using method JJ to give title compound 36JJ (1.10 g, 76%) LC/MS: 94% MH⁺, m/z 368, Rt = 2.06 mins.

### 37JJ. 3-(2,4,6-Trichloro-benzenesulfonylaminocarbonyl)-benzoic acid methyl ester

The compound was synthesised using method JJ to give title compound 37JJ (0.89 g, 57%) LC/MS: 100% MH⁺, m/z 422, Rt = 2.12 mins.

### Example 4: 11 β-HSD Assays

### Biochemical assays monitoring the mouse 11β-HSD type 1 reductase, human 11β-HSD type 2 dehydrogenase, human 11β-HSD type 1 reductase and dehydrogenase activity

As source for the respective enzyme the human embryonic kidney cell line, HEK 293, was transfected with the full length cDNAs for human and mouse 11β-HSD and human 11β-HSD2 followed by selection of stably expressing clones according to standard procedures.

Whole cell lysates of 11β-HSD cell lines were prepared by growing of cells in 15 cm dishes in culture medium (DMEM, 10% FCS and 600 µg/ml G418) until 70% confluency. To force expression of 11β-HSD, cells were incubated overnight with 1.5 mM sodium butyrate (Aldrich, cat. no. 30,341-0), a histone deacetylase inhibitor. Cells were then harvested by addition of 2 ml PBS with 2.5 mM EDTA per 15 cm dish for 5 to 10 min at RT. Cell suspensions were combined and after centrifugation by 1000g for 5 min the cell pellet was homogenized in 20 mM potassium phosphate buffer, pH 7.4, 5 mM EDTA, 250 mM sucrose (1.5 ml per cell pellet of five 15 cm dishes) using a Ultra Turrax Homogenizer. The whole cell lysate was frozen in liquid nitrogen and stored at -80°C in aliquots suitable for single use.

The enzymatic reaction was carried out in the presence of cofactors NAD⁺ (dehydrogenase activity) or NADPH (reductase activity) and the respective steroid substrates. In order to maintain an excess of the cofactor NADPH, NADPH was regenerated by adding glucose-6-phosphate (G6P) and G6P-dehydrogenase in non-limiting concentrations to the 11β-HSD1 reductase enzymatic reaction. All assay components and compounds were diluted in reaction buffer, 20 mM Tris-HCl (pH 7.4), 250 mM sucrose, 100 mM NaCl, 1 mM MgCl₂, 1 mM EDTA, 1 mM EGTA, and stored on ice prior to use.

The following reaction mixtures plus compounds in concentrations between 10 pM and 300 µM were prepared in a total volume of 102 µl and were incubated for 15 min at 37°C.

Human and mouse 11 β-HSD type 1 reductase activity:
100 µM NADPH (AppliChem, cat no. A1395,0500), 10 mM glucose-6-phosphate (Sigma cat no. G-7879), 0.37 U/ml glucose-6-phosphate-dehydrogenase (Fluka, cat. no 49275) and 3 µM 11-dehydrocorticosterone (Research Plus, cat. no. 3306-16) or 3 µM cortisone (Sigma, cat. no. C-2755) for mouse and human enzyme, respectively.
The compounds exemplified in this invention typically have IC₅₀ values < 100 µM

Human 11β-HSD type 1 and type 2 dehydrogenase activity:
500 µM NAD⁺ (Applichem, cat. no. A1124,0001) and 3 µM cortisol (Sigma, cat no H-0888).

The enzymatic reaction was started by adding 18 µl whole cell lysates, equivalent to 80.000 cells of the respective HSD overexpressing cell lines. After 60 minutes at 37°C the reaction was stopped by carbenoxolone (Sigma, cat. no C-4790), an inhibitor of all HSD enzymes analysed, in a final concentration of 100 µM.

To determine the extent of the enzymatic reactions, cortisole or corticosterone were measured in an ELISA-based assay principle. This cortisol/corticosterone ELISA makes use of a monoclonal antibody specifically binding to cortisol/corticosterone. The detection is competition-based, since cortisol or corticosterone in a sample compete with cortisol covalently attached to alkaline phosphatase (AP). The amount of alkaline phosphatase bound, was detected by a colourimetric measurement, using pNPP as phosphatase substrate.

96well ELISA plates (Falcon, cat. no. BD 353915) were coated with 5 µg/ml rabbit anti mouse IgG antibody (Sigma, cat. no. M-9637) in PBS overnight at 4°C. After aspiration of this solution the wells were blocked with 200 mM acetate citrate buffer, pH 6.0, 20% FCS and 2% gelatine for one hour at RT. The plates were washed three times (50 mM Tris-HCl, pH 7.8, 0.1% Tween 20) and 100 µl of samples and standards, cortisol or corticosterone ranging from 78 nM to 10 µM, were added. The stocks of Cortisol-AP conjugate (Fitzgerald, cat. no. 65-IC07) and mouse monoclonal anti-cortisol antibody (Biotrend cat. no. 2330-4879) were diluted 1:1600 or 1:3600, respectively, in 100 mM Tris-HCl, pH 7.7, 10 mM MgCl₂, 0.1% BSA. 50 µl of each dilution were added per well. After a incubation of two hours at RT under shaking, plates were washed three times (50 mM Tris-HCl, pH 7.8, 0.1% Tween 20).

For detection, 200 µl of 25 mM Glycin, pH 10.3, 10 mM pNPP (Fluka, cat. no. 71768), 1 mM ZnSO₄ and 1 mM MgSO₄, were added and incubated for two hours at RT under shaking. Colourimetric measurement was performed in a microplate reader at 405 nm (Versamax, Molecular Devices). The concentrations of cortisol or corticosterone were calculated with SoflmaxPro software (Molecular Devices) relative to standard values. The inhibitory potency of 11 β-HSD inhibitors was determined by IC50 calculation with Prism 4.0 software (GrraphPad).

The compounds exemplified in this invention show no activity against the 11β-HSD-2 enzyme.

### Cell-based assay with human 11β-HSD type 1 stably expressing HEK293 cells

30.000 11β-HSD1 stably expressing HEK293 cells were seeded per 96 well in 100 µl culture medium (DMEM, 10% FCS and 600 µg/ml G418). After overnight incubation at 37°C and 5% CO₂ 11β-HSD inhibitors in final concentrations between 10 pM and 300 µM, 3 µM cortisone and 20 mM HEPES were added to the culture medium. All components, i.e. inhibitors, steroid substrate and HEPES were pre-diluted in DMEM prior to use. After incubation of 4 h at 37°C the culture medium was diluted 1:10 in 100 mM Tris-HCl, pH 7.7, 10 mM MgCl₂ and 0.1 % BSA. The determination of cortisol concentrations was carried out with a commercially available Cortisol ELISA (Assay Designs, cat. no. 901071) according to the recommended protocol. The concentrations of cortisol were calculated with SoftmaxPro software (Molecular Devices), the inhibitory potency of 11β-HSD inhibitors was determined by IC50 calculation with Prism 4.0 software (GraphPad).

The compounds exemplified in this invention typically have IC₅₀ values < 50 µM.

### Cell-based assay with a human adipocyte cell line, endogenously expressing 11β-HSD1

Human SGBS preadipocytes were differentiated to mature adipocytes as described by Wabitsch et. al. (Wabitsch M, Brenner RE, Melzner I, Braun M, Moller P, Heinze E, Debatin KM, Hauner H.: Characterization of a human preadipocyte cell strain with high capacity for adipose differentiation. Int J Obes Relat Metab Disord. 2001 Jan;25(1):8-15). 3500 cells were seeded per 96 well and cultured for 3 days in 200 µl culture medium (DMEM/Nutrient Mix F12, 100 U/ml penicillin, 100 µg/ml streptomycin, 17 µM biotin, 33 µM pantothenate, 10% none heat inactivated fetal calf serum) until confluence (day 0). Differentiation was initiated by medium change to 100 µl differentiation medium (DMEM/Nutrient Mix F12, 100 U/ml penicillin, 100 µg/ml streptomycin, 17 µM biotin, 33 µM pantothenate, 0.01 mg/ml transferrin, 100 nM hydrocortisone, 20 nM human insulin, 0.2 nM T3, 25 nM dexamethasone, 250 µM IBMX, 3 µM rosiglitazone). On day 4 of differentiation, the medium was changed to post-differentiation medium (DMEM/Nutrient Mix F12, 100 U/ml penicillin, 100 µg/ml streptomycin, 17 µM biotin, 33 µM pantothenate, 0.01 mg/ml transferrin, 100 nM hydrocortisone, 20 nM human insulin, 0.2 nM T3). The assay was performed with differentiated adipocytes between day 11 and day 15 of differentiation in assay medium (DMEM/Nutrient Mix F12, 17 µM biotin, 33 µM pantothenate, 0.01 mg/ml transfer 20 nM human insulin, 0.2 nM T3, 0.5 µM cortisone). The procedures for incubation with compound, determination of cortisol concentrations and data analysis were performed identically to the cellular assay with HEK293 cells described above.

The compounds exemplified in this invention typically have IC₅₀ values < 50 µM.

## Claims

1. A compound of formula (I) 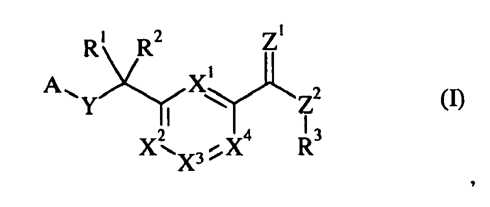 or a pharmaceutically acceptable salt thereof, wherein
X¹, X², X³, X⁴ are independently selected from the group consisting of N; and CR⁴, wherein R⁴ is independently H; halogen; CN; C(O)OR^{4a}; C(O)N(R^{4a}R^{4b}); OR^{4a}; N(R^{4a}R^{4b}); phenyl; C₁₋₆ alkyl; or C₃₋₇ cycloalkyl, wherein C₁₋₆ alkyl and C₃₋₇ cycloalkyl are optionally substituted with one or more halogen, which are the same or different;
R^{4a}, R^{4b} are independently selected from the group consisting of H; C₁₋₆ alkyl; and C₃₋₇ cycloalkyl;
R¹, R² are independently selected from the group consisting of H; C₁₋₆ alkyl; C₃₋₇ cycloalkyl; and halogen, wherein C₁₋₆ alkyl and C₃₋₇ cycloalkyl are optionally substituted with halogen;
Optionally R¹, R² are combined to form oxo (=O);
Y is -N(R^{1a})S(O)₂-; -S(O)₂N(R^{1b})-; or-N(R^{1a})S(O)₂N(R^{1b})-;
A is T; or C₁₋₆ alkyl, wherein C₁₋₆ alkyl is optionally substituted with one or more R⁵, wherein R⁵ is independently halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); or T;
R^{1a}, R^{1b} are independently selected from the group consisting of H; allyl; benzyl; CH₂-C(O)NH₂; CH₂-COOH; CH₂-C(O)O-C₁₋₆ alkyl; C₁₋₆ alkyl; and C₃₋₇ cycloalkyl, wherein C₁₋₆alkyl and C₃₋₇ cycloalkyl are optionally substituted with one or more R^{1c}, wherein R^{1c} is independently halogen; C₁₋₆ alkyl; -CH=CH₂; phenyl; C(O)NH₂; COOH; C(O)O-C₁₋₆ alkyl or C₃₋₇ cycloalkyl;
Optionally A and R^{1a} form together with the nitrogen to which they are attached to a heterocycle or heterobicycle optionally substituted with one or more R⁷, which are the same or different;
R⁶, R^{6a} R^{6b} are independently selected from the group consisting of H; C₁₋₆ alkyl; and T, wherein C₁₋₆ alkyl is optionally substituted with one or more halogen, which are the same of different;
T is C₃₋₇ cycloalkyl; heterocyclyl; heterobicyclyl; phenyl; naphthyl; indenyl; indanyl; tetralinyl; decalinyl; or adamantyl, wherein T is optionally substituted with one or more R⁷, which are the same or different;
R⁷ is independently halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), where the ring is at least partially saturated; C(O)R⁸; T¹; or C₁₋₆ alkyl, wherein C₁₋₆ alkyl is optionally substituted with one or more R⁹;
R⁸, R^{8a}, R^{8b} are independently selected from the group consisting of H; T¹; and C₁₋₆ alkyl, wherein C₁₋₆ alkyl is optionally substituted with one or more R¹⁰;
R⁹, R¹⁰ are independently selected from the group consisting of halogen; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); and T¹;
R¹¹, R^{11a}, R^{11b} are independently selected from the group consisting of H; C₁₋₆ alkyl; and T¹;
T¹ is C₃₋₇ cycloalkyl; heterocyclyl; heterobicyclyl; phenyl; naphthyl; indenyl; indanyl; tetralinyl; decalinyl; or adamantyl, wherein T¹ is optionally substituted with one or more R¹², wherein R¹² is independently halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}_{;} OC(O)N(R¹³R^{13a}); oxo (=O), where the ring is at least partially saturated; C(O)R¹³; C₁₋₆alkyl; phenyl; C₃₋₇ cycloalkyl; or heterocyclyl, wherein C₁₋₆ alkyl; phenyl; C₃₋₇ cycloalkyl; and heterocyclyl are optionally substituted with one or more halogen, which are the same or different;
R¹³, R^{13a}, R^{13b} are independently selected from the group consisting of H; C₁₋₆ alkyl; phenyl; C₃₋₇ cycloalkyl; and heterocyclyl, wherein C₁₋₆ alkyl; phenyl; C₃₋₇ cycloalkyl; and heterocyclyl are optionally substituted with one or more halogen, which are the same or different;
Z¹ is =O or =S;
Optionally Z¹ is =N-R¹⁵ and R¹⁵, R³ jointly form together with the atoms to which they are attached a heterocycle or heterobicycle, wherein the heterocycle or heterobicycle is optionally substituted with one or more R¹⁶, provided that the heterocycle is other than 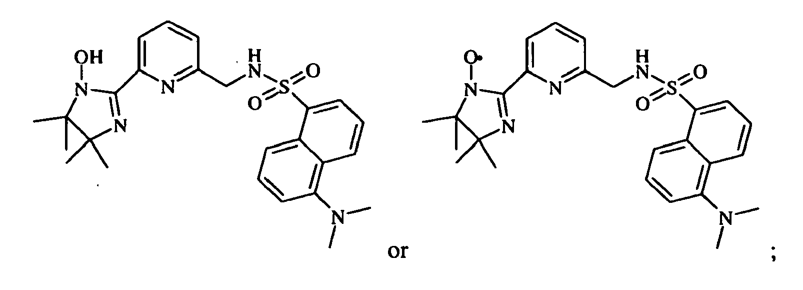
Z² is O; S; or N-R¹⁴;
R³, R¹⁴ are independently selected from the group consisting of H; T²; C₁₋₆ alkyl, wherein C₁₋₆alkyl is optionally substituted with one or more R¹⁷, provided that when one of R³ and R¹⁴ is H and the other is ethyl, R¹⁷ is other than -COOH;
Optionally R³, R¹⁴ jointly form together with the nitrogen to which they are attached a heterocycle or heterobicycle, wherein the heterocycle or heterobicycle is optionally substituted with one or more R¹⁸, provided that the heterobicycle is other than unsubstituted or in 3,6 and/or 7 position substituted 1,2,3,4-tetrahydroquinoline and its 4-oxa, -thia, -aza and/or 8-aza derivatives;
T² is C₃₋₇ cycloalkyl, heterocyclyl; heterobicyclyl; phenyl; naphthyl; indenyl; indanyl; tetralinyl; decalinyl; or adamantyl, wherein T² is optionally substituted with one or more R¹⁹; provided that when A is phenyl; Y is -S(O)₂NH-, wherein the sulfur is attached to A; R¹, R² are H; X¹, X³, X⁴ are CH; X² is C-R⁴, wherein R⁴ is OCH₃; Z¹ is =O; and Z² is NH, T² is other than unsubstituted phenyl;
R¹⁶, R¹⁸ , R¹⁹ are independently selected from the group consisting of halogen; CN; COOR²⁰; OR²⁰; C(O)N(R²⁰R^{20a}); S(O)₂N(R²⁰R^{20a}); S(O)N(R²⁰R^{20a}); S(O)₂R²⁰; N(R²⁰)S(O)₂N(R^{20a}R^{20b}); SR²⁰; N(R²⁰R^{20a}); OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); oxo (=O), where the ring is at least partially saturated; C(O)R²⁰; C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²¹;
R²⁰, R^{20a}, R^{20b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more halogen, which are the same or different;
R¹⁷, R²¹ are independently selected from the group consisting of halogen; CN; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²_{;} N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R²¹); C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²³;
R²², R^{22a}, R^{22b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more R^{23a};
R²³, R^{23a} are independently selected from the group consisting of halogen; CN; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a})_{;} OC(O)R²⁴_{;} N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); and C₁₋₆ alkyl, wherein C₁₋₆ alkyl is optionally substituted with one or more halogen, which are the same or different;
R²⁴, R^{24a}, R^{24b} are independently selected from the group consisting of H; C₁₋₆ alkyl, wherein C₁₋₆ alkyl is optionally substituted with one or more halogen, which are the same or different,
provided that the compound of formula (I) is other than N-[1-(3-aminocarbonyl-4-hydroxyphenyl)-2,2,2-trichloroethyl]-4-chlorobenzenesulfonamide.

2. A compound according to claim 1, wherein at most one of X¹, X², X³ and X⁴ are N.

3. A compound according to claim 1 or 2, wherein R¹ and R² are H or combined to form =O.

4. A compound according to any of claims 1 to 3, wherein R⁴ is F; Cl; Br; CH₃; OCH₃, NH₂; or phenyl.

5. A compound according to any of claims 1 to 4, wherein Y is -*S(O)₂-NH- or -*S(O)₂-N(CH₃)- wherein the atom marked with an asterisk is attached to A.

6. A compound according to any of claims 1 to 5, wherein A is phenyl, adamantyl; C₃₋₇ cycloalkyl; heterocyclyl; or C₁₋₆ alkyl, wherein C₁₋₆ alkyl is optionally substituted with T.

7. A compound according to claim 6, wherein A is phenyl; cyclohexyl; thiophenyl; benzothiophenyl; naphthyl; diazolyl; or pyridyl.

8. A compound according to any of claims 1 to 7, wherein A is T and T is optionally substituted with up to 3 R⁷, independently being F; Cl; CH₃; OCH₃; N(CH₃)₂; T¹; OT¹; or NHT¹.

9. A compound according to claim 8, wherein T¹ is phenyl, which is optionally substituted with up to three halogens, independently being F; and Cl.

10. A compound according to any of claims 1 to 9, wherein A is T and T is heterobicyclyl.

11. A compound according to claim 10, wherein heterobicyclyl is decahydroquinoline or tetrahydroisoquinoline and wherein the ring nitrogen is directly linked to the sulphur of the sulphonamide group representing Y.

12. A compound according to any of claims 1 to 11, wherein Z¹ is =O and Z² is -NR¹⁴_{.}

13. A compound according to any of claims 1 to 12, wherein R¹⁴ is H.

14. A compound according to any of claims 1 to 13, wherein R³ is phenyl; benzyl; heterocyclyl; C₃₋₇ cycloalkyl; or C₁₋₆ alkyl, wherein C₁₋₆ alkyl is optionally substituted with O-C₁₋₆ alkyl or C₃₋₇ cycloalkyl.

15. A compound according to any of claims 1 to 14, wherein R³ is T² and T² is substituted with up to three R¹⁹, independently being F; Cl; CH₃; OCH₃; N(CH₃)₂; C(O)OH; C(O)OCH₃; CF₃; or C(O)NH₂.

16. A compound according to claim 15, wherein T² is phenyl; cyclohexyl; or pyridyl.

17. A compound according to any of claims 1 to 16, wherein R³, R¹⁴ are independently H or C₁₋₆ alkyl, independently being ethyl; isopropyl; n-propyl; n-butyl, wherein C₁₋₆ alkyl is optionally substituted with N(CH₃)₂; or N(C₂H₅)₂.

18. A compound according to any of claims 1 to 17, wherein Z¹ is =N-R¹⁵ and R¹⁵, R³ jointly form a heterocycle.

19. A compound according to claim 18, wherein the heterocycle is oxazole; oxadiazole; diazole; or triazole, wherein the heterocycle is optionally substituted with one R¹⁶ being C₁₋₆ alkyl, optionally substituted with N(CH₃)₂ or N(CH₂CH₃)₂; C₃₋₇ cycloalkyl; phenyl; or heterocyclyl.

20. A compound according to claim 19, wherein R¹⁶ is methyl; ethyl; iso-propyl; cyclohexyl; phenyl; or piperidinyl.

21. A compound according to any of claims 1 to 20, wherein R³, R¹⁴ jointly form a heterocycle.

22. A compound according to claim 21, wherein the heterocycle is piperazine; morpholine; piperidine; or pyrrolidine, and wherein the heterocycle is optionally substituted with methyl.

23. A compound of formula (I) 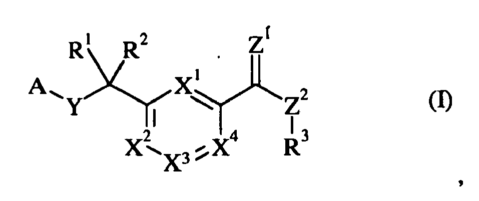 or a pharmaceutically acceptable salt thereof, wherein
X¹, X², X³, X⁴ are independently selected from the group consisting of N; and CR⁴, wherein R⁴ is independently H; halogen; CN; C(O)OR^{4a}; C(O)N(R^{4a}R^{4b}); OR^{4a}; N(R^{4a}R^{4b}); phenyl; C₁₋₆ alkyl; or C₃₋₇ cycloalkyl, wherein C₁₋₆ alkyl and C₃₋₇ cycloalkyl are optionally substituted with one or more halogen, which are the same or different;
R^{4a}, R^{4b} are independently selected from the group consisting of H; C₁₋₆ alkyl; and C₃₋₇ cycloalkyl;
R¹, R² are independently selected from the group consisting of H; C₁₋₆ alkyl; C₃₋₇ cycloalkyl; and halogen, wherein C₁₋₆ alkyl and C₃₋₇ cycloalkyl are optionally substituted with halogen;
Optionally R¹, R² are combined to form oxo (=O);
Y is -N(R^{1a})S(O)₂-; -S(O)₂N(R^{1b})-; or -N(R^{1b})S(O)₂N(R^{1b})-;
A is T; or C₁₋₆ alkyl, wherein C₁₋₆ alkyl is optionally substituted with one or more R⁵, wherein R⁵ is independently halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); or T;
R^{1a}, R^{1b} are independently selected from the group consisting of H; allyl; benzyl; CH₂-C(O)NH₂; CH₂-COOH; CH₂-C(O)O-C₁₋₆ alkyl; C₁₋₆ alkyl; and C₃₋₇ cycloalkyl, wherein C₁₋₆ alkyl and C₃₋₇ cycloalkyl are optionally substituted with one or more R^{1c}, wherein R^{1c} is independently halogen; C₁₋₆ alkyl; -CH=CH₂; phenyl; C(O)NH₂; COOH; C(O)O-C₁₋₆ alkyl or C₃₋₇ cycloalkyl;
Optionally A and R^{1a} form together with the nitrogen to which they are attached to a heterocycle or heterobicycle optionally substituted with one or more R⁷, which are the same or different;
R⁶, R^{6a} R^{6b} are independently selected from the group consisting of H; C₁₋₆ alkyl; and T, wherein C₁₋₆ alkyl is optionally substituted with one or more halogen, which are the same of different;
T is C₃₋₇ cycloalkyl; heterocyclyl; heterobicyclyl; phenyl; naphthyl; indenyl; indanyl; tetralinyl; decalinyl; or adamantyl, wherein T is optionally substituted with one or more R⁷, which are the same or different;
R⁷ is independently halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), where the ring is at least partially saturated; C(O)R⁸; T¹; or C₁₋₆ alkyl, wherein C₁₋₆ alkyl is optionally substituted with one or more R⁹;
R⁸, R^{8a}, R^{8b} are independently selected from the group consisting of H; T¹; and C₁₋₆ alkyl, wherein C₁₋₆ alkyl is optionally substituted with one or more R¹⁰;
R⁹, R¹⁰ are independently selected from the group consisting of halogen; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); and T¹;
R¹¹, R^{11a}, R^{11b} are independently selected from the group consisting of H; C₁₋₆ alkyl; and T¹;
T¹ is C₃₋₇ cycloalkyl; heterocyclyl; heterobicyclyl; phenyl; naphthyl; indenyl; indanyl; tetralinyl; decalinyl; or adamantyl, wherein T¹ is optionally substituted with one or more R¹², wherein R¹² is independently halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}; OC(O)N(R¹³R^{13a}); oxo (=O), where the ring is at least partially saturated; C(O)R¹³; C₁₋₆ alkyl; phenyl; C₃₋₇ cycloalkyl; or heterocyclyl, wherein C₁₋₆ alkyl; phenyl; C₃₋₇ cycloalkyl; and heterocyclyl are optionally substituted with one or more halogen, which are the same or different;
R¹³, R^{13a}, R^{13b} are independently selected from the group consisting of H; C₁₋₆ alkyl; phenyl; C₃₋₇ cycloalkyl; and heterocyclyl, wherein C₁₋₆ alkyl; phenyl; C₃₋₇ cycloalkyl; and heterocyclyl are optionally substituted with one or more halogen, which are the same or different;
Z¹ is =O or =S;
Optionally Z¹ is =N-R¹⁵ and R¹⁵, R³ jointly form together with the atoms to which they are attached a heterocycle or heterobicycle, wherein the heterocycle or heterobicycle is optionally substituted with one or more R¹⁶;
Z² is O; S; or N-R¹⁴;
R³, R¹⁴ are independently selected from the group consisting of H; T²; C₁₋₆ alkyl, wherein C₁₋₆ alkyl is optionally substituted with one or more R¹⁷, provided that when one of R³ and R¹⁴ is H and the other is ethyl, R¹⁷ is other than -COOH;
Optionally R³, R¹⁴ jointly form together with the nitrogen to which they are attached a heterocycle or heterobicycle, wherein the heterocycle or heterobicycle is optionally substituted with one or more R¹⁸, provided that the heterobicycle is other than unsubstituted or in 3,6 and/or 7 position substituted 1,2,3,4-tetrahydroquinoline and its 4-oxa, -thia, -aza and/or 8-aza derivatives;
T² is C₃₋₇ cycloalkyl, heterocyclyl; heterobicyclyl; phenyl; naphthyl; indenyl; indanyl; tetralinyl; decalinyl; or adamantyl, wherein T² is optionally substituted with one or more R¹⁹; provided that when A is phenyl; Y is -S(O)₂NH-, wherein the sulfur is attached to A; R¹, R² are H; X¹, X³, X⁴ are CH; X² is C-R⁴, wherein R⁴ is OCH₃; Z¹ is =O and Z² is NH, T² is other than unsubstituted phenyl;
R¹⁶, R¹⁸, R¹⁹ are independently selected from the group consisting of halogen; CN; COOR²⁰; OR²⁰; C(O)N(R²⁰R^{20a}); S(O)₂N(R²⁰R^{20a}); S(O)N(R²⁰R^{20a}); S(O)₂R²⁰; N(R²⁰)S(O)₂N(R^{20a}R^{20b}); SR²⁰; N(R²⁰R^{20a}); OC(O)R²⁰; N(R²⁰)C(O)R²⁰a; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); oxo (=O), where the ring is at least partially saturated; C(O)R²⁰; C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²¹; R²⁰, R^{20a}, R^{20b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more halogen, which are the same or different;
R¹⁷, R²¹ are independently selected from the group consisting of halogen; CN; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²³;
R²², R^{22a}, R^{22b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more R^{23a};
R²³, R^{23a} are independently selected from the group consisting of halogen; CN; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); and C₁₋₆ alkyl, wherein C₁₋₆ alkyl is optionally substituted with one or more halogen, which are the same or different;
R²⁴, R^{24a}, R^{24b} are independently selected from the group consisting of H; C₁₋₆ alkyl, wherein C₁₋₆ alkyl is optionally substituted with one or more halogen, which are the same or different,
for use as a medicament.

24. A pharmaceutical composition comprising a compound or a pharmaceutically acceptable salt thereof as defined in claim 23 together with a pharmaceutically acceptable carrier, optionally in combination with one or more other pharmaceutical compositions.

25. Use of a compound of formula (I) 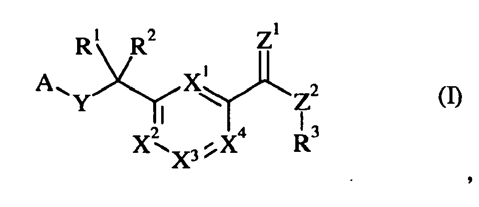 or a pharmaceutically acceptable salt thereof, wherein
X¹, X², X³, X⁴ are independently selected from the group consisting of N; and CR⁴, wherein R⁴ is independently H; halogen; CN; C(O)OR^{4a}; C(O)N(R^{4a}R^{4b})_{;} OR^{4a}; N(R^{4a}R^{4b}); phenyl; C₁₋₆ alkyl; or C₃₋₇ cycloalkyl, wherein C₁₋₆ alkyl and C₃₋₇ cycloalkyl are optionally substituted with one or more halogen, which are the same or different;
R^{4a}, R^{4b} are independently selected from the group consisting of H; C₁₋₆ alkyl; and C₃₋₇ cycloalkyl;
R¹, R² are independently selected from the group consisting of H; C₁₋₆ alkyl; C₃₋₇ cycloalkyl; and halogen, wherein C₁₋₆ alkyl and C₃₋₇ cycloalkyl are optionally substituted with halogen;
Optionally R¹, R² are combined to form oxo (=O);
Y is -N(R^{1a})S(O)₂-; -S(O)₂N(R^{1b})-; or -N(R^{1a})S(O)₂N(R^{1b})-;
A is T; or C₁₋₆ alkyl, wherein C₁₋₆ alkyl is optionally substituted with one or more R⁵, wherein R⁵ is independently halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{ba}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); or T;
R^{1a}, R^{1b} are independently selected from the group consisting of H; allyl; benzyl; CH₂-C(O)NH₂; CH₂-COOH; CH₂-C(O)O-C₁₋₆ alkyl; C₁₋₆ alkyl; and C₃₋₇ cycloalkyl, wherein C₁₋₆ alkyl and C₃₋₇ cycloalkyl are optionally substituted with one or more R^{1c}, wherein R^{1c} is independently halogen; C₁₋₆ alkyl; -CH=CH₂; phenyl; C(O)NH₂; COOH; C(O)O-C₁₋₆ alkyl or C₃₋₇ cycloalkyl;
Optionally A and R^{1a} form together with the nitrogen to which they are attached to a heterocycle or heterobicycle optionally substituted with one or more R⁷, which are the same or different;
R⁶, R^{6a} R^{6b} are independently selected from the group consisting of H; C₁₋₆ alkyl; and T, wherein C₁₋₆ alkyl is optionally substituted with one or more halogen, which are the same of different;
T is C₃₋₇ cycloalkyl; heterocyclyl; heterobicyclyl; phenyl; naphthyl; indenyl; indanyl; tetralinyl; decalinyl; or adamantyl, wherein T is optionally substituted with one or more R⁷, which are the same or different;
R⁷ is independently halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), where the ring is at least partially saturated; C(O)R⁸; T¹; or C₁₋₆ alkyl, wherein C₁₋₆ alkyl is optionally substituted with one or more R⁹_{;}
R⁸, R^{8a}, R^{8b} are independently selected from the group consisting of H; T¹; and C₁₋₆ alkyl, wherein C₁₋₆ alkyl is optionally substituted with one or more R¹⁰;
R⁹, R¹⁰ are independently selected from the group consisting of halogen; CN; COOR¹¹; OR¹¹; C(O)R¹¹; C(Q)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); and T¹;
R¹¹, R^{11a}, R^{11b} are independently selected from the group consisting of H; C₁₋₆ alkyl; and T¹;
T¹ is C₃₋₇ cycloalkyl; heterocyclyl; heterobicyclyl; phenyl; naphthyl; indenyl; indanyl; tetralinyl; decalinyl; or adamantyl, wherein T¹ is optionally substituted with one or more R¹², wherein R¹² is independently halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}; OC(O)N(R¹³R^{13a}); oxo (=O), where the ring is at least partially saturated; C(O)R¹³; C₁₋₆alkyl; phenyl; C₃₋₇ cycloalkyl; or heterocyclyl, wherein C₁₋₆ alkyl; phenyl; C₃₋₇ cycloalkyl; and heterocyclyl are optionally substituted with one or more halogen, which are the same or different;
R¹³, R^{13a}, R^{13b} are independently selected from the group consisting of H; C₁₋₆ alkyl; phenyl; C₃₋₇ cycloalkyl; and heterocyclyl, wherein C₁₋₆ alkyl; phenyl; C₃₋₇ cycloalkyl; and heterocyclyl are optionally substituted with one or more halogen, which are the same or different;
Z¹ is =O or =S;
Optionally Z¹ is =N-R¹⁵ and R¹⁵, R³ jointly form together with the atoms to which they are attached a heterocycle or heterobicycle, wherein the heterocycle or heterobicycle is optionally substituted with one or more R¹⁶;
Z² is O; S; or N-R¹⁴;
R³, R¹⁴ are independently selected from the group consisting of H; T²; C₁₋₆ alkyl, wherein C₁₋₆ alkyl is optionally substituted with one or more R¹⁷, provided that when one of R³ and R¹⁴ is H and the other is ethyl, R¹⁷ is other than -COOH;
Optionally R³, R¹⁴ jointly form together with the nitrogen to which they are attached a heterocycle or heterobicycle, wherein the heterocycle or heterobicycle is optionally substituted with one or more R¹⁸;
T² is C₃₋₇ cycloalkyl, heterocyclyl; heterobicyclyl; phenyl; naphthyl; indenyl; indanyl; tetralinyl; decalinyl; or adamantyl, wherein T² is optionally substituted with one or more R¹⁹; provided that when A is phenyl; Y is -S(O)₂NH-, wherein the sulfur is attached to A; R¹, R² are H; X¹, X³, X⁴ are CH; X² is C-R⁴, wherein R⁴ is OCH₃; Z¹ is =O; and Z² is NH, T² is other than unsubstituted phenyl;
R¹⁶, R¹⁸, R¹⁹ are independently selected from the group consisting of halogen; CN; COOR²⁰; OR²⁰; C(O)N(R²⁰R^{20a}); S(O)₂N(R²⁰R^{20a}); S(O)N(R²¹R^{20a}); S(O)₂R²⁰; N(R²⁰)S(O)₂N(R^{20a}R^{20b}); SR²⁰, N(R²⁰R^{20a}); OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); oxo (=O), where the ring is at least partially saturated; C(O)R²⁰; C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²¹;
R²⁰, R^{20a}, R^{20b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more halogen, which are the same or different;
R¹⁷, R²¹ are independently selected from the group consisting of halogen; CN; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a})_{;} OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²³_{;}
R²², R^{22a}, R^{22b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more R^{23a};
R²³, R^{23a} are independently selected from the group consisting of halogen; CN; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR²⁴a; OC(O)N(R²⁴R^{24a}); and C₁₋₆ alkyl, wherein C₁₋₆ alkyl is optionally substituted with one or more halogen, which are the same or different;
R²⁴, R^{24a}, R^{24b} are independently selected from the group consisting of H; C₁₋₆ alkyl, wherein C₁₋₆ alkyl is optionally substituted with one or more halogen, which are the same or different,
for the manufacture of a medicament for the treatment or prophylaxis of non-insulin dependent diabetes mellitus, hyperglycemia, low glucose tolerance, insulin resistance, obesity, lipid disorders, dyslipidemia, hyperlipidemia, hypertriglyceridemia, hypercholesterolemia, low HDL levels, high LDL levels, atherosclerosis and its sequelae, vascular restenosis, pancreatitis, abdominal obesity, neurodegenerative disease, retinopathy, nephropathy, neuropathy, Syndrome X, Alzheimer's disease, osteoporosis, cancer, epilepsy, depression, HAART-associated lipodystrophy or other conditions and disorders where insulin resistance is a component or that may be treated by inhibition of the 11β-HSD1 enzyme.

26. A pharmaceutical composition comprising a compound or a pharmaceutically acceptable salt thereof as defined in claim 25 together with a pharmaceutically acceptable carrier, further comprising one or more additional compounds or pharmaceutically acceptable salts thereof selected from the group consisting of compounds as defined in claim 25 and not being the first compound; other 11β-HSD1 inhibitors; DPP-IV inhibitors; insulin sensitizers, e.g. PPAR agonists and biguanides; insulin and insulin mimetics; sulfonylureas and other insulin secretagogues; α-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents, e.g. HMG-CoA reductase inhibitors, sequestrants, nicotinyl alcohol, nicotinic acid or a salt thereof, PPARγ agonists, PPARα/γ dual agonists, inhibitors of cholesterol absorption, acyl CoA: cholesterol acyltransferase inhibitors, and anti-oxidants; PPAR agonists; antiobesity compounds ; ileal bile acid transporter inhibitors; anti-inflammatory agents; and protein tyrosine phosphatase-1B (PTP-1B) inhibitors; anti-hypertensive compounds; and compounds for the treatment of cardiovascular diseases, particularly atherosclerosis.

27. A process for the preparation of a compound as defined in claim 25, comprising the steps of
• Reducing a compound of formula (II) 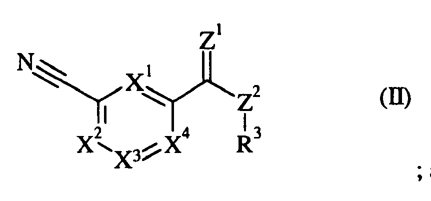 and
• Coupling the resulting amine (III) 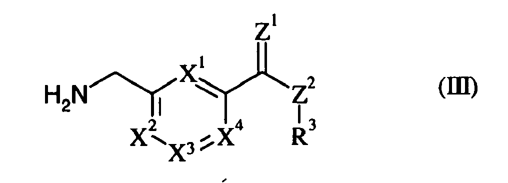 with A-Y-Cl, wherein A is T or C₁₋₆ alkyl; or
with A-H and SO₂Cl₂, wherein A is T-N(R^{1c})-.

## Patentansprüche

1. Verbindung der Formel (I) 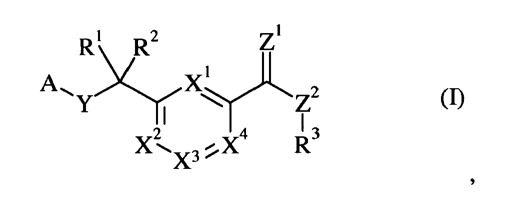 oder ein pharmaceutisch verträgliches Salz davon, worin
X¹, X², X³, X⁴ unabhängig ausgewählt sind aus der Gruppe bestehend aus N; und CR⁴, worin R⁴ unabhängig H; Halogen; CN; C(O)OR^{4a}; C(O)N(R^{4a}R^{4b}); OR^{4a}; N(R^{4a}R^{4b}); Phenyl; C₁₋₆ Alkyl; oder C₃₋₇ Cycloalkyl ist, worin C₁₋₆ Alkyl und C₃₋₇ Cycloalkyl gegebenenfalls mit einem oder mehreren Halogenen substituiert sind, welche gleich oder verschieden sind;
R^{4a}, R^{4b} unabhängig ausgewählt sind aus der Gruppe bestehend aus H; C₁₋₆ Alkyl; und C₃₋₇ Cycloalkyl;
R¹, R² unabhängig ausgewählt sind aus der Gruppe bestehend aus H; C₁₋₆ Alkyl; C₃₋₇ Cycloalkyl; und Halogen, worin C₁₋₆ Alkyl und C₃₋₇ Cycloalkyl gegebenenfalls mit Halogen substituiert sind;
Gegebenenfalls R¹, R² verbunden sind, um oxo (=O) zu bilden;
Y -N(R^{1a})S(O)₂-; -S(O)₂N(R^{1b})-; oder -N(R^{1a})S(O)₂N(R^{1b})- ist;
A T; oder C₁₋₆ alkyl ist, wobei C₁₋₆ Alkyl gegebenenfalls mit einem oder mehreren R⁵ substituiert ist, worin R⁵ unabhängig Halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); oder T ist;
R^{1a}, R^{1b} unabhängig ausgewählt sind aus der Gruppe bestehend aus H; Allyl; Benzyl; CH₂-C(O)NH₂; CH₂-COOH; CH₂-C(O)O-C₁₋₆ Alkyl; C₁₋₆ Alkyl; und C₃₋₇ Cycloalkyl, worin C₁₋₆ Alkyl und C₃₋₇ Cycloalkyl gegebenenfalls mit einem oder mehreren R^{1c} substituiert sind, worin R^{1c} unabhängig Halogen; C₁₋₆ Alkyl; - CH=CH₂; Phenyl; C(O)NH₂; COOH; C(O)O-C₁₋₆ Alkyl oder C₃₋₇ Cycloalkyl ist;
Gegebenenfalls A and R^{1a} gemeinsam mit dem Stickstoff, an den sie gebunden sind, einen Heterocyclus oder Heterobicyclus bilden, die gegebenenfalls mit einem oder mehreren R⁷ substituiert sind, welche gleich oder verschieden sind;
R⁶, R^{6a} R^{6b} unabhängig ausgewählt sind aus der Gruppe bestehend aus H; C₁₋₆ Alkyl; und T, worin C₁₋₆ Alkyl gegebenenfalls mit einem oder mehreren Halogenen substituiert ist, welche gleich oder verschieden sind;
T C₃₋₇ Cycloalkyl; Heterocyclyl; Heterobicyclyl; Phenyl; Naphthyl; Indenyl; Indanyl; Tetralinyl; Decalinyl; oder Adamantyl ist, worin T gegebenenfalls mit einem oder mehreren R⁷ substituiert ist, welche gleich oder verschieden sind;
R⁷ ist unabhängig Halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), wo der Ring zumindest teilweise gesättigt ist; C(O)R⁸; T¹; oder C₁₋₆ Alkyl, worin C₁₋₆ Alkyl gegebenenfalls mit einem oder mehreren R⁹ substituiert ist;
R⁸, R^{8a}, R^{8b} unabhängig ausgewählt sind aus der Gruppe bestehend aus H; T¹; und C₁₋₆ Alkyl, worin C₁₋₆ Alkyl gegebenenfalls mit einem oder mehreren R¹⁰ substituiert ist;
R⁹, R¹⁰ unabhängig ausgewählt sind aus der Gruppe bestehend aus Halogen; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); und T¹;
R¹¹, R^{11a}, R^{11b} unabhängig ausgewählt sind aus der Gruppe bestehend aus H; C₁₋₆ Alkyl; und T¹;
T¹ C₃₋₇ Cycloalkyl; Heterocyclyl; Heterobicyclyl; Phenyl; Naphthyl; Indenyl; Indanyl; Tetralinyl; Decalinyl; oderr Adamantyl ist, worin T¹ gegebenenfalls mit einem oder mehreren R¹² substituiert ist, worin R¹² unabhängig Halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}; OC(O)N(R¹³R^{13a}); oxo (=O), wo der Ring zumindest teilweise gesättigt ist; C(O)R¹³; C₁₋₆ Alkyl; Phenyl; C₃₋₇ Cycloalkyl; oder Heterocyclyl ist, worin C₁₋₆ Alkyl; Phenyl; C₃₋₇ Cycloalkyl; und Heterocyclyl gegebenenfalls mit einem oder mehreren Halogenen substituiert sind, welche gleich oder verschieden sind;
R¹³, R^{13a}, R^{13b} unabhängig ausgewählt sind aus der Gruppe bestehend aus H; C₁₋₆ Alkyl; Phenyl; C₃₋₇ Cycloalkyl; und Heterocyclyl, worin C₁₋₆ Alkyl; Phenyl; C₃₋₇ Cycloalkyl; und Heterocyclyl gegebenenfalls mit einem oder mehreren Halogenen substituiert sind, welche gleich oder verschieden sind;
Z¹ =O oder =S ist;
Gegebenenfalls Z¹ =N-R¹⁵ ist und R¹⁵, R³ gemeinsam mit den Atomen an die sie gebunden sind, einen Heterocyclus or Heterobicyclus bilden, worin der Heterocyclus oder Heterobicyclus gegebenenfalls mit einem oder mehreren R¹⁶ substituiert ist, mit der Maßgabe, dass der Heterocyclus nicht 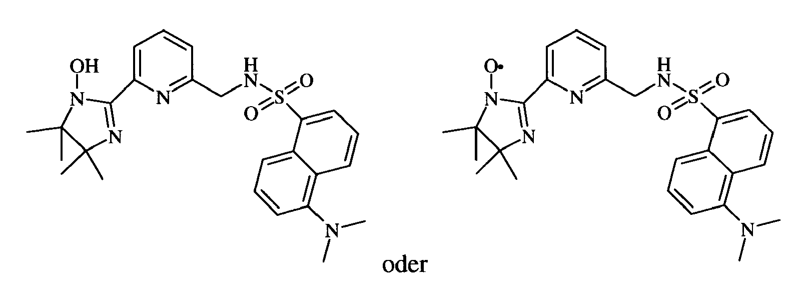 ist;
Z² O; S; oder N-R¹⁴ ist;
R³, R¹⁴ unabhängig ausgewählt sind aus der Gruppe bestehend aus H; T²; C₁₋₆ Alkyl, worin C₁₋₆ Alkyl gegebenenfalls mit einem oder mehreren R¹⁷ substituiert ist, mit der Maßgabe, dass R¹⁷ nicht -COOH ist; wenn einer von R³ und R¹⁴ H und der andere Ethyl ist;
Gegebenenfalls R³, R¹⁴ gemeinsam mit dem Stickstoff, an den sie gebunden sind, einen Heterocyclus oder Heterobicyclus bilden, worin der Heterocyclus oder Heterobicyclus gegebenenfalls mit einem oder mehreren R¹⁸ substituiert ist, mit der Maßgabe, dass der Heterobicyclus kein unsubstituiertes oder in 3,6 und/oder 7 Position substituiertes 1,2,3,4-Tetrahydrochinolin und eines deren 4-oxa, -thia, -aza und/oder 8-aza Derivative ist;
T² C₃₋₇ Cycloalkyl, Heterocyclyl; Heterobicyclyl; Phenyl; Naphthyl; Indenyl; Indanyl; Tetralinyl; Decalinyl; oder Adamantyl ist, worin T² gegebenenfalls mit einem oder mehreren R¹⁹ substituiert ist, mit der Maßgabe, dass T² kein unsubstituiertes Phenyl ist, wenn A Phenyl ist; Y -S(O)₂NH- ist, worin der Schwefel an A gebunden ist; R¹, R² H sind; X¹, X³, X⁴ CH sind; X² C-R⁴ ist, worin R⁴ OCH₃ ist; Z¹ =O ist; und Z² NH ist;
R¹⁶, R¹⁸, R¹⁹ unabhängig ausgewählt sind aus der Gruppe bestehend aus Halogen; CN; COOR²⁰; OR²⁰; C(O)N(R²⁰R^{20a}); S(O)₂N(R²⁰R²⁰a); S(O)N(R²⁰R^{20a}); S(O)₂R²⁰; N(R²⁰)S(O)₂N(R^{20a}R^{20b})_{;} SR²⁰; N(R²⁰R^{2oa}); OC(O)R²⁰; N(R²⁰)C₍O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); oxo (=O), wo der Ring zumindest teilweise gesättigt ist; C(O)R²⁰; C₁₋₆ Alkyl; Phenyl; Heterocyclyl; und C₃₋₇ Cycloalkyl, worin C₁₋₆ Alkyl; Phenyl; Heterocyclyl; und C₃₋₇ Cycloalkyl gegebenenfalls mit einem oder mehreren R²¹ substituiert sind;
R²⁰, R^{20a}, R^{20b} unabhängig ausgewählt sind aus der Gruppe bestehend aus H; Phenyl; Heterocyclyl; C₃₋₇ Cycloalkyl und C₁₋₆ Alkyl, worin Phenyl; Heterocyclyl; C₃₋₇ Cycloalkyl; und C₁₋₆ Alkyl gegebenenfalls mit einem oder mehreren Halogenen substituiert sind, welche gleich oder verschieden sind;
R¹⁷, R²¹ unabhängig ausgewählt sind aus der Gruppe bestehend aus Halogen; CN; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ Alkyl; Phenyl; Heterocyclyl; und C₃₋₇ Cycloalkyl, worin C₁₋₆ Alkyl; Phenyl; Heterocyclyl; und C₃₋₇ Cycloalkyl gegebenenfalls mit einem oder mehreren R²³ substituiert sind;
R²², R^{22a}, R^{22b} unabhängig ausgewählt sind aus der Gruppe bestehend aus H; Phenyl; Heterocyclyl; C₃₋₇ Cycloalkyl und C₁₋₆ Alkyl, worin Phenyl; Heterocyclyl; C₃₋₇ Cycloalkyl; und C₁₋₆ Alkyl gegebenenfalls mit einem oder mehreren R^{23a} substituiert sind;
R²³, R^{23a} unabhängig ausgewählt sind aus der Gruppe bestehend aus Halogen; CN; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); und C₁₋₆ Alkyl, worin C₁₋₆ Alkyl gegebenenfalls mit einem oder mehreren Halogenen substituiert ist, welche gleich oder verschieden sind;
R²⁴, R^{24a}, R^{24b} unabhängig ausgewählt sind aus der Gruppe bestehend aus H; C₁₋₆ Alkyl, worin C₁₋₆ Alkyl gegebenenfalls mit einem oder mehreren Halogenen substituiert ist, welche gleich oder verschieden sind;
mit der Maßgabe, dass die Verbindung der Formel (I) nicht N-[1-(3-aminocarbonyl-4-hydroxyphenyl)-2,2,2-trichlorethyl]-4-chlorbenzolsulfonamid ist.

2. Verbindung nach Anspruch 1, worin höchstens ein X¹, X², X³ und X⁴ N ist.

3. Verbindung nach Anspruch 1 oder 2, worin R¹ und R² H oder verbunden sind, um =O zu bilden.

4. Verbindung nach einem der Ansprüche 1 bis 3, worin R⁴ F; Cl; Br; CH₃; OCH₃, NH₂; oder Phenyl ist.

5. Verbindung nach einem der Ansprüche 1 bis 4, worin Y -*S(O)₂-NH- oder -*S(O)₂-N(CH₃)- ist, worin das mit Stern **gekennzeichnet**e Atom an A gebunden ist.

6. Verbindung nach einem der Ansprüche 1 bis 5, worin A Phenyl, Adamantyl; C₃₋₇ Cycloalkyl; Heterocyclyl; oder C₁₋₆ Alkyl ist, worin C₁₋₆ Alkyl gegebenenfalls mit T substituiert ist.

7. Verbindung nach Anspruch 6, worin A Phenyl; Cyclohexyl; Thiophenyl; Benzothiophenyl; Naphthyl; Diazolyl; oder Pyridyl ist.

8. Verbindung nach einem der Ansprüche 1 bis 7, worin A T ist und T gegebenenfalls mit bis zu 3 R⁷ substituiert ist, welche unabhängig F; Cl; CH₃; OCH₃; N(CH₃)₂; T¹; OT¹; oder NHT¹ sind.

9. Verbindung nach Anspruch 8, worin T¹ Phenyl ist, welches gegebenenfalls mit bis zu drei Halogenen substituiert ist, welche unabhängig F; und Cl sind.

10. Verbindung nach einem der Ansprüche 1 bis 9, worin A T ist und T Heterobicyclyl ist.

11. Verbindung nach Anspruch 10, worin Heterobicyclyl Decahydrochinolin or Tetrahydroisochinolin ist und worin der Ringstickstoff direkt mit dem Schwefel der Sulfonamidgruppe, welche Y bildet, verbunden ist.

12. Verbindung nach einem der Ansprüche 1 bis 11, worin Z¹ =O ist und Z² -NR¹⁴ ist.

13. Verbindung nach einem der Ansprüche 1 bis 12, worin R¹⁴ H ist.

14. Verbindung nach einem der Ansprüche 1 bis 13, worin R³ Phenyl; Benzyl; Heterocyclyl; C₃₋₇ Cycloalkyl; oder C₁₋₆ Alkyl ist, worin C₁₋₆ Alkyl gegebenenfalls mit O-C₁₋₆ Alkyl oder C₃₋₇ Cycloalkyl substituiert ist.

15. Verbindung nach einem der Ansprüche 1 bis 14, worin R³ T² ist und T² mit bis zu drei R¹⁹ substituiert ist, welche unabhängig F; Cl; CH₃; OCH₃; N(CH₃)₂; C(O)OH; C(O)OCH₃; CF₃; oder C(O)NH₂ sind.

16. Verbindung nach Anspruch 15, worin T² Phenyl; Cyclohexyl; oder Pyridyl ist.

17. Verbindung nach einem der Ansprüche 1 bis 16, worin R³, R¹⁴ unabhängig H oder C₁₋₆ Alkyl sind, welche unabhängig Ethyl; Isopropyl; n-Propyl; n-Butyl sind, worin C₁₋₆ Alkyl gegebenenfalls mit N(CH₃)₂; oder N(C₂H₅)₂ substituiert ist.

18. Verbidnung nach einem der Ansprüche 1 bis 17, worin Z¹ =N-R¹⁵ ist und R¹⁵, R³ gemeinsam einen Heterocyclus bilden.

19. Verbindung nach Anspruch 18, worin der Heterocyclus Oxazol; Oxadiazol; Diazol; oder Triazol ist, worin der Heterocyclus gegebenenfalls mit einem R¹⁶ substitiert ist, welcher C₁₋₆ Alkyl, das gegebenenfalls mit N(CH₃)₂ oder N(CH₂CH₃)₂ substituiert ist; C₃₋₇ Cycloalkyl; Phenyl; oder Heterocyclyl ist.

20. Verbindung nach Anspruch 19, worin R¹⁶ Methyl; Ethyl; iso-Propyl; Cyclohexyl; Phenyl; or Piperidinyl ist.

21. Verbindung nach einem der Ansprüche 1 bis 20, worin R³, R¹⁴ gemeinsam einen Heterocyclus bilden.

22. Verbidnung nach Anspruch 21, worin der Heterocyclus Piperazin; Morpholin; Piperidin; oder Pyrrolidin ist, und worin der Heterocyclus gegebenenfalls mit Methyl substituiert ist.

23. Verbindung der Formel (I) 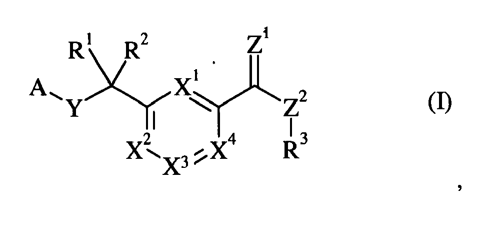 oder ein pharmaceutisch verträgliches Salz davon, worin
X¹, X², X³, X⁴ unabhängig ausgewählt sind aus der Gruppe bestehend aus N; und CR⁴, worin R⁴ unabhängig H; Halogen; CN; C(O)OR^{4a}; C(O)N(R^{4a}R^{4b}); OR^{4a}; N(R^{4a}R^{4b}); Phenyl; C₁₋₆ Alkyl; oder C₃₋₇ Cycloalkyl ist, worin C₁₋₆ Alkyl und C₃₋₇ Cycloalkyl gegebenenfalls mit einem oder mehreren Halogenen substituiert sind, welche gleich oder verschieden sind;
R^{4a}, R^{4b} unabhängig ausgewählt sind aus der Gruppe bestehend aus H; C₁₋₆ Alkyl; und C₃₋₇ Cycloalkyl;
R¹, R² unabhängig ausgewählt sind aus der Gruppe bestehend aus H; C₁₋₆ Alkyl; C₃₋₇ Cycloalkyl; und Halogen, worin C₁₋₆ Alkyl und C₃₋₇ Cycloalkyl gegebenenfalls mit Halogen substituiert sind;
Gegebenenfalls R¹, R² verbunden sind, um oxo (=O) zu bilden;
Y -N(R^{1a})S(O)₂-; -S(O)₂N(R^{1b})-; oder -N(R^{1a})S(O)₂N(R^{1b})- ist;
A T; oder C₁₋₆ Alkyl ist, wobei C₁₋₆ Alkyl gegebenenfalls mit einem oder mehreren R⁵ substituiert ist, worin R⁵ unabhängig Halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N^{6a}R^{6b}); N(R⁶)C(CO)OR^{6a}; OC(O)N(R⁶R^{6a}); oder T ist;
R^{1a}, R^{1b} unabhängig ausgewählt sind aus der Gruppe bestehend aus H; Allyl; Benzyl; CH₂-C(O)NH₂; CH₂-COOH; CH₂-C(O)O-C₁₋₆ Alkyl; C₁₋₆ Alkyl; und C₃₋₇ Cycloalkyl, worin C₁₋₆ Alkyl und C₃₋₇ Cycloalkyl gegebenenfalls mit einem oder mehreren R^{1c} substituiert sind, worin R^{1c} unabhängig Halogen; C₁₋₆ Alkyl; - CH=CH₂; Phenyl; C(O)NH₂; COOH; C(O)O-C₁₋₆ Alkyl oder C₃₋₇ Cycloalkyl ist;
Gegebenenfalls A and R^{1a} gemeinsam mit dem Stickstoff, an den sie gebunden sind, einen Heterocyclus oder Heterobicyclus bilden, die gegebenenfalls mit einem oder mehreren R⁷ substituiert sind, welche gleich oder verschieden sind;
R⁶, R^{6a} R^{6b} unabhängig ausgewählt sind aus der Gruppe bestehend aus H; C₁₋₆ Alkyl; und T, worin C₁₋₆ Alkyl gegebenenfalls mit einem oder mehreren Halogenen substituiert ist, welche gleich oder verschieden sind;
T C₃₋₇ Cycloalkyl; Heterocyclyl; Heterobicyclyl; Phenyl; Naphthyl; Indenyl; Indanyl; Tetralinyl; Decalinyl; oder Adamantyl ist, worin T gegebenenfalls mit einem oder mehreren R⁷ substituiert ist, welche gleich oder verschieden sind;
R⁷ ist unabhängig Halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), wo der Ring zumindest teilweise gesättigt ist; C(O)R⁸; T¹; oder C₁₋₆ Alkyl, worin C₁₋₆ Alkyl gegebenenfalls mit einem oder mehreren R⁹ substituiert ist;
R⁸, R^{8a}, R^{8b} unabhängig ausgewählt sind aus der Gruppe bestehend aus H; T¹; und C₁₋₆ Alkyl, worin C₁₋₆ Alkyl gegebenenfalls mit einem oder mehreren R¹⁰ substituiert ist;
R⁹, R¹⁰ unabhängig ausgewählt sind aus der Gruppe bestehend aus Halogen; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); und T¹;
R¹¹, R^{11a}, R^{11b} unabhängig ausgewählt sind aus der Gruppe bestehend aus H; C₁₋₆ Alkyl; und T¹;
T¹ C₃₋₇ Cycloalkyl; Heterocyclyl; Heterobicyclyl; Phenyl; Naphthyl; Indenyl; Indanyl; Tetralinyl; Decalinyl; oder Adamantyl ist, worin T¹ gegebenenfalls mit einem oder mehreren R¹² substituiert ist, worin R¹² unabhängig Halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a};N(R¹³)S(O)R^{13a};N(R¹³)C(O)N(R^{13a}R^{13b});N(R¹³)C(O)OR^{13a}; OC(O)N(R¹³R^{13a}); oxo (=O), wo der Ring zumindest teilweise gesättigt ist; C(O)R¹³; C₁₋₆ Alkyl; Phenyl; C₃₋₇ Cycloalkyl; oder Heterocyclyl ist, worin C₁₋₆ Alkyl; Phenyl; C₃₋₇ Cycloalkyl; und Heterocyclyl gegebenenfalls mit einem oder mehreren Halogenen substituiert sind, welche gleich oder verschieden sind;
R¹³, R^{13a}, R^{13b} unabhängig ausgewählt sind aus der Gruppe bestehend aus H; C₁₋₆ Alkyl; Phenyl; C₃₋₇ Cycloalkyl; und Heterocyclyl, worin C₁₋₆ Alkyl; Phenyl; C₃₋₇ Cycloalkyl; und Heterocyclyl gegebenenfalls mit einem oder mehreren Halogenen substituiert sind, welche gleich oder verschieden sind;
Z¹ =O oder =S ist;
Gegebenenfalls Z¹ =N-R¹⁵ ist und R¹⁵, R³ gemeinsam mit den Atomen an die sie gebunden sind, einen Heterocyclus or Heterobicyclus bilden, worin der Heterocyclus oder Heterobicyclus gegebenenfalls mit einem oder mehreren R¹⁶ substituiert ist;
Z² O; S; oder N-R¹⁴ ist;
R³, R¹⁴ unabhängig ausgewählt sind aus der Gruppe bestehend aus H; T²; C₁₋₆ Alkyl, worin C₁₋₆ Alkyl gegebenenfalls mit einem oder mehreren R¹⁷ substituiert ist, mit der Maßgabe, dass R¹⁷ nicht -COOH ist; wenn einer von R³ und R¹⁴ H und der andere Ethyl ist;
Gegebenenfalls R³, R¹⁴ gemeinsam mit dem Stickstoff, an den sie gebunden sind, einen Heterocyclus oder Heterobicyclus bilden, worin der Heterocyclus oder Heterobicyclus gegebenenfalls mit einem oder mehreren R¹⁸ substituiert ist, mit der Maßgabe, dass der Heterobicyclus kein unsubstituiertes oder in 3,6 und/oder 7 Position substituiertes 1,2,3,4-Tetrahydrochinolin und eines deren 4-oxa, -thia, -aza und/oder 8-aza Derivative ist;
T² C₃₋₇ Cycloalkyl, Heterocyclyl; Heterobicyclyl; Phenyl; Naphthyl; Indenyl; Indanyl; Tetralinyl; Decalinyl; oder Adamantyl ist, worin T² gegebenenfalls mit einem oder mehreren R¹⁹ substituiert ist, mit der Maßgabe, dass T² kein unsubstituiertes Phenyl ist, wenn A Phenyl ist; Y -S(O)₂NH- ist, worin der Schwefel an A gebunden ist; R¹, R² H sind; X¹, X³, X⁴ CH sind; X² C-R⁴ ist, worin R⁴ OCH₃ ist; Z¹ =O ist; und Z² NH ist;
R¹⁶, R¹⁸, R¹⁹ unabhängig ausgewählt sind aus der Gruppe bestehend aus Halogen; CN; COOR²⁰; OR²⁰; C(O)N(R²⁰R^{20a}); S(O)₂N(R²⁰R^{20a}); S(O)N(R²⁰R^{20a}); S(O)₂R²⁰; N(R²⁰)S(O)₂N(R^{20a}R^{20b}); SR²⁰; N(R²⁰R^{20a}); OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); oxo (=O), wo der Ring zumindest teilweise gesättigt ist; C(O)R²⁰; C₁₋₆ Alkyl; Phenyl; Heterocyclyl; und C₃₋₇ Cycloalkyl, worin C₁₋₆ Alkyl; Phenyl; Heterocyclyl; und C₃₋₇ Cycloalkyl gegebenenfalls mit einem oder mehreren R²¹ substituiert sind;
R²⁰, R^{20a}, R^{20b} unabhängig ausgewählt sind aus der Gruppe bestehend aus H; Phenyl; Heterocyclyl; C₃₋₇ Cycloalkyl und C₁₋₆ Alkyl, worin Phenyl; Heterocyclyl; C₃₋₇ Cycloalkyl; und C₁₋₆ Alkyl gegebenenfalls mit einem oder mehreren Halogenen substituiert sind, welche gleich oder verschieden sind;
R¹⁷, R²¹ unabhängig ausgewählt sind aus der Gruppe bestehend aus Halogen; CN; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ Alkyl; Phenyl; Heterocyclyl; und C₃₋₇ Cycloalkyl, worin C₁₋₆ Alkyl; Phenyl; Heterocyclyl; und C₃₋₇ Cycloalkyl gegebenenfalls mit einem oder mehreren R²³ substituiert sind;
R²², R^{22a}, R^{22b} unabhängig ausgewählt sind aus der Gruppe bestehend aus H; Phenyl; Heterocyclyl; C₃₋₇ Cycloalkyl und C₁₋₆ Alkyl, worin Phenyl; Heterocyclyl; C₃₋₇ Cycloalkyl; und C₁₋₆ Alkyl gegebenenfalls mit einem oder mehreren R^{23a} substituiert sind;
R²³, R^{23a} unabhängig ausgewählt sind aus der Gruppe bestehend aus Halogen; CN; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); und C₁₋₆ Alkyl, worin C₁₋₆ Alkyl gegebenenfalls mit einem oder mehreren Halogenen substituiert ist, welche gleich oder verschieden sind;
R²⁴, R^{24a}, R^{24b} unabhängig ausgewählt sind aus der Gruppe bestehend aus H; C₁₋₆ Alkyl, worin C₁₋₆ Alkyl gegebenenfalls mit einem oder mehreren Halogenen substituiert ist, welche gleich oder verschieden sind;
zur Verwendung als Medikament.

24. Pharmaceutische Zusammensetzung enthaltend eine Verbindung oder ein pharmaceutisch verträgliches Salz davon wie in Anspruch 23 definiert zusammen mit einem pharmaceutischen Träger, gegebenenfalls in Kombination mit einem oder mehreren anderen pharmaceutischen Zusammensetzungen.

25. Verwendung einer Verbindung der Formel (I) 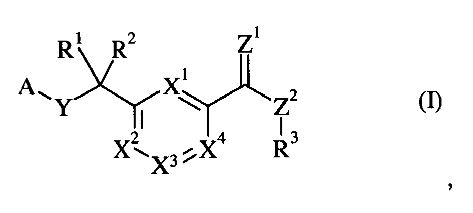 oder ein pharmaceutisch verträgliches Salz davon, worin
X¹, X², X³, X⁴ unabhängig ausgewählt sind aus der Gruppe bestehend aus N; und CR⁴, worin R⁴ unabhängig H; Halogen; CN; C(O)OR^{4a}; C(O)N(R^{4a}R^{4b}); OR^{4a}; N(R^{4a}R^{4b}); Phenyl; C₁₋₆ Alkyl; oder C₃₋₇ Cycloalkyl ist, worin C₁₋₆ Alkyl und C₃₋₇ Cycloalkyl gegebenenfalls mit einem oder mehreren Halogenen substituiert sind, welche gleich oder verschieden sind;
R^{4a}, R^{4b} unabhängig ausgewählt sind aus der Gruppe bestehend aus H; C₁₋₆ Alkyl; und C₃₋₇ Cycloalkyl;
R¹, R² unabhängig ausgewählt sind aus der Gruppe bestehend aus H; C₁₋₆ Alkyl; C₃₋₇ Cycloalkyl; und Halogen, worin C₁₋₆ Alkyl und C₃₋₇ Cycloalkyl gegebenenfalls mit Halogen substituiert sind;
Gegebenenfalls R¹, R² verbunden sind, um oxo (=O) zu bilden;
Y -N(R^{1a})S(O)₂-; -S(O)₂N(R^{1b})-; oder -N(R^{1a})S(O)₂N(R^{1b})- ist;
A T; oder C₁₋₆ alkyl ist, wobei C₁₋₆ Alkyl gegebenenfalls mit einem oder mehreren R⁵ substituiert ist, worin R⁵ unabhängig Halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); oder T ist;
R^{1a}, R^{1b} unabhängig ausgewählt sind aus der Gruppe bestehend aus H; Allyl; Benzyl; CH₂-C(O)NH₂; CH₂-COOH; CH₂-C(O)O-C₁₋₆ Alkyl; C₁₋₆ Alkyl; und C₃₋₇ Cycloalkyl, worin C₁₋₆ Alkyl und C₃₋₇ Cycloalkyl gegebenenfalls mit einem oder mehreren R^{1c} substituiert sind, worin R^{1c} unabhängig Halogen; C₁₋₆ Alkyl; - CH=CH₂; Phenyl; C(O)NH₂; COOH; C(O)O-C₁₋₆ Alkyl oder C₃₋₇ Cycloalkyl ist;
Gegebenenfalls A and R^{1a} gemeinsam mit dem Stickstoff, an den sie gebunden sind, einen Heterocyclus oder Heterobicyclus bilden, die gegebenenfalls mit einem oder mehreren R⁷ substituiert sind, welche gleich oder verschieden sind;
R⁶, R^{6a} R^{6b} unabhängig ausgewählt sind aus der Gruppe bestehend aus H; C₁₋₆ Alkyl; und T, worin C₁₋₆Alkyl gegebenenfalls mit einem oder mehreren Halogenen substituiert ist, welche gleich oder verschieden sind;
T C₃₋₇ Cycloalkyl; Heterocyclyl; Heterobicyclyl; Phenyl; Naphthyl; Indenyl; Indanyl; Tetralinyl; Decalinyl; oder Adamantyl ist, worin T gegebenenfalls mit einem oder mehreren R⁷ substituiert ist, welche gleich oder verschieden sind;
R⁷ ist unabhängig Halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), wo der Ring zumindest teilweise gesättigt ist; C(O)R⁸; T¹; oder C₁₋₆ Alkyl, worin C₁₋₆ Alkyl gegebenenfalls mit einem oder mehreren R⁹ substituiert ist;
R⁸, R^{8a}, R^{8b} unabhängig ausgewählt sind aus der Gruppe bestehend aus H; T¹; und C₁₋₆ Alkyl, worin C₁₋₆ Alkyl gegebenenfalls mit einem oder mehreren R¹⁰ substituiert ist;
R⁹, R¹⁰ unabhängig ausgewählt sind aus der Gruppe bestehend aus Halogen; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R11^{a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}_{;} OC(O)N(R¹¹R^{11a}); und T¹;
R¹¹, R^{11a}, R^{11b} unabhängig ausgewählt sind aus der Gruppe bestehend aus H; C₁₋₆ Alkyl; und T¹;
T¹ C₃₋₇ Cycloalkyl; Heterocyclyl; Heterobicyclyl; Phenyl; Naphthyl; Indenyl; Indanyl; Tetralinyl; Decalinyl; oder Adamantyl ist, worin T¹ gegebenenfalls mit einem oder mehreren R¹² substituiert ist, worin R¹² unabhängig Halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}; OC(O)N(R¹³R^{13a}); oxo (=O), wo der Ring zumindest teilweise gesättigt ist; C(O)R¹³; C₁₋₆ Alkyl; Phenyl; C₃₋₇ Cycloalkyl; oder Heterocyclyl ist, worin C₁₋₆ Alkyl; Phenyl; C₃₋₇ Cycloalkyl; und Heterocyclyl gegebenenfalls mit einem oder mehreren Halogenen substituiert sind, welche gleich oder verschieden sind;
R¹³, R^{13a}, R^{13b} unabhängig ausgewählt sind aus der Gruppe bestehend aus H; C₁₋₆ Alkyl; Phenyl; C₃₋₇ Cycloalkyl; und Heterocyclyl, worin C₁₋₆ Alkyl; Phenyl; C₃₋₇ Cycloalkyl; und Heterocyclyl gegebenenfalls mit einem oder mehreren Halogenen substituiert sind, welche gleich oder verschieden sind;
Z¹ =O oder =S ist;
Gegebenenfalls Z¹ =N-R¹⁵ ist und R¹⁵, R³ gemeinsam mit den Atomen an die sie gebunden sind, einen Heterocyclus or Heterobicyclus bilden, worin der Heterocyclus oder Heterobicyclus gegebenenfalls mit einem oder mehreren R¹⁶ substituiert ist;
Z² O; S; oder N-R¹⁴ ist;
R³,R¹⁴ unabhängig ausgewählt sind aus der Gruppe bestehend aus H; T²; C₁₋₆ Alkyl, worin C₁₋₆ Alkyl gegebenenfalls mit einem oder mehreren R¹⁷ substituiert ist, mit der Maßgabe, dass R¹⁷ nicht -COOH ist; wenn einer von R³ und R¹⁴ H und der andere Ethyl ist;
Gegebenenfalls R³, R¹⁴ gemeinsam mit dem Stickstoff, an den sie gebunden sind, einen Heterocyclus oder Heterobicyclus bilden, worin der Heterocyclus oder Heterobicyclus gegebenenfalls mit einem oder mehreren R¹⁸ substituiert ist;
T² C₃₋₇ Cycloalkyl, Heterocyclyl; Heterobicyclyl; Phenyl; Naphthyl; Indenyl; Indanyl; Tetralinyl; Decalinyl; oder Adamantyl ist, worin T² gegebenenfalls mit einem oder mehreren R¹⁹ substituiert ist, mit der Maßgabe, dass T² kein unsubstituiertes Phenyl ist, wenn A Phenyl ist; Y -S(O)₂NH- ist, worin der Schwefel an A gebunden ist; R¹, R² H sind; X¹, X³, X⁴ CH sind; X² C-R⁴ ist, worin R⁴ OCH₃ ist; Z¹ =O ist; und Z² NH ist;
R¹⁶, R¹⁸, R¹⁹ unabhängig ausgewählt sind aus der Gruppe bestehend aus Halogen; CN; COOR²⁰; OR²⁰; C(O)N(R²⁰R^{20a}); S(O)₂N(R²⁰R^{20a}); S(O)N(R²⁰R^{20a}); S(O)₂R²⁰; N(R²⁰)S(O)₂N(R^{20a}R^{20b}); SR²⁰; N(R²⁰R^{20a}); OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); oxo (=O), wo der Ring zumindest teilweise gesättigt ist; C(O)R²⁰; C₁₋₆ Alkyl; Phenyl; Heterocyclyl; und C₃₋₇ Cycloalkyl, worin C₁₋₆ Alkyl; Phenyl; Heterocyclyl; und C₃₋₇ Cycloalkyl gegebenenfalls mit einem oder mehreren R²¹ substituiert sind;
R²⁰, R^{20a}, R^{20b} unabhängig ausgewählt sind aus der Gruppe bestehend aus H; Phenyl; Heterocyclyl; C₃₋₇ Cycloalkyl und C₁₋₆ Alkyl, worin Phenyl; Heterocyclyl; C₃₋₇ Cycloalkyl; und C₁₋₆ Alkyl gegebenenfalls mit einem oder mehreren Halogenen substituiert sind, welche gleich oder verschieden sind;
R¹⁷, R²¹ unabhängig ausgewählt sind aus der Gruppe bestehend aus Halogen; CN; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); C₁₋₆ Alkyl; Phenyl; Heterocyclyl; und C₃₋₇ Cycloalkyl, worin C₁₋₆ Alkyl; Phenyl; Heterocyclyl; und C₃₋₇ Cycloalkyl gegebenenfalls mit einem oder mehreren R²³ substituiert sind;
R²², R^{22a}, R^{22b} unabhängig ausgewählt sind aus der Gruppe bestehend aus H; Phenyl; Heterocyclyl; C₃₋₇ Cycloalkyl und C₁₋₆ Alkyl, worin Phenyl; Heterocyclyl; C₃₋₇ Cycloalkyl; und C₁₋₆ Alkyl gegebenenfalls mit einem oder mehreren R^{23a} substituiert sind;
R²³, R^{23a} unabhängig ausgewählt sind aus der Gruppe bestehend aus Halogen; CN; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); und C₁₋₆ Alkyl, worin C₁₋₆ Alkyl gegebenenfalls mit einem oder mehreren Halogenen substituiert ist, welche gleich oder verschieden sind; R²⁴, R^{24a}, R^{24b} unabhängig ausgewählt sind aus der Gruppe bestehend aus H; C₁₋₆ Alkyl, worin C₁₋₆ Alkyl gegebenenfalls mit einem oder mehreren Halogenen substituiert ist, welche gleich oder verschieden sind;
zur Herstellung eines Medikaments für die Behandlung oder Vorbeugung von Insulin unabhängiger Diabetes mellitus, Hyperglykämie, geringe Glucose Toleranz, Insulin Resistenz, Fettleibigkeit, Lipid Funktionsstörungen, Dyslipidämie, Hyperlipidämie, Hypertriglyzeridämie, Hypercholesterinämie, geringen HDL Spiegeln, hohen LDL Spiegeln, Atherosclerose und deren Folgekrankheiten, Gefäßrestenose, Pankreatitis, Unterleibsfettleibigkeit, neurodegenerativer Krankheit, Retinopathie, Nephropathie, Neuropathie, Syndrom X, Alzheimer Krankheit, Osteoporose, Krebs, Epilepsie, Depression, HAART assoziierter Lipodystrophie oder anderen Leiden und Funktionsstörungen, bei denen Insulinresistenz eine Komponente ist, oder die durch Inhibition des 11β-HSD 1 Enzyms behandelt werden können.

26. Pharmaceutische Zusammensetzung enthaltend eine Verbindung oder deren pharmaceutisch verträgliches Salz wie in Anspruch 25 definiert zusammen mit einem pharmaceutisch verträglichen Träger, weiterhin eine oder mehrere zusätzliche Verbindungen oder deren pahrmaceutisch verträgliche Salze enthaltend ausgewählt aus der Gruppe bestehend aus Verbindungen wie in Anspruch 25 definiert, welche nicht die erste Verbindung sind; andere 11β-HSD1 Inhibitoren; DPP-IV Inhibitoren; Insulin Sensibilisierungsmittel, z.B. PPAR Agonisten and Biguanide; Insulin und Insulin Mimetika; Sulfonylharnstoffe und andere Insulin sekretanregende Mittel; α-Glucosidase Inhibitoren; Glucagon Rezeptor Antagonisten; GLP-1, GLP-1 Mimetika, und GLP-1 Rezeptor Agonisten; GIP, GIP Mimetika, und GIP Rezeptor Agonisten; PACAP, PACAP Mimetika, und PACAP Rezeptor 3 Agonisten; Cholestrerin senkende Mittel, z.B. HMG-CoA Reductase Inhibitoren, Komplexbildner, Nicotinylalkohol, Nicotinsäure oder ein Salz davon, PPARγ Agonisten, PPARα/γ Dualagonisten, Inhibitoren der Cholesterinabsorption, Acyl CoA: Cholesterinacyltransferase Inhibitoren, und Antioxidanzien; PPAR Agonisten; Antifettleibigkeitsmittel ; Ileal Gallensäure Transporter Inhibitoren; entzündungshemmende Mittel; und Protein Tyrosin Phosphatase-1B (PTP-1B) Inhibitoren; antihypertensive Verbindungen; und Verbindungen zur Behandlung von cardiovaskulären Krankheiten, insbesondere Atherosclerose.

27. Verfahren zur Herstellung einer Verbindung wie in Anspruch 25 definiert, die Schritte enthaltend
• Reduktion einer Verbindung der Formel (II) 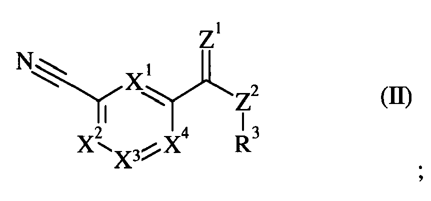 und
• Kupplung des erhaltenen Amins (III) 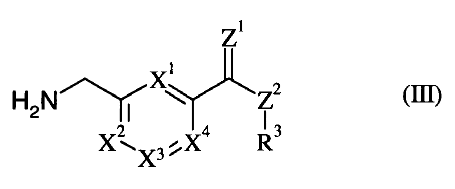 mit A-Y-Cl, worin A T or C₁₋₆ alkyl ist; oder
mit A-H und SO₂Cl₂, worin A T-N(R^{1c})- ist.

## Revendications

1. Composé de formule (I) 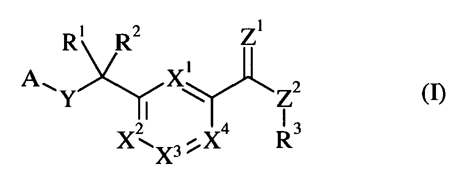 ou sel, acceptable sur le plan pharmaceutique, de celui-ci, dans lequel
X¹, X², X³ et X⁴ sont choisis indépendamment l'un de l'autre dans le groupe constitué de N ; et CR⁴, où R⁴ représente indépendamment H ; un atome d'halogène ; CN ; C(O)OR^{4a}; C(O)N(R^{4a}R^{4b}); OR^{4a}; N(R^{4a}R^{4b}); un groupe phényle ; un groupe alkyle en C₁-C₆ ; ou un groupe cycloalkyle en C₃-C₇, le groupe alkyle en C₁-C₆ et le groupe cycloalkyle en C₃-C₇ étant éventuellement substitués par un ou plusieurs atomes d'halogène, ces derniers étant identiques ou différents ;
R^{4a} et R^{4b} sont choisis indépendamment l'un de l'autre dans le groupe constitué de H ; un groupe alkyle en C₁-C₆; et un groupe cycloalkyle en C₃-C₇ ;
R¹ et R² sont choisis indépendamment l'un de l'autre dans le groupe constitué de H ; un groupe alkyle en C₁-C₆ ; un groupe cycloalkyle en C₃-C₇ ; et un atome d'halogène, le groupe alkyle en C₁-C₆ et le groupe cycloalkyle en C₃-C₇ étant éventuellement substitués par un atome d'halogène ;
R¹ et R² sont éventuellement combinés pour former un groupe oxo (=O) ;
Y représente -N(R^{1a})S(O)₂-; -S(O)₂N(R^{1b})- ; ou -N(R^{1a})S(O)₂N(R^{1b})- ;
A représente T ; ou un groupe alkyle en C₁-C₆, le groupe alkyle en C₁-C₆ étant éventuellement substitué par au moins un R⁵, chaque R⁵ représentant indépendamment l'un de l'autre un atome d'halogène; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); ou T ;
R^{1a} et R^{1b} sont choisis indépendamment l'un de l'autre dans le groupe constitué de H ; un groupe allyle ; un groupe benzyle ; CH₂-C(O)NH₂; CH₂-COOH; CH₂-C(O)O-alkyle en C₁-C₆ un groupe alkyle en C₁-C₆; et un groupe cycloalkyle en C₃-C₇, le groupe alkyle en C₁-C₆ et le groupe cycloalkyle en C₃-C₇ étant éventuellement substitués par au moins un R^{1c}, chaque groupe R^{1c} étant choisi indépendamment l'un de l'autre parmi un halogène ; un groupe alkyle en C₁-C₆; -CH=CH₂; un groupe phényle ; C(O)NH₂; COOH ; C(O)O-alkyle en C₁-C₆ ou un groupe cycloalkyle en C₃-C₇;
A et R^{1a} forment, éventuellement, conjointement avec l'azote auquel ils sont fixés, un hétérocycle ou un hétérobicycle éventuellement substitué par un ou plusieurs R⁷, ces derniers étant identiques ou différents ;
R⁶, R^{6a} et R^{6b} sont choisis indépendamment l'un de l'autre dans le groupe constitué de H; un groupe alkyle en C₁-C₆; et T, le groupe alkyle en C₁-C₆ étant éventuellement substitué par un ou plusieurs atomes d'halogène, ces derniers étant identiques ou différents ;
T représente un groupe cycloalkyle en C₃-C₇; un groupe hétérocyclyle ; un groupe hétérobicyclyle; un groupe phényle ; un groupe naphtyle ; un groupe indényle ; un groupe indanyle; un groupe tétralinyle ; un groupe décalinyle ; ou un groupe adamantyle, T étant éventuellement substitué par un ou plusieurs R⁷, ces derniers étant identiques ou différents ;
R⁷ représente indépendamment un atome d'halogène ; CN ; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); un groupe oxo (=O), dont le noyau est au moins partiellement saturé ; C(O)R⁸; T¹; ou un groupe alkyle en C₁-C₆, le groupe alkyle en C₁-C₆ étant éventuellement substitué par au moins un R⁹;
R⁸, R^{8a} et R^{8b} sont choisis indépendamment l'un de l'autre dans le groupe constitué de H ; T¹; et un groupe alkyle en C₁-C₆, le groupe alkyle en C₁-C₆ étant éventuellement substitué par au moins un R¹⁰;
R⁹ et R¹⁰ sont choisis indépendamment l'un de l'autre dans le groupe constitué d'un atome d'halogène ; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); et T¹;
R¹¹, R^{11a} et R^{11b} sont choisis indépendamment l'un de l'autre dans le groupe constitué de H ; un groupe alkyle en C₁-C₆ et T¹;
T¹ représente un groupe cycloalkyle en C₃-C₇; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle, T¹ étant éventuellement substitué par un ou plusieurs R¹², chaque R¹² représentant indépendamment l'un de l'autre un atome d'halogène ; CN ; COOR¹³ ; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a}; N(R¹³)S(O)R^{13a}; N(R¹³)C(O)N(R^{13a}R^{13b}); N(R¹³)C(O)OR^{13a}; OC(O)N(R¹³R^{13a}); un groupe oxo (=O), dont le noyau est au moins partiellement saturé ; C(O)R¹³; un groupe alkyle en C₁-C₆; un groupe phényle ; un groupe cycloalkyle en C₃-C₇; ou un groupe hétérocyclyle, le groupe alkyle en C₁-C₆; le groupe phényle ; le groupe cycloalkyle en C₃-C₇; et le groupe hétérocyclyle étant éventuellement substitués par un ou plusieurs atomes d'halogène, ces derniers étant identiques ou différents;
R¹³, R^{13a} et R^{13b} sont choisis indépendamment l'un de l'autre dans le groupe constitué de H ; un groupe alkyle en C₁-C₆; un groupe phényle ; un groupe cycloalkyle en C₃-C₇; et un groupe hétérocyclyle, le groupe alkyle en C₁-C₆; le groupe phényle ; le groupe cycloalkyle en C₃-C₇; et le groupe hétérocyclyle étant éventuellement substitués par un ou plusieurs atomes d'halogène, ces derniers étant identiques ou différents ;
Z¹ représente =O ou =S ;
éventuellement, Z¹ représente =N-R¹⁵ et R¹⁵ et R³ forment conjointement avec les atomes auxquels ils sont fixés un hétérocycle ou un hétérobicycle, l'hétérocycle ou l'hétérobicycle étant éventuellement substitués par au moins un R¹⁶, sous réserve que l'hétérocycle soit différent de 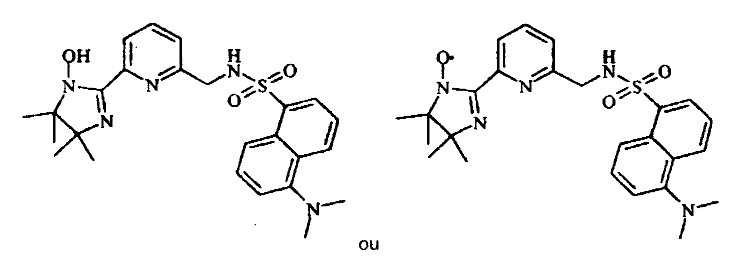
Z² représente O ; S ; ou N-R¹⁴;
R³ et R¹⁴ sont choisis indépendamment l'un de l'autre dans le groupe constitué de H ; T² ; un groupe alkyle en C₁-C₆, le groupe alkyle en C₁-C₆ étant éventuellement substitué par au moins un R¹⁷, sous réserve que lorsque l'un parmi R³ et R¹⁴ représente H et que l'autre représente un groupe éthyle, R¹⁷ soit autre que -COOH; éventuellement, R³ et R¹⁴ forment conjointement avec l'azote auxquels ils sont fixés un hétérocycle ou un hétérobicycle, ledit hétérocycle ou hétérobicycle étant éventuellement substitué par au moins un R¹⁸, sous réserve que l'hétérobicycle soit autre que la 1,2,3,4-tétrahydroquinoline et ses dérivés 4-oxa, -thia, -aza ou 8-aza non substitués ou substitués en position 3, 6 et/ou 7 ;
T² représente un groupe cycloalkyle en C₃-C₇ ; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle, T² étant éventuellement substitué par au moins un R¹⁹ ; sous réserve que lorsque A représente un groupe phényle ; Y représente -S(O)₂NH-, le soufre étant fixé à A ; R¹ et R² représentent H^{;} X¹, X³ et X⁴ représentent CH ; X² représente C-R⁴, R⁴ représentant OCH₃ ; Z¹ représente =O^{;} et Z² représente NH, T² étant autre qu'un groupe phényle non substitué ;
R¹⁶, R¹⁸ et R¹⁹ sont choisis indépendamment l'un de l'autre dans le groupe constitué d'un atome d'halogène ; CN ; COOR²⁰; OR²⁰; C(O)N(R²⁰R^{20a}); S(O)₂N(R²⁰R^{20a}); S(O)N(R²⁰R^{20a}); S(O)₂R²⁰; N(R²⁰)S(O)₂N(R^{20a}R^{20b}); SR²⁰; N(R²⁰R^{20a}); OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); un groupe oxo (=O), dont le noyau est au moins partiellement saturé ; C(O)R²⁰; un groupe alkyle en C₁-C₆; un groupe phényle ; un groupe hétérocyclyle ; et un groupe cycloalkyle en C₃-C₇, le groupe alkyle en C₁-C₆ le groupe phényle ; le groupe hétérocyclyle ; et le groupe cycloalkyle en C₃-C₇ étant éventuellement substitués par au moins un R²¹;
R²⁰, R^{20a} et R^{20b} sont choisis indépendamment l'un de l'autre dans le groupe constitué de H ; un groupe phényle ; un groupe hétérocyclyle ; un groupe cycloalkyle en C₃-C₇; et un groupe alkyle en C₁-C₆, le groupe phényle ; le groupe hétérocyclyle ; le groupe cycloalkyle en C₃-C₇ ; et le groupe alkyle en C₁-C₆ étant éventuellement substitués par un ou plusieurs atomes d'halogène, ces derniers étant identiques ou différents ;
R¹⁷ et R²¹ sont choisis indépendamment l'un de l'autre dans le groupe constitué d'un atome d'halogène; CN ; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); un groupe alkyle en C₁-C₆; un groupe phényle ; un groupe hétérocyclyle ; et un groupe cycloalkyle en C₃-C₇; le groupe alkyle en C₁-C_{6;} le groupe phényle ; le groupe hétérocyclyle ; et le groupe cycloalkyle en C₃-C₇ étant éventuellement substitués par au moins un R²³;
R²², R^{22a} et R^{22b} sont choisis indépendamment l'un de l'autre dans le groupe constitué de H ; un groupe phényle ; un groupe hétérocyclyle; un groupe cycloalkyle en C₃-C₇; et un groupe alkyle en C₁-C₆, le groupe phényle ; le groupe hétérocyclyle ; le groupe cycloalkyle en C₃-C₇; et le groupe alkyle en C₁-C₆ étant éventuellement substitués par au moins un R^{23a};
R²³ et R^{23a} sont choisis indépendamment l'un de l'autre dans le groupe constitué d'un atome d'halogène; CN ; COOR²⁴; OR²⁴; C(O)R²⁴; C(O)N(R²⁴R^{24a}); S(O)₂N(R²⁴R^{24a})_{;} S(O)N(R²⁴R^{24a}); S(O)₂R²⁴; N(R²⁴)S(O)₂N(R^{24a}R^{24b}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); et un groupe alkyle en C₁-C₆; le groupe alkyle en C₁-C₆ étant éventuellement substitué par un ou plusieurs atomes d'halogène, ces derniers étant identiques ou différents ;
R²⁴, R^{24a} et R^{24b} sont choisis indépendamment l'un de l'autre dans le groupe constitué de H ; un groupe alkyle en C₁-C₆, le groupe alkyle en C₁-C₆ étant éventuellement substitué par un ou plusieurs atomes d'halogène, ces derniers étant identiques ou différents,
sous réserve que le composé de formule (I) soit autre que le N-[1-(3-aminocarbonyl-4-hydroxyphényl)-2,2,2-trichloroéthyl]-4-chlorobenzènesulfamide.

2. Composé selon la revendication 1, dans lequel pas plus d'un parmi X¹, X², X³ et X⁴ ne représente N.

3. Composé selon la revendication 1 ou 2, dans lequel R¹ et R² représentent H ou sont combinés pour former =O.

4. Composé selon l'une quelconque des revendications 1 à 3, dans lequel R⁴ représente F ; Cl ; Br ; CH₃ ; OCH₃ ; NH₂ ; ou un groupe phényle.

5. Composé selon l'une quelconque des revendications 1 à 4, dans lequel Y représente -*S(O)₂-NH- ou -*S(O)₂-N(CH₃)-, l'atome marqué d'un astérisque étant fixé à A.

6. Composé selon l'une quelconque des revendications 1 à 5, dans lequel A représente un groupe phényle ; un groupe adamantyle ; un groupe cycloalkyle en C₃-C₇ ; un groupe hétérocyclyle ; ou un groupe alkyle en C₁-C6, le groupe alkyle en C₁-C₆ étant éventuellement substitué par T.

7. Composé selon la revendication 6, dans lequel A représente un groupe phényle ; un groupe cyclohexyle ; un groupe thiophényle ; un groupe benzothiophényle ; un groupe naphtyle ; un groupe diazolyle ; ou un groupe pyridyle.

8. Composé selon l'une quelconque des revendications 1 à 7, dans lequel A représente T et T est éventuellement substitué par jusqu'à 3 R⁷, représentant indépendamment l'un de l'autre F ; Cl ; CH₃ ; OCH₃ ; N(CH₃)₂ ; T¹ ; OT¹ ; ou NHT¹.

9. Composé selon la revendication 8, dans lequel T¹ représente un groupe phényle, éventuellement substitué par jusqu'à trois atomes d'halogène, représentant indépendamment l'un de l'autre F ; et Cl.

10. Composé selon l'une quelconque des revendications 1 à 9, dans lequel A représente T et T correspond à un groupe hétérobicyclyle.

11. Composé selon la revendication 10, dans lequel le groupe hétérobicyclyle est une décahydroquinoline ou une tétrahydroisoquinoline et dans lequel le noyau azoté est directement lié à l'atome de soufre du groupe sulfamide représentant Y.

12. Composé selon l'une quelconque des revendications 1 à 11, dans lequel Z¹ représente =O et Z² représente -NR¹⁴.

13. Composé selon l'une quelconque des revendications 1 à 12, dans lequel R¹⁴ représente H.

14. Composé selon l'une quelconque des revendications 1 à 13, dans lequel R³ représente un groupe phényle ; un groupe benzyle ; un groupe hétérocyclyle ; un groupe cycloalkyle en C₃-C₇; ou un groupe alkyle en C₁-C₆, le groupe alkyle en C₁-C₆ étant éventuellement substitué par un groupe O-alkyle en C₁-C₆ ou un groupe cycloalkyle en C₃-C₇.

15. Composé selon l'une quelconque des revendications 1 à 14, dans lequel R³ représente T² et T² est substitué par jusqu'à trois R¹⁹, représentant indépendamment l'un de l'autre F ; Cl ; CH₃; OCH₃; N(CH₃)₂; C(O)OH; C(O)OCH₃; CF₃; ou C(O)NH₂.

16. Composé selon la revendication 15, dans lequel T² représente un groupe phényle; un groupe cyclohexyle ; ou un groupe pyridyle.

17. Composé selon l'une quelconque des revendications 1 à 16, dans lequel R³ et R¹⁴ représentent indépendamment l'un de l'autre H ou un groupe alkyle en C₁-C₆ pouvant correspondre à un groupe éthyle ; un groupe isopropyle ; un groupe n-propyle ; un groupe n-butyle, le groupe alkyle en C₁-C₆ étant éventuellement substitué par N(CH₃)₂ ; ou N(C₂H₅)₂.

18. Composé selon l'une quelconque des revendications 1 à 17, dans lequel Z¹ représente =N-R¹⁵ et R¹⁵ et R³ forment conjointement un hétérocycle.

19. Composé selon la revendication 18, dans lequel l'hétérocycle est un groupe oxazole; oxadiazole; diazole; ou triazole ; l'hétérocycle étant éventuellement substitué par un R¹⁶ représentant un groupe alkyle en C₁-C₆, éventuellement substitué par N(CH₃)₂ ou N(CH₂CH₃)₂; un groupe cycloalkyle en C₃-C₇ ; un groupe phényle ; ou un groupe hétérocyclyle.

20. Composé selon la revendication 19, dans lequel R¹⁶ représente un groupe méthyle ; un groupe éthyle ; un groupe isopropyle; un groupe cyclohexyle ; un groupe phényle ; ou un groupe pipéridinyle.

21. Composé selon l'une quelconque des revendications 1 à 20, dans lequel R³ et R¹⁴ forment conjointement un hétérocycle.

22. Composé selon la revendication 21, dans lequel l'hétérocycle est la pipérazine ; la morpholine ; la pipéridine ; ou la pyrrolidine, l'hétérocycle étant éventuellement substitué par du méthyle.

23. Composé de formule (I) 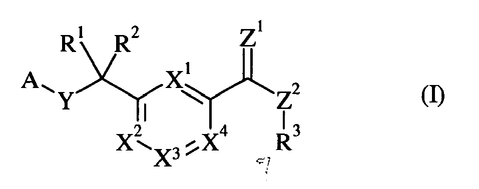 ou sel, acceptable sur le plan pharmaceutique, de celui-ci, dans lequel
X¹, X², X³ et X⁴ sont choisis indépendamment l'un de l'autre dans le groupe constitué de N ; et CR⁴, où R⁴ représente indépendamment H ; un atome d'halogène ; CN ; C(O)OR^{4a}_{;} C(O)N(R^{4a}R^{4b}) ; OR^{4a} ; N(R^{4a}R^{4b}) ; un groupe phényle ; un groupe alkyle en C₁-C₆ ; ou un groupe cycloalkyle en C₃-C₇, le groupe alkyle en C₁-C₆ et le groupe cycloalkyle en C₃-C₇ étant éventuellement substitués par un ou plusieurs atomes d'halogène, ces derniers étant identiques ou différents ;
R^{4a} et R^{4b} sont choisis indépendamment l'un de l'autre dans le groupe constitué de H ; un groupe alkyle en C₁-C₆ ; et un groupe cycloalkyle en C₃-C₇ ;
R¹ et R² sont choisis indépendamment l'un de l'autre dans le groupe constitué de H ; un groupe alkyle en C₁-C₆ un groupe cycloalkyle en C₃-C₇ ; et un atome d'halogène, le groupe alkyle en C₁-C₆ et le groupe cycloalkyle en C₃-C₇ étant éventuellement substitués par un atome d'halogène ;
R¹ et R² sont éventuellement combinés pour former un groupe oxo (=O) ;
Y représente -N(R^{1a})S(O)₂- ; -S(O)₂N(R^{1b})- ; ou -N(R^{1a})S(O)₂N(R^{1b})- ;
A représente T ; ou un groupe alkyle en C₁-C₆, le groupe alkyle en C₁-C₆ étant éventuellement substitué par au moins un R⁵, chaque R⁵ représentant indépendamment l'un de l'autre un atome d'halogène; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); ou T ;
R^{1a} et R^{1b} sont choisis indépendamment l'un de l'autre dans le groupe constitué de H ; un groupe allyle ; un groupe benzyle ; CH₂-C(O)NH₂; CH₂-COOH; CH₂-C(O)O-alkyle en C₁-C₆; un groupe alkyle en C₁-C₆; et un groupe cycloalkyle en C₃-C₇, le groupe alkyle en C₁-C₆ et le groupe cycloalkyle en C₃-C₇ étant éventuellement substitués par au moins un R^{1c}, chaque R^{1c} étant choisi indépendamment l'un de l'autre parmi un halogène ; un groupe alkyle en C₁-C₆; -CH=CH₂; un groupe phényle ; C(O)NH₂; COOH; C(O)O-alkyle en C₁-C₆ ou un groupe cycloalkyle en C₃-C₇;
A et R^{1a} forment, éventuellement, conjointement avec l'azote auquel ils sont fixés, un hétérocycle ou un hétérobicycle éventuellement substitué par un ou plusieurs R⁷, ces derniers étant identiques ou différents ;
R⁶, R^{6a} et R^{6b} sont choisis indépendamment l'un de l'autre dans le groupe constitué de H ; un groupe alkyle en C₁-C₆; et T, le groupe alkyle en C₁-C₆ étant éventuellement substitué par un ou plusieurs atomes d'halogène, ces derniers étant identiques ou différents ;
T représente un groupe cycloalkyle en C₃-C₇; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle ; T étant éventuellement substitué par un ou plusieurs R⁷, ces derniers étant identiques ou différents ;
R⁷ représente indépendamment un atome d'halogène ; CN ; COOR⁸; OR⁸ ; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸ ; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); un groupe oxo (=O), dont le noyau est au moins partiellement saturé ; C(O)R⁸ ; T¹ ; ou un groupe alkyle en C₁-C₆; le groupe alkyle en C₁-C₆ étant éventuellement substitué par au moins un R⁹ ;
R⁸, R^{8a} et R^{8b} sont choisis indépendamment l'un de l'autre dans le groupe constitué de H ; T¹ ; et un groupe alkyle en C₁-C₆, le groupe alkyle en C₁-C₆ étant éventuellement substitué par au moins un R¹⁰ ;
R⁹ et R¹⁰ sont choisis indépendamment l'un de l'autre dans le groupe constitué d'un atome d'halogène; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); et T¹ ;
R¹¹, R^{11a} et R^{11b} sont choisis indépendamment l'un de l'autre dans le groupe constitué de H ; un groupe alkyle en C₁-C₆ ; et T¹ ;
T¹ représente un groupe cycloalkyle en C₃-C₇ ; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle, T¹ étant éventuellement substitué par au moins un R¹², chaque R¹² représentant indépendamment l'un de l'autre un atome d'halogène ; CN ; COOR¹³ ; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a}; N(R¹³)S(O)R^{13a}; N(R¹³)C(O)N(R^{13a}R^{13b}); N(R¹³)C(O)OR^{13a}; OC(O)N(R¹³R^{13a}); un groupe oxo (=O), dont le noyau est au moins partiellement saturé ; C(O)R¹³; un groupe alkyle en C₁-C₆; un groupe phényle ; un groupe cycloalkyle en C₃-C₇; ou un groupe hétérocyclyle, le groupe alkyle en C₁-C₆; le groupe phényle ; le groupe cycloalkyle en C₃-C₇; et le groupe hétérocyclyle étant éventuellement substitués par un ou plusieurs atomes d'halogène, ces derniers étant identiques ou différents ;
R¹³, R^{13a} et R^{13b} sont choisis indépendamment l'un de l'autre dans le groupe constitué de H ; un groupe alkyle en C₁-C₆; un groupe phényle ; un groupe cycloalkyle en C₃-C₇; et un groupe hétérocyclyle, le groupe alkyle en C₁-C₆; le groupe phényle ; le groupe cycloalkyle en C₃-C₇; et le groupe hétérocyclyle étant éventuellement substitués par un ou plusieurs atomes d'halogène, ces derniers étant identiques ou différents ;
Z¹ représente =O ou =S ;
éventuellement, Z¹ représente =N-R¹⁵ et R¹⁵ et R¹³ forment conjointement avec les atomes auxquels ils sont fixés un hétérocycle ou un hétérobicycle, l'hétérocycle ou l'hétérobicycle étant éventuellement substitué par au moins un R¹⁶;
Z² représente O ; S ; ou N-R¹⁴ ;
R³ et R¹⁴ sont choisis indépendamment l'un de l'autre dans le groupe constitué de H ; T² ; un groupe alkyle en C₁-C₆, le groupe alkyle en C₁-C₆ étant éventuellement substitué par au moins un R¹⁷, sous réserve que lorsque l'un parmi R³ et R¹⁴ représente H et que l'autre représente un groupe éthyle, R¹⁷ soit autre que -COOH ;
éventuellement, R³ et R¹⁴ forment conjointement avec l'azote auxquels ils sont fixés un hétérocycle ou un hétérobicycle, ledit hétérocycle ou hétérobicycle étant éventuellement substitué par au moins un R¹⁸, sous réserve que l'hétérobicycle soit autre que la 1,2,3,4-tétrahydroquinoline et ses dérivés 4-oxa, -thia, -aza et/ou 8-aza non substitués ou substitués en position 3, 6 et/ou 7 ;
T² représente un groupe cycloalkyle en C₃-C₇ ; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle, T² étant éventuellement substitué par au moins un R¹⁹ ; sous réserve que quand A représente un groupe phényle ; Y représente -S(O)₂NH-, le soufre étant fixé à A ; R¹ et R² représentent H ; X¹, X³ et X⁴ représentent CH ; X² représente C-R⁴, R⁴ correspondant à OCH₃ ; Z¹ représente =O^{;} et Z² représente NH, T² étant autre qu'un groupe phényle non substitué ;
R¹⁶, R¹⁸ et R¹⁹ sont choisis indépendamment l'un de l'autre dans le groupe constitué d'un atome d'halogène ; CN ; COOR²⁰ ; OR²¹ ; C(O)N(R²⁰R^{20a}) ; S(O)₂N(R²⁰R^{20a}) ; S(O)N(R²⁰R^{20a}); S(O)₂R²⁰; N(R²⁰)S(O)₂N(R^{20a}R^{20b}); SR²⁰; N(R²⁰R^{20a}); OC(O)R²⁰; N(R²⁰)C(O)R^{20a}; N(R²⁰)S(O)₂R^{20a}; N(R²⁰)S(O)R^{20a}; N(R²⁰)C(O)N(R^{20a}R^{20b}); N(R²⁰)C(O)OR^{20a}; OC(O)N(R²⁰R^{20a}); un groupe oxo (=O), dont le noyau est au moins partiellement saturé ; C(O)R²⁰; un groupe alkyle en C₁-C₆; un groupe phényle ; un groupe hétérocyclyle ; et un groupe cycloalkyle en C₃-C₇, le groupe alkyle en C₁-C₆; le groupe phényle ; le groupe hétérocyclyle ; et le groupe cycloalkyle en C₃-C₇ étant éventuellement substitués par au moins un R²¹;
R²⁰, R^{20a} et R^{20b} sont choisis indépendamment l'un de l'autre dans le groupe constitué de H ; un groupe phényle ; un groupe hétérocyclyle ; un groupe cycloalkyle en C₃-C₇ et un groupe alkyle en C₁-C₆ ; le groupe phényle ; le groupe hétérocyclyle ; le groupe cycloalkyle en C₃-C₇ ; et le groupe alkyle en C₁-C₆ étant éventuellement substitués par un ou plusieurs atomes d'halogène, ces derniers étant identiques ou différents ;
R¹⁷ et R²¹ sont choisis indépendamment l'un de l'autre dans le groupe constitué d'un atome d'halogène; CN ; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}); OC(O)R²²; N(R²²)C(O)R^{22a}; N(R²²)SO₂R^{22a}; N(R²²)S(O)R^{22a}; N(R²²)C(O)N(R^{22a}R^{22b}); N(R²²)C(O)OR^{22a}; OC(O)N(R²²R^{22a}); un groupe alkyle en C₁-C₆ ; un groupe phényle ; un groupe hétérocyclyle ; et un groupe cycloalkyle en C₃-C₇ ; le groupe alkyle en C₁-C₆ ; le groupe phényle ; le groupe hétérocyclyle ; et le groupe cycloalkyle en C₃-C₇ étant éventuellement substitués par au moins un R²³ ;
R²², R^{22a} et R^{22b} sont choisis indépendamment l'un de l'autre dans le groupe constitué de H ; un groupe phényle ; un groupe hétérocyclyle ; un groupe cycloalkyle en C₃-C₇ et un groupe alkyle en C₁-C₆, le groupe phényle ; le groupe hétérocyclyle ; le groupe cycloalkyle en C₃-C₇ ; et le groupe alkyle en C₁-C₆ étant éventuellement substitués par au moins un R^{23a} ;
R²³ et R^{23a} sont choisis indépendamment l'un de l'autre dans le groupe constitué d'un atome d'halogène; CN ; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a}); OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}); N(R²⁴)C(O)OR^{24a}; OC(O)N(R²⁴R^{24a}); et un groupe alkyle en C₁-C₆ ; le groupe alkyle en C₁-C₆ étant éventuellement substitué par un ou plusieurs atomes d'halogène, ces derniers étant identiques ou différents ;
R²⁴, R^{24a} et R^{24b} sont choisis indépendamment l'un de l'autre dans le groupe constitué de H ; un groupe alkyle en C₁-C₆, le groupe alkyle en C₁-C₆ étant éventuellement substitué par un ou plusieurs atomes d'halogène, ces derniers étant identiques ou différents,
utilisable en tant que médicament.

24. Composition pharmaceutique comprenant un composé ou son sel, acceptable sur le plan pharmaceutique, comme défini selon la revendication 23, ainsi qu'un support acceptable sur le plan pharmaceutique, éventuellement en association avec au moins une autre composition pharmaceutique.

25. Utilisation d'un composé de formule (I) 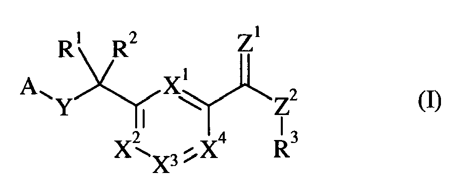 ou sel, acceptable sur le plan pharmaceutique, de celui-ci dans lequel
X¹, X², X³ et X⁴ sont choisis indépendamment l'un de l'autre dans le groupe constitué de N ; et CR⁴, où R⁴ représente indépendamment H ; un atome d'halogène ; CN ; C(O)OR^{4a}; C(O)N(R^{4a}R^{4b}); OR^{4a}; N(R^{4a}R^{4b}); un groupe phényle ; un groupe alkyle en C₁-C₆ ou un groupe cycloalkyle en C₃-C₇, le groupe alkyle en C₁-C₆ et le groupe cycloalkyle en C₃-C₇ étant éventuellement substitués par un ou plusieurs atomes d'halogène, ces derniers étant identiques ou différents ;
R^{4a} et R^{4b} sont choisis indépendamment l'un de l'autre dans le groupe constitué de H ; un groupe alkyle en C₁-C₆ ; et un groupe cycloalkyle en C₃-C₇ ;
R¹ et R² sont choisis indépendamment l'un de l'autre dans le groupe constitué de H ; un groupe alkyle en C₁-C₆ ; un groupe cycloalkyle en C₃-C₇ ; et un atome d'halogène, le groupe alkyle en C₁-C₆ et le groupe cycloalkyle en C₃-C₇ étant éventuellement substitués par un atome d'halogène ;
R¹ et R² sont éventuellement combinés pour former un groupe oxo (=O) ;
Y représente -N(R^{1a})S(O)₂- ; -S(O)₂N(R^{1b})- ; ou -N(R^{1a})S(O)₂N(R^{1b})- ;
A représente T ; ou un groupe alkyle en C₁-C₆, le groupe alkyle en C₁-C₆ étant éventuellement substitué par au moins un R⁵, chaque R⁵ représentant indépendamment l'un de l'autre un atome d'halogène ; CN ; COOR⁶; OR⁶; C(O)N(R⁶R^{6a}) ; S(O)₂N(R⁶R^{6a}); S(O)N(R⁶R^{6a}); S(O)₂R⁶; N(R⁶)S(O)₂N(R^{6a}R^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); ou T ;
R^{1a} et R^{1b} sont choisis indépendamment l'un de l'autre dans le groupe constitué de H ; un groupe allyle ; un groupe benzyle ; CH₂-C(O)NH₂; CH₂-COOH ; CH₂-C(O)O-alkyle en C₁-C₆; un groupe alkyle en C₁-C₆; et un groupe cycloalkyle en C₃-C₇, le groupe alkyle en C₁-C₆ et le groupe cycloalkyle en C₃-C₇ étant éventuellement substitués par au moins un R^{1c}, chaque R^{1c} étant choisi indépendamment l'un de l'autre parmi un halogène ; un groupe alkyle en C₁-C₆; -CH=CH₂; un groupe phényle ; C(O)NH₂; COOH ; C(O)O-alkyle en C₁-C₆ ou un groupe cycloalkyle en C₃-C₇ ;
A et R^{1a} forment éventuellement, conjointement avec l'azote auquel ils sont fixés, un hétérocycle ou un hétérobicycle éventuellement substitué par un ou plusieurs R⁷, ces derniers étant identiques ou différents ;
R⁶, R^{6a} et R^{6b} sont choisis indépendamment l'un de l'autre dans le groupe constitué de H; un groupe alkyle en C₁-C₆; et T, le groupe alkyle en C₁-C₆ étant éventuellement substitué par un ou plusieurs atomes d'halogène, ces derniers étant identiques ou différents ;
T représente un groupe cycloalkyle en C₃-C₇ ; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle, T étant éventuellement substitué par un ou plusieurs R⁷, ces derniers étant identiques ou différents ;
R⁷ représente indépendamment un atome d'halogène ; CN ; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}R^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); un groupe oxo (=O), dont le noyau est au moins partiellement saturé ; C(O)R⁸; T¹ ; ou un groupe alkyle en C₁-C₆ ; le groupe alkyle en C₁-C₆ étant éventuellement substitué par au moins un R⁹;
R⁸, R^{8a} et R^{8b} sont choisis indépendamment l'un de l'autre dans le groupe constitué de H ; T¹ ; et un groupe alkyle en C₁-C₆, le groupe alkyle en C₁-C₆ étant éventuellement substitué par au moins un R¹⁰ ;
R⁹ et R¹⁰ sont choisis indépendamment l'un de l'autre dans le groupe constitué d'un atome d'halogène; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}R^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); et T¹ ;
R¹¹, R^{11a} et R^{11b} sont choisis indépendamment l'un de l'autre dans le groupe constitué de H ; un groupe alkyle en C₁-C₆ ; et T¹ ;
T¹ représente un groupe cycloalkyle en C₃-C₇ ; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle, T¹ étant éventuellement substitué par au moins un R¹², chaque R¹² représentant indépendamment l'un de l'autre un atome d'halogène ; CN ; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}R^{13b}); SR¹³; N(R¹³R^{13a}); OC(O)R¹³; N(R¹³)C(O)R^{13a}; N(R¹³)S(O)₂R^{13a}; N(R¹³)S(O)R^{13a}; N(R¹³)C(O)N(R^{13a}R^{13b}); N(R¹³)C(O)OR^{13a}; OC(O)N(R¹³R^{13a}); un groupe oxo (=O), dont le noyau est au moins partiellement saturé ; C(O)R¹³; un groupe alkyle en C₁-C₆ ; un groupe phényle ; un groupe cycloalkyle en C₃-C₇ ; ou un groupe hétérocyclyle, le groupe alkyle en C₁-C₆ ; le groupe phényle ; le groupe cycloalkyle en C₃-C₇ ; et le groupe hétérocyclyle étant éventuellement substitués par un ou plusieurs atomes d'halogène, ces derniers étant identiques ou différents ;
R¹³, R^{13a} et R^{13b} sont choisis indépendamment l'un de l'autre dans le groupe constitué de H ; un groupe alkyle en C₁-C₆; un groupe phényle ; un groupe cycloalkyle en C₃-C₇; et un groupe hétérocyclyle, le groupe alkyle en C₁-C₆ ; le groupe phényle ; le groupe cycloalkyle en C₃-C₇ ; et le groupe hétérocyclyle étant éventuellement substitués par un ou plusieurs atomes d'halogène, ces derniers étant identiques ou différents ;
Z¹ représente =O ou =S ;
éventuellement, Z¹ représente =N-R¹⁵ et R¹⁵ et R³ forment conjointement avec les atomes auxquels ils sont fixés un hétérocycle ou un hétérobicycle, l'hétérocycle ou l'hétérobicycle étant éventuellement substitué par au moins un R¹⁶ ;
Z² représente O ; S ; ou N-R¹⁴ ;
R³ et R¹⁴ sont choisis indépendamment l'un de l'autre dans le groupe constitué de H ;
T² ; un groupe alkyle en C₁-C₆, le groupe alkyle en C₁-C₆ étant éventuellement substitué par au moins un R¹⁷, sous réserve que lorsque l'un parmi R³ et R¹⁴ représente H et que l'autre représente un groupe éthyle, R¹⁷ soit autre que -COOH ;
éventuellement, R³ et R¹⁴ forment conjointement avec l'azote auxquels ils sont fixés un hétérocycle ou un hétérobicycle, ledit hétérocycle ou hétérobicycle étant éventuellement substitué par au moins un R¹⁸ ;
T² représente un groupe cycloalkyle en C₃-C₇ ; un groupe hétérocyclyle ; un groupe hétérobicyclyle ; un groupe phényle ; un groupe naphtyle ; un groupe indényle ; un groupe indanyle ; un groupe tétralinyle ; un groupe décalinyle ; ou un groupe adamantyle, T² étant éventuellement substitué par au moins un R¹⁹ ; sous réserve que lorsque A représente un groupe phényle ; Y représente -S(O)₂NH-, le soufre étant fixé à A ; R¹ et R² représentent H^{;} X¹, X³ et X⁴ représentent CH ; X² représente C-R⁴,
R⁴ représentant OCH₃ ; Z¹ représente =O^{;} et Z² représente NH, T² étant autre qu'un groupe phényle non substitué ;
R¹⁶, R¹⁸ et R¹⁹ sont choisis indépendamment l'un de l'autre dans le groupe constitué d'un atome d'halogène ; CN ; COOR²⁰ ; OR²⁰ ; C(O)N(R²⁰R^{20a}) ; S(O)₂N(R²⁰R^{20a}) ; S(O)N(R²⁰R^{20a}) ; S(O)₂R²⁰ ; N(R²⁰)S(O)₂N(R^{20a}R^{20b}) ; SR²⁰ ; N(R²⁰R^{20a}) ; OC(O)R²⁰ ; N(R²⁰)C(O)R^{20a} ; N(R²⁰)S(O)₂R^{20a} ; N(R²⁰)S(O)R^{20a} ; N(R²⁰)C(O)N(R^{20a}R^{20b}) ; N(R²⁰)C(O)OR^{20a} ; OC(O)N(R²⁰R^{20a}) ; un groupe oxo (=O), dont le noyau est au moins partiellement saturé ; C(O)R²⁰ ; un groupe alkyle en C₁-C₆ ; un groupe phényle ; un groupe hétérocyclyle ; et un groupe cycloalkyle en C₃-C₇, le groupe alkyle en C₁-C₆ ; le groupe phényle ; le groupe hétérocyclyle ; et le groupe cycloalkyle en C₃-C₇ étant éventuellement substitués par au moins un R²¹ ;
R²⁰, R^{20a} et R^{20b} sont choisis indépendamment l'un de l'autre dans le groupe constitué de H ; un groupe phényle ; un groupe hétérocyclyle ; un groupe cycloalkyle en C₃-C₇ et un groupe alkyle en C₁-C₆ ; le groupe phényle ; le groupe hétérocyclyle ; le groupe cycloalkyle en C₃-C₇ ; et le groupe alkyle en C₁-C₆ étant éventuellement substitués par un ou plusieurs atomes d'halogène, ces derniers étant identiques ou différents ;
R¹⁷ et R²¹ sont choisis indépendamment l'un de l'autre dans le groupe constitué d'un atome d'halogène; CN ; COOR²²; OR²²; C(O)R²²; C(O)N(R²²R^{22a}); S(O)₂N(R²²R^{22a}); S(O)N(R²²R^{22a}); S(O)₂R²²; N(R²²)S(O)₂N(R^{22a}R^{22b}); SR²²; N(R²²R^{22a}) ; OC(O)R²²; N(R²²)C(O)R^{22a}; N(R²²)SO₂R^{22a}; N(R²²)S(O)R^{22a}; N(R²²)C(O)N(R^{22a}R^{22b}) ; N(R²²)C(O)OR^{22a} ; OC(O)N(R²²R^{22a}) ; un groupe alkyle en C₁-C₆ ; un groupe phényle ; un groupe hétérocyclyle ; et un groupe cycloalkyle en C₃-C₇ ; le groupe alkyle en C₁-C₆ ; le groupe phényle ; le groupe hétérocyclyle ; et le groupe cycloalkyle en C₃-C₇ étant éventuellement substitués par au moins un R²³ ;
R²², R^{22a} et R^{22b} sont choisis indépendamment l'un de l'autre dans le groupe constitué de H ; un groupe phényle ; un groupe hétérocyclyle ; un groupe cycloalkyle en C₃-C₇ et un groupe alkyle en C₁-C₆, le groupe phényle ; le groupe hétérocyclyle ; le groupe cycloalkyle en C₃-C₇ ; et le groupe alkyle en C₁-C₆ étant éventuellement substitués par au moins un R^{23a} ;
R²³ et R^{23a} sont choisis indépendamment l'un de l'autre dans le groupe constitué d'un atome d'halogène ; CN ; COOR²⁴; OR²⁴; C(O)R²⁴; C(O)N(R²⁴R^{24a}); S(O)₂N(R²⁴R^{24a}); S(O)N(R²⁴R^{24a}); S(O)₂R²⁴; N(R²⁴)S(O)₂N(R^{24a}R^{24b}); SR²⁴; N(R²⁴R^{24a}) ; OC(O)R²⁴; N(R²⁴)C(O)R^{24a}; N(R²⁴)SO₂R^{24a}; N(R²⁴)S(O)R^{24a}; N(R²⁴)C(O)N(R^{24a}R^{24b}) ; N(R²⁴)C(O)OR^{24a} ; OC(O)N(R²⁴R^{24a}) ; et un groupe alkyle en C₁-C₆ ; le groupe alkyle en C₁-C₆ étant éventuellement substitué par un ou plusieurs atomes d'halogène, ces derniers étant identiques ou différents ;
R²⁴, R^{24a}, R^{24b} sont choisis indépendamment l'un de l'autre dans le groupe constitué de H ; un groupe alkyle en C₁-C₆, le groupe alkyle en C₁-C₆ étant éventuellement substitué par un ou plusieurs atomes d'halogène, ces derniers étant identiques ou différents,
pour la fabrication d'un médicament destiné au traitement ou à la prophylaxie d'un diabète sucré non insulinodépendant, d'une hyperglycémie, d'une faible tolérance au glucose, d'une résistance à l'insuline, d'une obésité, de troubles lipidiques, d'une dyslipidémie, d'une hyperlipidémie, d'une hypertriglycéridémie, d'une hypercholestérolémie, d'un taux de HDL bas, d'un taux de LDL élevé, d'une athérosclérose et de ses séquelles, d'une resténose vasculaire, d'une pancréatite, d'une obésité abdominale, d'une maladie neurodégénérative, d'une rétinopathie, d'une néphropathie, d'une neuropathie, du syndrome X, de la maladie d'Alzheimer, de l'ostéoporose, du cancer, de l'épilepsie, de la dépression, de la lipodystrophie associée à une thérapie antirétrovirale hautement active ou d'autres affections et troubles dont la résistance à l'insuline est une composante ou pouvant être traités par l'inhibition de l'enzyme 11β-HSD1.

26. Composition pharmaceutique comprenant un composé ou son sel, acceptable sur le plan pharmaceutique, comme défini selon la revendication 25, ainsi qu'un support acceptable sur le plan pharmaceutique, comprenant, en outre, un ou plusieurs composés supplémentaires ou sels, acceptables sur le plan pharmaceutique, de ceux-ci, choisis dans le groupe constitué de composés tels que définis dans la revendication 25 et autres que le premier composé ; d'autres inhibiteurs de la 11β-HSD1 ; des inhibiteurs de la DPP-IV ; des sensibilisateurs à l'insuline, par exemple des agonistes des PPAR et des biguanides ; de l'insuline et des mimétiques de l'insuline ; des sulfonylurées et d'autres sécrétagogues de l'insuline ; des inhibiteurs de l'α-glucosidase ; des antagonistes du récepteur du glucagon ; de la GLP-1, des mimétiques de la GLP-1 et des agonistes du récepteur de la GLP-1 ; de la GIP, des mimétiques de la GIP et des agonistes du récepteur de la GIP ; du PACAP, des mimétiques du PACAP et des agonistes du récepteur 3 du PACAP ; des agents abaissant le taux de cholestérol, par exemple des inhibiteurs de la HMG-CoA réductase, des séquestrants, de l'alcool nicotinylique, de l'acide nicotinique ou un sel de ceux-ci, des agonistes des PPARγ, des agonistes multiples des PPARα/γ, des inhibiteurs de l'absorption du cholestérol, de l'acyl-CoA ; des inhibiteurs de la cholestérol acyltransférase et des anti-oxydants ; des agonistes des PPAR ; des composés anti-obésité ; des inhibiteurs des transporteurs de l'acide biliaire iléal ; des agents anti-inflammatoires ; et des inhibiteurs de la protéine tyrosine phosphatase-IB (PTP-IB) ; des composés anti-hypertenseurs ; et des composés destinés au traitement des maladies cardiovasculaires dont, en particulier, l'athérosclérose.

27. Procédé de préparation d'un composé tel que défini selon la revendication 25, comprenant les étapes suivantes :
- réduire un composé de formule (II) 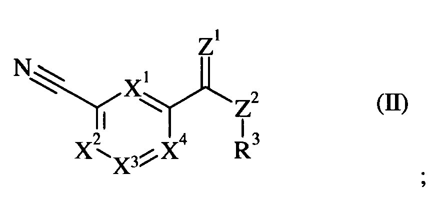 et
- coupler l'amine résultante (III) 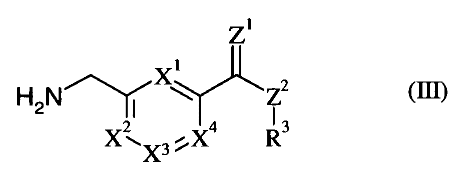 avec A-Y-Cl, où A représente T ou un groupe alkyle en C₁-C₆ ou
avec A-H et SO₂Cl₂, où A représente T-N(R^{1c})-.
